# EUROPEAN PATENT APPLICATION

(11) **EP 4 226 948 A1**
(43) Date of publication of application: **16.08.2023**
(21) Application number: 21877758.9
(22) Date of filing: 08.10.2021
(51) Int. Cl.: A61K 47/64, A61K 9/51, A61K 31/7105, A61K 38/08, A61K 38/10, A61K 38/12, A61P 43/00, C12N 15/113

(54) **OLIGONUCLEIC ACID CONJUGATE**

(30) Priority: 09.10.2020 JP 2020171447
(71) Applicant: Sumitomo Pharma Co., Ltd., Osaka 541-0045 (JP)
(72) Inventor: UESAKA, Akihiro, Suita-shi, Osaka 564-0053 (JP); MAKITA, Naoki, Suita-shi, Osaka 564-0053 (JP); TAKEDA, Masashi, Osaka-shi, Osaka 554-0022 (JP)
(74) Representative: Vossius & Partner Patentanwälte Rechtsanwälte mbB
(86) International application number: PCT/JP2021/037412
(87) International publication number: WO 2022/075459

(57) **Abstract**

An object of the present invention is to increase an amount of oligonucleotide transported into cytoplasm by allowing a cellular internalization enhancer to efficiently interact with target cells. An oligonucleotide conjugate according to the present invention contains a dendritic polymer, a plurality of oligonucleotides, one or a plurality of cellular internalization enhancers, and one or a plurality of hydrophilic linkers, wherein each oligonucleotide is bonded to the dendritic polymer directly or through a linker, and each cellular internalization enhancer is bonded to the dendritic polymer through the hydrophilic linker.

## Description

### Technical Field

The present invention relates to an oligonucleotide conjugate.

### Background Art

Nucleic acid therapeutics, which can directly regulate the expression of various gene products expressed in cells, can be therapeutic agents for diseases to which conventional medicines cannot be applied, and thus, thier medical applications are strongly expected. However, nucleic acid therapeutics cannot spontaneously permeate cell membranes because the nucleic acid molecules themselves have a large molecular weight, many negative charges, and high hydrophilicity. In order to transport a nucleic acid molecule into cytoplasm, where it works, a method which involves modifying the nucleic acid molecule with a cellular internalization enhancer such as a hydrophobic molecule or a saccharide, and a method which involves encapsulating a nucleic acid molecule in a functional nanoparticle are known.

For example, Patent Literature 1 and Non-Patent Literature 1 disclose a method of preparing a nanostructure by covalently bonding a nucleic acid molecule and a cellular internalization enhancer to a polymer, and transporting the nucleic acid molecule into cytoplasm.

### Citation List

### Patent Literature

Patent Literature 1: International Publication No. 2013/062982

### Non-Patent Literature

Non-Patent Literature 1: Charles L. McCormick et al., Biomacromolecules, vol. 11, p. 505-514 (2010)

### Summary of Invention

### Technical Problem

The methods of Patent Literature 1 and Non-Patent Literature 1 have room for improvement in terms of allowing a cellular internalization enhancer to efficiently interact with a target cell. A main object of the present invention is to increase the amount of oligonucleotides transported into cytoplasm by allowing a cellular internalization enhancer to efficiently interact with a target cell.

### Solution to Problem

As a result of extensive research, the present inventors have developed a method capable of efficiently transporting oligonucleotides into a cell. The present invention provides an oligonucleotide conjugate, which is a functional nanoparticle containing a cellular internalization enhancer, and a method for producing the same.

That is, according to one embodiment of the present invention, there is provided an oligonucleotide conjugate, which is a nanoparticle composed of a single molecule comprising a core of a dendritic polymer, and a plurality of oligonucleotides, one or a plurality of hydrophilic linkers, and one or a plurality of cellular internalization enhancers, which are arranged around the core, wherein the oligonucleotides and the hydrophilic linkers are bonded to the core, preferably through covalent bonds, and the cellular internalization enhancers are bonded to the hydrophilic linkers, preferably through covalent bonds. According to another embodiment of the present invention, reactive functional groups of the core dendritic polymer are used to bond to capping agents in addition to the oligonucleotides and the hydrophilic linkers. According to still another embodiment of the present invention, the linear length of the hydrophilic linker are longer than the molecular lengths of the oligonucleotides, or the spatial extent of the hydrophilic linkers (radius of gyration) is not completely enclosed within the spatial extent of the oligonucleotides, so that it becomes easier for the cellular internalization enhancers to be presented on the outermost layer of the functional nanoparticles and to interact with a target cell.

Namely, the present invention is as follows.
[1] An oligonucleotide conjugate comprising: a dendritic polymer; a plurality of oligonucleotides; one or a plurality of cellular internalization enhancers; and one or a plurality of hydrophilic linkers, wherein
   each oligonucleotide is bonded to the dendritic polymer directly or through a linker, and
   each cellular internalization enhancer is bonded to the dendritic polymer through the hydrophilic linker.
[2] The oligonucleotide conjugate according to [1], wherein bonds between the dendritic polymer and the oligonucleotides, bonds between the dendritic polymer and the hydrophilic linkers, bonds between the dendritic polymer and the linkers, bonds between the cellular internalization enhancers and the hydrophilic linkers, and bonds between the linkers and the oligonucleotides are covalent bonds, metal coordinations, or host-guest interactions.
[3] The oligonucleotide conjugate according to [1], wherein bonds between the dendritic polymer and the oligonucleotides, bonds between the dendritic polymer and the hydrophilic linkers, bonds between the dendritic polymer and the linkers, bonds between the cellular internalization enhancers and the hydrophilic linkers, and bonds between the linkers and the oligonucleotides are covalent bonds or metal coordinations.
[4] The oligonucleotide conjugate according to [1], wherein bonds between the dendritic polymer and the oligonucleotides, bonds between the dendritic polymer and the hydrophilic linkers, bonds between the dendritic polymer and the linkers, bonds between the cellular internalization enhancers and the hydrophilic linkers, and bonds between the linkers and the oligonucleotides are covalent bonds.
[5] The oligonucleotide conjugate according to any one of [1] to [4], wherein at least some of reactive functional groups of the dendritic polymer are capped with a capping agent.
[6] The oligonucleotide conjugate according to [5], wherein the capping agent is one or more molecules selected from the group consisting of a hydrophilic molecule and hydrophobic molecule.
[7] The oligonucleotide conjugate according to [6], wherein the capping agent is a hydrophilic molecule.
[8] The oligonucleotide conjugate according to [6], wherein the capping agent is one or more hydrophilic molecules selected from the group consisting of an electrically neutral hydrophilic molecule, polar molecule that protonates under acidic conditions, anionic molecule, and cationic molecule.
[9] The oligonucleotide conjugate according to [6], wherein the capping agent is one or more hydrophilic molecules selected from the group consisting of an electrically neutral hydrophilic molecule, polar molecule that protonates under acidic conditions, and anionic molecule.
[10] The oligonucleotide conjugate according to [6], wherein the capping agent is a hydrophobic molecule.
[11] The oligonucleotide conjugate according to [6], wherein the capping agent is one or more molecules selected from the group consisting of an aliphatic compound, an aromatic compound, a trialkylamine, and a steroid.
[12] The oligonucleotide conjugate according to [6], wherein the capping agent is an aliphatic compound.
[13] The oligonucleotide conjugate according to any one of [1] to [12], wherein the dendritic polymer is a dendrigraft or a dendrimer.
[14] The oligonucleotide conjugate according to any one of [1] to [12], wherein monomers in the dendritic polymer are bonded to each other by amide bonds, ester bonds, or glycosidic bonds.
[15] The oligonucleotide conjugate according to any one of [1] to [9], wherein monomers in the dendritic polymer are bonded to each other by amide bonds or ester bonds.
[16] The oligonucleotide conjugate according to any one of [1] to [12], wherein the dendritic polymer is a poly-L-lysine dendrigraft, a polyamidoamine dendrimer, or a 2,2-bis(hydroxyl-methyl)propionic acid dendrimer.
[17] The oligonucleotide conjugate according to any one of [1] to [16], wherein the oligonucleotide is a gene expression modifier.
[18] The oligonucleotide conjugate according to [17], wherein the gene expression modifier is a molecule that downregulates mRNA expression.
[19] The oligonucleotide conjugate according to [17], wherein the gene expression modifier is an RNA interference inducer or an antisense oligonucleotide.
[20] The oligonucleotide conjugate according to any one of [1] to [19], wherein an average linear distance between ends of each hydrophilic linker is 1/5 or more of a length of the oligonucleotide.
[21] The oligonucleotide conjugate according to any one of [1] to [19], wherein an average linear distance between ends of each hydrophilic linker is 1/4 or more of a length of the oligonucleotide.
[22] The oligonucleotide conjugate according to any one of [1] to [19], wherein an average linear distance between ends of each hydrophilic linker is 1/3 or more of a length of the oligonucleotide.
[23] The oligonucleotide conjugate according to any one of [1] to [19], wherein an average linear distance between ends of each hydrophilic linker is 2/5 or more of a length of the oligonucleotide.
[24] The oligonucleotide conjugate according to any one of [1] to [19], wherein an average linear distance between ends of each hydrophilic linker is half or more of a length of the oligonucleotide.
[25] The oligonucleotide conjugate according to any one of [1] to [24], wherein the hydrophilic linker is one or more hydrophilic linkers selected from the group consisting of polyethylene glycol, poly(2-alkyl-2-oxazoline), polypeptide, and polypeptoid.
[26] The oligonucleotide conjugate according to any one of [1] to [24], wherein the hydrophilic linker is one or more hydrophilic linkers selected from the group consisting of polyethylene glycol, poly(2-methyl-2-oxazoline), EK peptide, and polysarcosine.
[27] The oligonucleotide conjugate according to any one of [1] to [26], wherein the cellular internalization enhancer is one or more cellular internalization enhancers selected from the group consisting of a small-molecule ligand, polypeptide, aptamer, antibody or fragment thereof, saccharide, and lipid.
[28] The oligonucleotide conjugate according to any one of [1] to [26], wherein the cellular internalization enhancer is a small-molecule ligand.
[29] The oligonucleotide conjugate according to any one of [1] to [26], wherein the cellular internalization enhancer is a polypeptide.
[30] The oligonucleotide conjugate according to any one of [1] to [26], wherein the cellular internalization enhancer is an aptamer.
[31] The oligonucleotide conjugate according to any one of [1] to [26], wherein the cellular internalization enhancer is an antibody or a fragment thereof.
[32] The oligonucleotide conjugate according to any one of [1] to [26], wherein the cellular internalization enhancer is a saccharide.
[33] The oligonucleotide conjugate according to any one of [1] to [26], wherein the cellular internalization enhancer is a lipid.
[34] A pharmaceutical composition comprising the oligonucleotide conjugate according to any one of [1] to [33] as an active ingredient.
[35] A therapeutic agent or a preventive agent comprising the oligonucleotide conjugate according to any one of [1] to [33] as an active ingredient,
   wherein the therapeutic agent or the preventive agent is for a disease selected from the group consisting of inborn errors of metabolism, a congenital endocrine disease, a single gene disorder, a neurodegenerative disease, a neurologic disease, a myopathy, a meningitis, an encephalitis, an encephalopathy, a lysosome disease, a malignant neoplasm, a fibrosis, an inflammatory disease, an immunodeficiency disease, an autoimmune disease, and an infectious disease.
[36] A method for treating and/or preventing a disease selected from the group consisting of inborn errors of metabolism, a congenital endocrine disease, a single gene disorder, a neurodegenerative disease, a neurologic disease, a myopathy, a meningitis, an encephalitis, an encephalopathy, a lysosome disease, a malignant neoplasm, a fibrosis, an inflammatory disease, an immunodeficiency disease, an autoimmune disease, and an infectious disease, the method comprising:
   administering a therapeutically effective amount of the oligonucleotide conjugate according to any one of [1] to [33].
[37] A use of the oligonucleotide conjugate according to any one of [1] to [33], for producing a therapeutic agent and/or a preventive agent for a disease selected from the group consisting of inborn errors of metabolism, a congenital endocrine disease, a single gene disorder, a neurodegenerative disease, a neurologic disease, a myopathy, a meningitis, an encephalitis, an encephalopathy, a lysosome disease, a malignant neoplasm, a fibrosis, an inflammatory disease, an immunodeficiency disease, an autoimmune disease, and an infectious disease.
[38] The oligonucleotide conjugate according to any one of [1] to [33] for use in the treatment and/or prevention of a disease selected from the group consisting of inborn errors of metabolism, a congenital endocrine disease, a single gene disorder, a neurodegenerative disease, a neurologic disease, a myopathy, a meningitis, an encephalitis, an encephalopathy, a lysosome disease, a malignant neoplasm, a fibrosis, an inflammatory disease, an immunodeficiency disease, an autoimmune disease, and an infectious disease.
[39] A medicament comprising a combination of
   the oligonucleotide conjugate according to any one of [1] to [33]; and
   one or more therapeutic agents and/or one or more preventive agents for a disease,
   wherein the disease is selected from the group consisting of inborn errors of metabolism, a congenital endocrine disease, a single gene disorder, a neurodegenerative disease, a neurologic disease, a myopathy, a meningitis, an encephalitis, an encephalopathy, a lysosome disease, a malignant neoplasm, a fibrosis, an inflammatory disease, an immunodeficiency disease, an autoimmune disease, and an infectious disease.
[40] The oligonucleotide conjugate according to any one of [1] to [33] for treating a disease in combination with one or more therapeutic agents and/or one or more preventive agents for the disease,
   wherein the disease is selected from the group consisting of inborn errors of metabolism, a congenital endocrine disease, a single gene disorder, a neurodegenerative disease, a neurologic disease, a myopathy, a meningitis, an encephalitis, an encephalopathy, a lysosome disease, a malignant neoplasm, a fibrosis, an inflammatory disease, an immunodeficiency disease, an autoimmune disease, and an infectious disease.
[41] A method for producing the oligonucleotide conjugate according to any one of [1] to [33], the method comprising steps of
   bonding a plurality of oligonucleotides and one or more hydrophilic linkers to a dendritic polymer; and
   bonding a cellular internalization enhancer to each hydrophilic linker.
[42] The method for producing the oligonucleotide conjugate according to [41], further comprising a step of bonding a capping agent to the dendritic polymer.

### Advantageous Effects of Invention

According to the present invention, since the cellular internalization enhancer can efficiently interact with a target cell, the oligonucleotides can be efficiently transported into the cells and accordingly, the amount of oligonucleotides transported into cytoplasm can be improved. In addition, according to the present invention, intrinsic limitations in the structure of self-assembled nanoparticles, which are representative of conventional functional nanoparticles, can be avoided. For example, functional nanoparticles using liposomes or micelles are structurally unstable and can be dissociated by organic solvents, surfactants, dilution, shear stress, or interaction with biological components. In addition, it was difficult to precisely control the size of these particles below 50 nm. In contrast, the oligonucleotide conjugate according to the present invention has a stable structure and the size thereof can be easily controlled.

### Brief Description of Drawings

FIGS. 1(A) and 1(B) are schematic diagrams of one embodiment of an oligonucleotide conjugate, and in FIG. 1(B), a hydration layer formed around hydrophilic linkers is shown.
FIG. 2 is a graph showing *in vitro* cellular uptake of an oligonucleotide conjugate which contains cRGD and uses a fourth generation polylysine dendrigraft as a core.
FIG. 3 is a graph showing *in vitro* gene knockdown efficiency of an oligonucleotide conjugate which contains cRGD and uses a fourth generation polylysine dendrigraft as a core.
FIG. 4 is a graph showing *in vitro* nucleic acid sequence-specific gene knockdown efficiency of an oligonucleotide conjugate which contains cRGD and uses a fourth generation polylysine dendrigraft as a core.
FIG. 5 is a graph showing *in vitro* cellular uptake of an oligonucleotide conjugate which contains GE11 and uses a fourth generation polylysine dendrigraft as a core.
FIG. 6 is a graph showing *in vitro* gene knockdown efficiency of an oligonucleotide conjugate which contains GE11 and uses a fourth generation polylysine dendrigraft as a core.
FIG. 7 is a graph showing *in vitro* cellular uptake of oligonucleotide conjugates which contain cRGDs and use PAMAMs as cores.
FIG. 8 is a graph showing *in vitro* gene knockdown efficiency of oligonucleotide conjugates which contain cRGDs and use PAMAMs as cores.
FIG. 9 is a graph showing *in vitro* comparison of the number of cRGD modifications and the amount of cellular uptake of an oligonucleotide conjugate which contains cRGD and uses a fourth generation polylysine dendrigraft as a core.
FIG. 10 is a graph showing *in vitro* comparison of the number of cRGD modifications and gene knockdown efficiency of an oligonucleotide conjugate which contains cRGD and uses a fourth generation polylysine dendrigraft as a core.
FIG. 11 is a graph showing *in vitro* cellular uptake of an oligonucleotide conjugate which contains c(avb6) and uses a fourth generation polylysine dendrigraft as a core.
FIG. 12 is a graph showing *in vitro* gene knockdown efficiency of an oligonucleotide conjugate which contains c(avb6) and uses a fourth generation polylysine dendrigraft as a core.
FIG. 13 is a graph showing *in vitro* cellular uptake of an oligonucleotide conjugate which contains a folic acid and uses a fourth generation polylysine dendrigraft as a core.
FIG. 14 is a graph showing *in vitro* cellular uptake of oligonucleotide conjugates which contain nucleolin aptamers and use fourth generation polylysine dendrigrafts as cores.
FIG. 15 is a graph showing *in vitro* gene knockdown efficiency of oligonucleotide conjugates which contain nucleolin aptamers and use fourth generation polylysine dendrigrafts as cores.
FIG. 16 is a graph showing *in vitro* comparison of cellular uptake of oligonucleotide conjugates which contain cRGDs and use polylysine dendrigrafts of different generations as cores.
FIG. 17 is a graph showing *in vitro* comparison of gene knockdown efficiency of oligonucleotide conjugates which contain cRGDs and use polylysine dendrigrafts of different generations as cores.
FIG. 18 is a graph showing *in vitro* comparison of cellular uptake of oligonucleotide conjugates which contain cRGDs, use fourth generation polylysine dendrigrafts as cores, and contain PEGs, which are hydrophilic linkers, with a molecular weight of 2k, 3.4k, or 5k.
FIG. 19 is a graph showing *in vitro* comparison of cellular uptake of oligonucleotide conjugates which contain cRGDs, use fourth generation polylysine dendrigrafts as cores, and contain PEGs, which are hydrophilic linkers, with a molecular weight of 5k or 10k.
FIG. 20 is a graph showing *in vitro* cellular uptake of an oligonucleotide conjugate which contains cRGD, uses a fourth generation polylysine dendrigraft as a core, and contains pMeOx10k as a hydrophilic linker.
FIG. 21 is a graph showing *in vitro* gene knockdown efficiency of an oligonucleotide conjugate which contains cRGD, uses a fourth generation polylysine dendrigraft as a core, and contains pMeOxlOk as a hydrophilic linker.
FIG. 22 is a graph showing *in vitro* cellular uptake of an oligonucleotide conjugate which contains cRGD, uses a fourth generation polylysine dendrigraft as a core, and contains pSar10k as a hydrophilic linker.
FIG. 23 is a graph showing *in vitro* gene knockdown efficiency of an oligonucleotide conjugate which contains cRGD, uses a fourth generation polylysine dendrigraft as a core, and contains pSar10k as a hydrophilic linker.
FIG. 24 is a graph showing *in vitro* comparison of cellular uptake of oligonucleotide conjugates which contain cRGDs, use fourth generation polylysine dendrigrafts as cores, and are modified with different capping agents.
FIG. 25 is a graph showing *in vitro* comparison of gene knockdown efficiency of oligonucleotide conjugates which contain cRGDs, use fourth generation polylysine dendrigrafts as cores, and are modified with different capping agents.
FIG. 26 is a graph showing *in vitro* comparison of cellular uptake of oligonucleotide conjugates which contain cRGDs, use fourth generation polylysine dendrigrafts as cores, and are modified with capping agents having protonation abilities.
FIG. 27 is a graph showing *in vitro* comparison of gene knockdown efficiency of oligonucleotide conjugates which contain cRGDs, use fourth generation polylysine dendrigrafts as cores, and are modified with capping agents having protonation abilities.
FIG. 28 is a graph showing comparison of pH sensitivity of fourth generation polylysine dendrigrafts modified with capping agents having protonation abilities.

### Description of Embodiments

Hereinafter, preferred embodiments of the present invention will be described.

An oligonucleotide conjugate according to an aspect of the present invention contains a dendritic polymer, a plurality of oligonucleotides, one or a plurality of cellular internalization enhancers, and one or a plurality of hydrophilic linkers, wherein each oligonucleotide is bonded to the dendritic polymer directly or through a linker, and each cellular internalization enhancer is bonded to the dendritic polymer through the hydrophilic linker. In the present specification, an oligonucleotide conjugate means a single molecule formed by conjugating oligonucleotides with other molecules.

In the present specification, a dendritic polymer means a polymer branched from the center in a dendritic manner and having regularity in branching. A dendritic polymer may be a dendrimer, dendron, or dendrigraft. Dendrimers are generally three-dimensionally highly branched molecules with a dendritic structure, and have an approximately spherical shape. A dendron has a structure in which at least one functional group in the center part of the dendrimer is unbranched. Dendrimers and dendrons have a regular branched structure, and the repeating units thereof are called "generation". In a dendrigraft, molecular chains are bonded in a comb-like manner to the side chains on the backbone, and further, molecular chains are bonded in a comb-like manner to the side chains of the comb-like molecular chains, thereby forming a structure that spreads in a radial shape. In the case of dendrigrafts, comb-like repeating units are called "generation".

The generation of the dendritic polymer is preferably third to twentieth generation. For example, in the case of a polyamidoamine (PAMAM) dendrimer with an ethylenediamine core, the generation is preferably fifth to twentieth generation, more preferably fifth to tenth generation. In the case of a polylysine dendrigraft, the generation is preferably third to sixth generation, more preferably third to fifth generation. In the case of a 2,2-bis(hydroxyl-methyl)propionic acid (Bis-MPA) dendrimer, the generation is preferably fourth to twentieth generation, more preferably fourth to tenth generation.

The average diameterof the dendritic polymer is preferably 5 nm or more, more preferably 5 nm to 25 nm, still more preferably 5 nm to 15 nm. In the present specification, the average diameter of the dendritic polymer means the average diameter in the particle size distribution measured by dynamic light scattering.

Monomers in the dendritic polymer may be bonded to each other by bonding types such as a single bond, a double bond, a triple bond, a carbon-silicon bond, an amide bond, a glycosidic bond, an ester bond, an ether bond, a urethane bond, an acetal bond, a phosphate ester bond, a thioether bond, a thioester bond, a disulfide bond, a triazole bond, a hydrazone bond, a hydrazide bond, an imine or oxime bond, a urea or thiourea bond, an amidine bond, or a sulfonamide bond, but bonding types are not limited to them. Although any of these bonding types may be used, from the viewpoint of safety, those of which bonds are cleaved by an enzyme, or of which bonds are cleaved under certain *in vivo* conditions such as an acidic condition or a reducing condition are preferable. Examples of preferable bonding types are an amide bond, an ester bond, or a glycosidic bond, but bonding types are not limited to them.

Examples of suitable dendritic polymers include polylysine dendrimers, polylysine dendrigrafts, PAMAM dendrimers, Bis-MPA dendrimers, or glucose dendrimers, but dendritic polymers are not limited to them. A dendritic polymer may be, for example, a poly-L-lysine dendrimer or a poly-L-lysine dendrigraft.

In the present specification, an oligonucleotide is a polymer of which a repeating unit is a nucleotide consisting of a base, a sugar and a phosphoric acid. The type of oligonucleotide is not particularly limited, and the oligonucleotide conjugate may contain one or two or more types of oligonucleotides. Examples of oligonucleotides include single-stranded or double-stranded RNA, DNA, or combinations thereof, and also include oligonucleotides in which RNA and DNA are mixed on the same strand. Nucleotides contained in the oligonucleotide may be natural nucleotides or chemically modified non-natural nucleotides, and may be nucleotides to which amino groups, thiol groups, or molecules such as fluorescent compounds are bonded. The oligonucleotide may be a non-natural oligonucleotide, and examples of the non-natural oligonucleotide include artificial molecules, such as a peptide nucleic acid (PNA) having a peptide structure in the backbone, or a morpholino nucleic acid having a morpholine ring in the backbone, that have the similar effect as natural oligonucleotides in controlling gene expression.

The function or action of oligonucleotides is not limited, but examples of oligonucleotides include antisense oligonucleotides, sgRNA, RNA editing nucleic acids, miRNA, siRNA, saRNA, shRNA, or dicer substrate RNA.

An oligonucleotide may be, for example, a gene expression modifier. Gene expression modifiers are compounds that activate or inhibit the expression of specific gene products. Examples of gene products include mRNA or precursors thereof, miRNA or precursors thereof, ncRNA, enzymes, antibodies, or other proteins. Examples of such gene expression modifiers include molecules that positively or negatively regulate mRNA expression (that is, activate or inhibit expression), molecules that edit RNA, and molecules that edit DNA. Examples of such gene expression modifiers include nucleic acids that induce RNA interference (RNAi) such as miRNA or siRNA (RNAi inducers), antisense oligonucleotides, miRNA inhibitors, RNA activating nucleic acids, RNA editing-inducing nucleic acids, or nucleic acids necessary to induce genome editing, but gene expression modifiers are not limited to them.

The lengths of oligonucleotides may be, for example, 4 to 200 bases (pairs), 7 to 100 bases (pairs), or 12 to 30 bases (pairs).

The number of oligonucleotides in the oligonucleotide conjugate is not particularly limited, and for example, may be 1 or more, 2 or more, 6 or more, 10 or more, 18 or more, 20 or more, 21 or more, 25 or more, 26 or more, 28 or more, 35 or more, or 50 or more, and may be 400 or less, 200 or less, or 100 or less. When the oligonucleotides are covalently bonded to the dendritic polymer, the number of oligonucleotides may be, for example, 1 or more, or 0.5% or more, 1% or more, or 2% or more of the reactive functional groups of the dendritic polymer, more preferably 3% or more or 5% or more of the reactive functional groups of the dendritic polymer. The number of oligonucleotides in the oligonucleotide conjugate may be determined, for example, by measuring the concentration of dendritic polymer and the concentration of oligonucleotide in the solution containing the oligonucleotide conjugate, and calculating the ratio of the oligonucleotide to the dendritic polymer based on these values. The concentration of dendritic polymer in the solution containing the oligonucleotide conjugate may be measured, for example, by high performance liquid chromatography (HPLC). The concentration of the oligonucleotide may be determined, for example, from absorbance at 260 nm measured using an ultraviolet-visible spectrophotometer.

Oligonucleotides may be produced as described anywhere in the literature. Oligonucleotides may be produced, for example, by the phosphoramidite chemistry or the triester chemistry in a solid-phase synthesis or a liquid-phase synthesis with or without automated oligonucleotide synthesizers.

Each oligonucleotide is bonded to a dendritic polymer either directly or through a linker. The linker that links the dendritic polymer and the oligonucleotide is not particularly limited and may be a known linker such as polyethylene glycol (PEG). The oligonucleotide conjugate may contain one or two or more types of the linker. From the viewpoint of allowing the cellular internalization enhancer to efficiently interact with a target cell to improve the transport efficiency of the oligonucleotide conjugate into the cell, the average linear distance between the ends of a linker that links the dendritic polymer and the oligonucleotide is preferably shorter than the average linear distance between the ends of a hydrophilic linker that links the dendritic polymer and the cellular internalization enhancer. In one example, when the linker that links the dendritic polymer and the oligonucleotide is PEG, the number average molecular weight thereof may be 1000 or less, 800 or less, 600 or less, or 300 or less.

In the present specification, a hydrophilic linker is a hydrophilic molecule for linking the dendritic polymer and the cellular internalization enhancer. A hydrophilic molecule means a molecule that easily forms a hydrogen bond with water and is easily dissolved or mixed with water. Hydrophilic molecules may be charged molecules or uncharged highly polar molecules. The charged groups of the charged molecules may be positively charged groups (cations), negatively charged groups (anions), or a combination thereof. The hydrophilicity of the hydrophilic linker for linking the dendritic polymer and the cellular internalization enhancer is advantageous from the viewpoint of suppressing aggregation, improving solubility, avoiding phagocytosis by the reticuloendothelial system, avoiding non-specific interactions with biological components, and improving pharmacokinetics (that is, prolonging blood circulation time) of the oligonucleotide conjugate. Examples of hydrophilic linkers include PEG, poly(2-alkyl-2-oxazoline), polypeptide, polypeptoid, or polybetaine, but hydrophilic linkers in the present invention are not limited to them. Oligonucleotide conjugates may contain one or two or more kinds of hydrophilic linkers. The hydrophilic linker is preferably one or two or more types selected from the group consisting of PEG, poly(2-methyl-2-oxazoline) (pMeOx), polysarcosines (pSar), and EK peptides. The EK peptide herein is a peptide comprised from alternating glutamic acid and lysine.

A single hydrophilic linker may have multiple segments. Examples of hydrophilic linkers having multiple segments include polymers formed by bonding EK peptides and PEG, but the hydrophilic linkers having multiple segments are not limited to them. A hydrophilic linker may have a linear structure or a branched structure.

In one example, when an oligonucleotide with a length of 12 to 30 bases (pairs) is bonded to the dendritic polymer directly or through PEG having a number average molecular weight of 800 or less, and the hydrophilic linker is PEG, from the viewpoint of allowing the cellular internalization enhancer to efficiently interact with a target cell to improve the transport efficiency of the oligonucleotide conjugate into the cell, the number average molecular weight of the hydrophilic linker is 2000 or more, 3400 or more, 5000 or more, 6000 or more, 8000 or more, or 10000 or more. Alternatively, when the hydrophilic linker is pMeOx or pSar, the number average molecular weight of the hydrophilic linker may be 4000 or more, 7000 or more, 10000 or more, 15000 or more, or 20000 or more. Alternatively, when the hydrophilic linker is an EK peptide, the repeating number of glutamic acid and lysine unit may be 5 or more, 7 or more, 10 or more, 15 or more, or 20 or more. In the present specification, the number average molecular weight is a value determined by an end-group analysis method using nuclear magnetic resonance (NMR) or a size exclusion chromatography (SEC) method.

The number of hydrophilic linkers may be set according to the type and number of cellular internalization enhancers. The number of hydrophilic linkers may be less than, more than, or the same as the number of cellular internalization enhancers. The number of hydrophilic linkers covalently bonded to the dendritic polymer may be, for example, 1 or more, 2 or more, or 1% or more of the reactive functional groups of the dendritic polymer, preferably 2% or more, more preferably 3% or more or 5% or more of the reactive functional groups of the dendritic polymer.

In the present specification, the cellular internalization enhancer is a molecular species that interacts specifically or non-specifically with a target cell to induce the internalization of a substance to which the cellular internalization enhancer is bonded into the target cell. In the oligonucleotide conjugate according to the present aspect, by bonding the cellular internalization enhancer to the dendritic polymer through a hydrophilic linker, the oligonucleotide can be efficiently transported into a target cell as compared with the case where the cellular internalization enhancer is not contained (for example, the case where the oligonucleotide is used alone). Examples of cellular internalization enhancers include substances that interact with cell surface receptors, substances that interact with membrane transporters, substances that interact with cell adhesion factors, and other substances that interact with the cell membrane surface, but the cellular internalization enhancers are not limited to them. Examples of cellular internalization enhancers include substances that interact with integrins, which are cell adhesion factors present on the cell membrane surface, substances that interact with epithelial cell adhesion molecules, substances that interact with a nucleolin, substances that interact with a vimentin, which is a cytoskeletal element, substances that interact with prostate-specific membrane antigens, substances that interact with cell surface receptors such as epidermal growth factor receptors, somatostatin receptors, mannose receptors, asialoglycoprotein receptors, or folate receptors, or substances that interact with transporters such as glucose transporters or non-selective monoamine transporters.

Examples of cellular internalization enhancers include hydrophobic molecules, polycations, small-molecule ligands, polypeptides, aptamers, antibodies or fragments thereof, saccharides, or lipids, but cellular internalization enhancers are not limited to them. The oligonucleotide conjugate may contain one or two or more kinds of cellular internalization enhancers. Cellular internalization enhancers are preferably one or two or more types selected from the group consisting of small-molecule ligands, polypeptides, aptamers, and saccharides. Cellular internalization enhancers are more preferably polypeptides, small-molecule ligands, or aptamers.

The molecular weight of the polypeptide may be, for example, 50 kDa or less, 15 kDa or less, 6 kDa or less, 2 kDa or less, or 1 kDa or less, but is not limited to them. The molecular weight of the polypeptide may be determined, for example, by mass spectrometry.

In the present specification, an antibody or fragment thereof refers to a scaffold protein that has an ability to specifically bind to a particular factor, and includes, but is not limited to, immunoglobulins such as IgA, IgD, IgE, IgG, or IgM, fragmented antibodies such as F(ab)'2, Fab', Fab, or scFv, single domain antibodies such as shark VNAR or camel VHH, and antibody mimetics such as affibodies, affilins, monobodies, or alphabodies.

Specific examples of cellular internalization enhancers include polypeptides shown in the following Formulas (I) to (IV). The polypeptide shown in Formula (I) is cRGDfK (molecular weight: 603.7 Da, Pharmaceutics, 2018, 10, 2), which is a type of cyclic peptide ligand containing an arginine-glycine-aspartic acid sequence (cRGD), which interacts with integrin α_{V}β₃. cRGD other than cRGDfK can also be used as a cellular internalization enhancer. The polypeptide shown in Formula (II) is c(avb6) (molecular weight: 1046.2, ACS Omega, 2018, 3, 2428-2436), which interacts with integrin α_{V}β₆. The polypeptide shown in Formula (III) is GE11 (molecular weight: 1539.7 Da), which interacts with epidermal growth factor receptors. The polypeptide shown in Formula (IV) is an octreotide derivative (OCT; molecular weight: 1577.8 Da), which interacts with somatostatin receptors. Commercially available products may be used as cRGD, and peptides shown in Formulas (II) to (IV) are readily available by well-known synthetic methods.

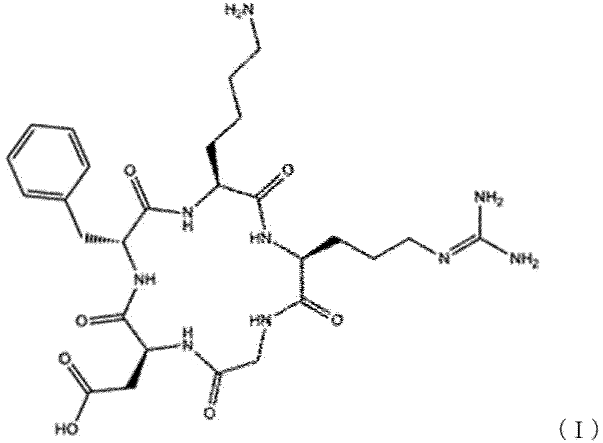

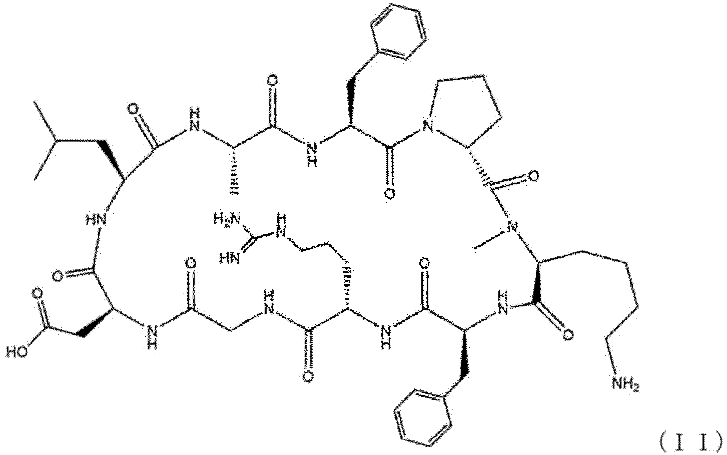

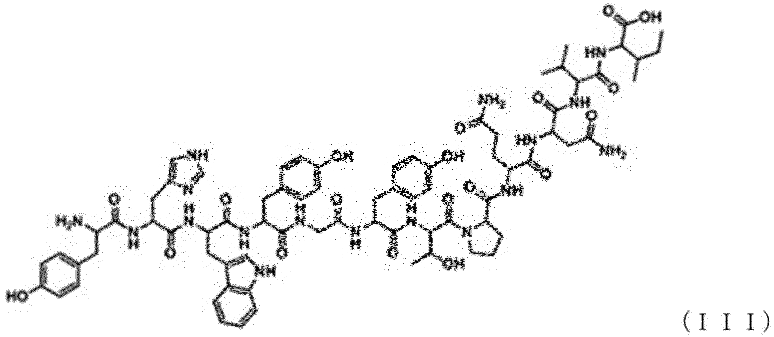

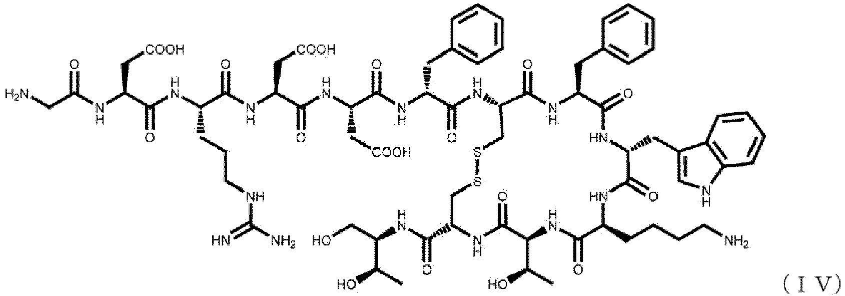

Specific examples of other cellular internalization enhancers include small molecules shown in the following Formulas (V) to (VII). The small molecule shown in Formula (V) is folic acid, which interacts with folate receptors. The small molecule shown in Formula (VI) is DUPA, which interacts with prostate-specific membrane antigens. The small molecule shown in Formula (VII) is indatraline (IND), which interacts with non-selective monoamine transporters.

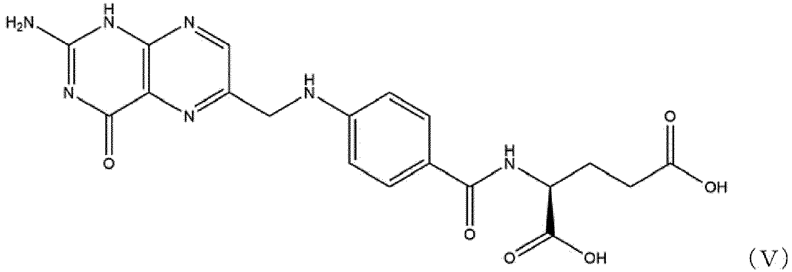

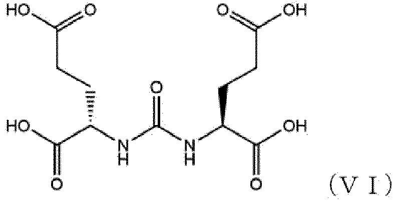

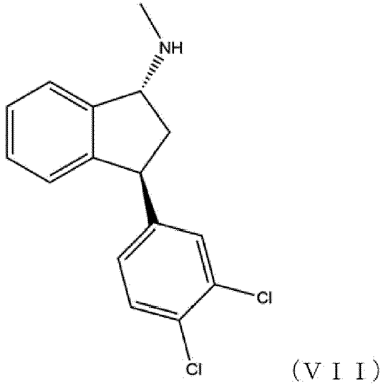

Specific examples of other cellular internalization enhancers include saccharides shown in the following Formulas (VIII) to (XII). The saccharide shown in Formula (VIII) is glucose (Glu), which interacts with glucose transporters. The saccharide shown in Formula (IX) is mannose (Man), which interacts with mannose receptors. The saccharides shown in Formulas (X) and (XI) are N-acetylgalactosamine (GalNAc) and galactose (Gal), which interact with asialoglycoprotein receptors. The saccharide shown in Formula (XII) is N-acetylglucosamine (GlcNAc), which interacts with a cytoskeletal element vimentin.

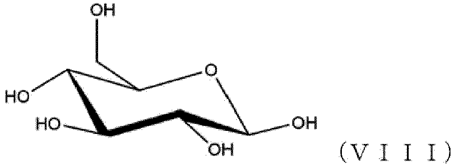

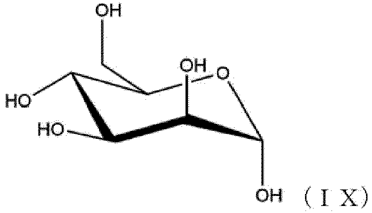

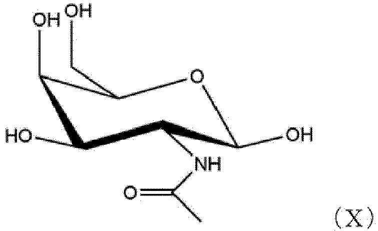

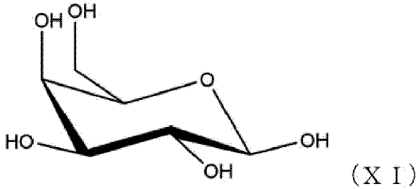

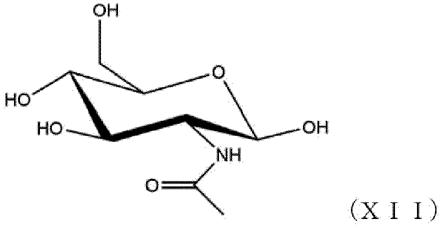

Examples of other cellular internalization enhancers include aptamers having the nucleotide sequences represented by SEQ ID NO: 1 to 6 shown in the table below. Examples of DNA aptamers that interact with nucleolin include AS1411 shown in SEQ ID NO: 1 (Oncotarget, 2015, 6(26), 22270-22281) and FAN-1524dI shown in SEQ ID NO: 2 (Scientific Reports, 2016, 6, 1-12). Examples of aptamers that interact with epithelial cell adhesion molecules include EpCAM Aptamer shown in SEQ ID NO: 3 (Molecular Cancer Therapeutics, 2015, 14 (10), 2279-2291) and EpCAM Aptamer shown in SEQ ID NO: 4 (Theranostics, 2015, 5(10), 1083-1097). Examples of aptamers that interact with transferrin receptors include FB4 shown in SEQ ID NO: 5 (Proc Natl Acad Sci USA., 2008, 105(41), 15908-15913) and GS24 shown in SEQ ID NO: 6 (Mol Ther Nucleic Acids, 2014, 3(1), e144).

**[Table 1]**

| SEQ ID NO | Sequence (from 5' to 3') |
|---|---|
| 1 | ggtggtggtggttgtggtggtggtgg |
| 2 | ggtggtggtggttgiggtggtggigg |
| 3 | GC(F)GAC(F)U(F)GGU(F)U(F)AC(F)C(F)C(F)GGU(F)C(F)GU(F) U(F)U(F) |
| 4 | cgcgcgccgcAC(F)GU(F)AU(F)C(F)C(F)C(F)U(F)U(F)U(F)U(F)C(F )GC(F)GU(F)Acggcgcgcg |
| 5 | GGGCGAAUUCCGCGUGUGCUGAGGGCGGAAGAACUAAUU UGGGACGGAUUGCGGCCGUUGUCUGUGGCGUCCGUUCGG G |
| 6 | gcgtgtgcacacggtcacttagtatcgctacgttctttggttccgttcgg |

| | |
|---|---|
| In the table: lower case = DNA, upper case = RNA, (F) = 2'-F substitution | |

From the viewpoint of allowing the cellular internalization enhancer to efficiently interact with a target cell to improve the transport efficiency of the oligonucleotide conjugate into the cell, the number of cellular internalization enhancers in the oligonucleotide conjugate may be, for example, 1 or more, 2 or more, 6 or more, 12 or more, 18 or more, 25 or more, or 26 or more, and may be 400 or less, 200 or less, or 100 or less. The number of cellular internalization enhancers in the oligonucleotide conjugate may be determined, for example, by measuring the concentration of dendritic polymer and the concentration of cellular internalization enhancer in the solution containing the oligonucleotide conjugate, and based on these values, calculating the ratio of the cellular internalization enhancer to the dendritic polymer. The concentration of dendritic polymer and the concentration of cellular internalization enhancer concentration may be measured, for example, by HPLC or ultraviolet-visible spectrophotometer.

As described above, oligonucleotides or hydrophilic linkers are bonded to the dendritic polymer, more specifically, at least some of the reactive functional groups (these are terminal functional groups) of the dendritic polymer. In one embodiment, at least some or all of the unreacted reactive functional groups that are not bonded to the oligonucleotide and hydrophilic linker may be capped with a capping agent. Capping a reactive functional group is, in other words, reducing the reactivity of the reactive functional group by bonding. Capping agents protect the dendritic polymer from various interactions or chemical reactions, by capping the reactive functional groups of the dendritic polymer. For example, capping agents protect the dendritic polymer from electrostatic interactions, degradation reactions, condensation reactions, addition reactions, and the like.

In addition, the capping agent, by bonding to the dendritic polymer, can add functions or activities that the dendritic polymer does not originally have to the dendritic polymer. Examples of such capping agents include molecules that improve stealth properties, molecules that interact with lipid bilayer membranes, and molecules that have proton buffering capacity, but capping agents are not limited to them.

The capping agent may be, for example, one or two kinds of molecules selected from the group consisting of a) hydrophilic molecules and b) hydrophobic molecules.

The a) hydrophilic molecule may be a-1) an electrically neutral hydrophilic molecule, a-2) a polar molecule that protonates under acidic conditions, a-3) an anionic molecule, or a-4) a cationic molecule, a) The hydrophilic molecule may be of the same molecular species as the hydrophilic linker or may be of a different molecular species than the hydrophilic linker. In the present specification, "electrically neutral" indicates that the number of cations and anions is equal, or the difference in the number of cations and anions is within 10% of the number of larger numbers of charged groups.

Examples of the above-mentioned a-1) electrically neutral hydrophilic molecules include molecules having hydrophilic groups such as hydroxyl groups, alkoxy groups, oxime groups, ester groups, amide groups, imide groups, alkoxyamide groups, carbonyl groups, sulfonyl groups, nitro groups, or pyrrolidone groups; zwitterion such as betaine; PEG; and alkoxy polyethylene glycol such as methoxypolyethylene glycol, but the hydrophilic molecules are not limited to them.

The above a-2) polar molecules that are protonated under acidic conditions are molecules that have different charges under acidic conditions such as in endosomes and under physiological conditions such as in blood or interstitial fluid. A polar molecule that is protonated under acidic conditions refers to a molecule that has an acid dissociation constant (pKa) of 7.4 or less, preferably 5.0 to 7.4. Examples of polar molecules that are protonated under acidic conditions include molecules having polar groups such as tertiary amino groups, diethyltriamine (DET) groups (-NH-CH₂-CH₂-NH-CH₂-CH₂-NH₂), morpholino groups, thiomorpholino groups, imidazolyl groups, pyridyl groups, or carboxy groups, but polar molecules that are protonated under acidic conditions are not limited to them.

The above a-3) anionic molecule is a negatively charged molecule under physiological conditions. Examples thereof include molecules having functional groups such as a carboxy group, a sulfo group, a phosphate group, or a phosphate ester group, but anionic molecules are not limited to them.

The above a-4) cationic molecule is a positively charged molecule under physiological conditions. Examples thereof include molecules having functional groups such as primary amino groups, secondary amino groups, tertiary amino groups, or guanidino groups, but cationic molecules are not limited to them.

The above b) hydrophobic molecule means a molecule that hardly forms a hydrogen bond with water and has a low affinity for water. Hydrophobic molecules may be non-polar molecules or molecules with a partition coefficient of 2.0 or greater. Examples of hydrophobic molecules include molecules having hydrophobic groups such as aliphatic compounds, trialkylamine aromatic groups, or cholesterol or steroids, but the hydrophobic molecules are not limited to them.

In the present specification, "bond" refers to direct or indirect, irreversible bond. An irreversible bond refers to a bond of which reaction does not proceed reversibly, that is, a bond that, once formed, does not dissociate by a reverse reaction or a bond that dissociates due to a reverse reaction to a negligible extent. The bond between the dendritic polymer and the oligonucleotide or the linker bonded to the oligonucleotide, the bond between the oligonucleotide and the linker, the bond between the dendritic polymer and the hydrophilic linker, and the bond between the cellular internalization enhancer and the hydrophilic linker may be, for example, covalent bonds resulting from chemical reactions such as nucleophilic addition reactions, nucleophilic substitution reactions, or electrophilic substitution reactions between functional groups, metal coordination bonds such as a bond between ammonia and platinum, or host-guest interaction such as a bond between biotin and avidin. From the viewpoint of achieving high structural stability and controlling the size of the oligonucleotide conjugate, the bonds are preferably covalent bonds.

Examples of covalent bonds include single bond, double bond, triple bond, amide bond, glycosidic bond, ester bond, ether bond, urethane bond, acetal bond, phosphate ester bond, thioether bond, thioester bond, disulfide bond, triazole bond, hydrazone bond, hydrazide bond, imine or oxime bond, urea or thiourea bond, amidine bond, sulfonamide bond, or bond formed by inverse electron demand Diels-Alder reaction, but the covalent bonds are not limited to them.

An amide bond is formed between a carboxy group and an amino group. Amide bonds are formed using conventional amide bond formation reactions, for example, between a suitably protected amino group and an activated carboxylic acid (such as a N-hydroxysuccinimide-activated ester).

A disulfide bond (-S-S-) is formed, for example, by thiol exchange between a component containing a thiol group (also called a mercaptan group) (-SH) and an activated thiol group of another component.

A thioether bond (-S-) is formed, for example, using a conventional thioether bond formation reaction that occurs between a thiol group and a maleimide group.

A triazole bond is formed between an azide group and a carbon-carbon triple bond. A triazole bond is formed, for example, by so-called click chemistry, such as Huisgen cycloaddition using a metal catalyst or strain-promoted alkyne-azide cycloaddition without using a metal catalyst.

Metal coordination is a bonding type in which a metal ion and a ligand are bonded by forming a complex. Examples of metal ions include, but are not limited to, ions of metal elements such as platinum group elements, manganese, cobalt, copper, or gadolinium. Examples of ligands include, but are not limited to, ammonia, pyridine, bipyridine, ethylenediamine, ethylenediaminetetraacetic acid, acetylacetonate, and derivatives thereof.

A host-guest interaction is an interaction between a host molecule, which is a molecule that provides a space in which a particular molecule can be selectively recognized, and a guest molecule, which is a molecule that is accepted therein. Examples of host molecules include, but are not limited to, cyclodextrin, carcerand, cavitand, crown ether, cryptand, cucurbituril, calixarene, avidin, and streptavidin. Examples of guest molecules include, but are not limited to, adamantane, diadamantane, cholesterol, naphthalene, and biotin.

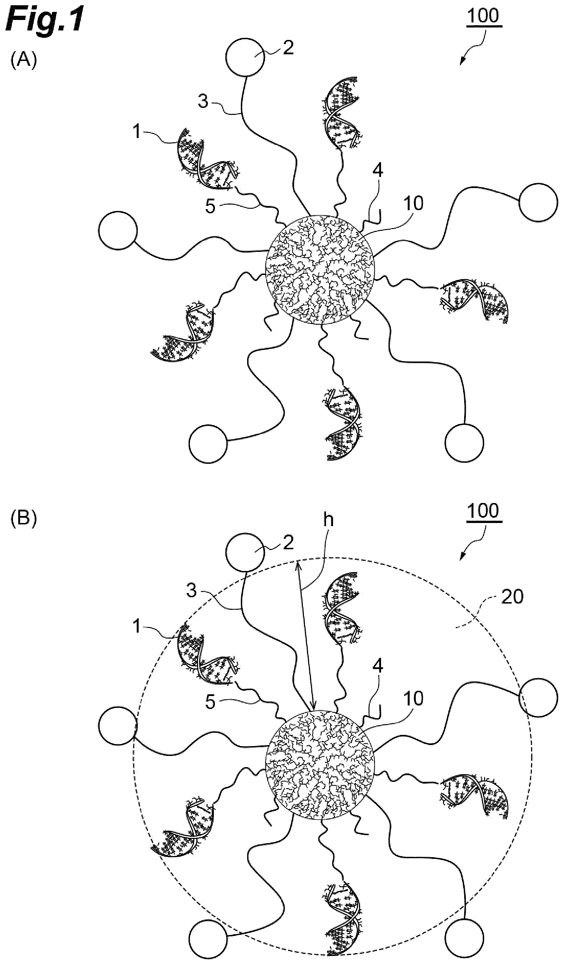

Next, the structure of the oligonucleotide conjugate will be described with reference to FIG. 1. FIG. 1(A) is a schematic diagram showing one embodiment of an oligonucleotide conjugate. An oligonucleotide conjugate 100 includes a core 10 of the dendritic polymer, and a plurality of oligonucleotides 1, cellular internalization enhancers 2, and hydrophilic linkers 3, that are arranged around the core 10. The oligonucleotides 1 are bonded to the core 10 through linkers 5. The hydrophilic linkers 3 are bonded to the core 10, and the cellular internalization enhancers 2 are bonded to the hydrophilic linkers 3. In addition, capping agents 4 are also bonded to the core 10. Since all the components of the oligonucleotide conjugate 100 other than the dendritic polymer are bonded to the dendritic polymer in this manner, the dendritic polymer constitutes the "core" 10, that is, the center part of the oligonucleotide conjugate 100. In addition, in an aqueous solution, the oligonucleotides 1 and the hydrophilic linkers 3 extend substantially radially from the core 10, and accordingly, the oligonucleotide conjugate 100 takes the shape of a substantially spherical nanoparticle. Thus, the oligonucleotide conjugate 100 exhibits the behavior of a nanoparticle. In this field, there is a wealth of knowledge regarding the behavior of nanoparticles *in vivo.* Note that, although the oligonucleotides 1 are bonded to the core 10 through the linkers 5 in FIG. 1, the oligonucleotides 1 may be bonded directly to the core 10 as described above.

The average particle diameter of the oligonucleotide conjugate 100 is preferably 10 to 100 nm, more preferably 15 to 45 nm, still more preferably 15 to 35 nm. In the present specification, the average particle diameter of the oligonucleotide conjugate means the average particle diameter in the particle size distribution obtained by dynamic light scattering. Since the oligonucleotide conjugate 100 has a dendritic polymer as the core 10, size control is easy and precise design is possible.

In order for the oligonucleotide conjugate 100 to be transported into a cell, the cellular internalization enhancer 2 needs to interact with the cell. From the viewpoint of improving the transport efficiency of the oligonucleotide conjugate into the cell, the density of the cellular internalization enhancers 2 is preferably high. According to the oligonucleotide conjugate 100, since the cellular internalization enhancers 2 are bonded to the core 10 of the highly branched dendritic polymer, a high density of the cellular internalization enhancers 2 can be achieved, and accordingly, the cellular internalization enhancers 2 can efficiently interact with a target cell.

Moreover, in order for the cellular internalization enhancer 2 to interact with a cell, the cellular internalization enhancer 2 is preferably present at the outer part of the nanoparticle of the oligonucleotide conjugate 100. In one embodiment, more preferably, as shown in FIG. 1(A), the cellular internalization enhancer 2 is present at the outermost part of a true sphere that approximates the structure of the oligonucleotide conjugate 100, that is, on the surface of the nanoparticle of the oligonucleotide conjugate 100. In another embodiment, even when the cellular internalization enhancer 2 is not present at the outermost part of the true sphere that approximates the structure of the oligonucleotide conjugate 100, it is preferable that the spatial extent (radius of gyration) of the hydrophilic linkers 3 is not completely enclosed by the spatial extent of the nucleic acids 1 and the hydrophilic linkers 3 are substantially exposed to the outside. In the oligonucleotide conjugate 100 according to the present aspect of the present invention, since the linker for linking the dendritic polymer and the cellular internalization enhancer is hydrophilic, non-specific interaction is suppressed, and the cellular internalization enhancer 2 is likely to be present at the outer part of nanoparticles of the oligonucleotide conjugate 100. The position of the cellular internalization enhancer 2 in the nanoparticle of the oligonucleotide conjugate 100 may be adjusted by the type and length of the hydrophilic linker 3. From the viewpoint of allowing the cellular internalization enhancer 2 to efficiently interact with a target cell to improve the transport efficiency of the oligonucleotide conjugate into the cell, the average linear distance between the ends of each hydrophilic linker 3 may be 1/5 or more, 1/4 or more, 1/3 or more, 2/5 or more, or half or more of the length of the oligonucleotide 1. Here, the linear distance between the ends of each hydrophilic linker 3 is the linear distance between the end bonded to the core 10 and the end bonded to the cellular internalization enhancer 2 of each hydrophilic linker 3. More precisely, the average linear distance between the ends of each hydrophilic linker 3 may be preferably 1/5 or more, 1/4 or more, 1/3 or more, 2/5 or more, or half or more of the average linear distance from the surface of the core 10 to the free end of the oligonucleotide 1. Here, the linear distance from the surface of the core 10 to the free end of the oligonucleotide 1 indicates the linear distance between the end of the linker 5 bonded to the core 10 (however, in the case where the oligonucleotide 1 is directly bonded to the core 10, the end of oligonucleotide 1 bonded to core 10) and the end of oligonucleotide 1 that is not bonded to the linker 5 or the core 10. For example, when the linear length of the oligonucleotides 1 is 5 nm and the oligonucleotides 1 are directly bonded to the core 10, the average linear distance between the ends of each hydrophilic linker 3 may be 1 nm or more, 1.25 nm or more, 1.67 nm, 2 nm or more, or 2.5 nm or more.

The average linear distance between the ends of each hydrophilic linker 3 may be determined by measuring the thickness of the hydration layer formed by the presence of the hydrophilic linkers 3 in some cases. The hydration layer will now be described with reference to FIG. 1(B). Since the hydrophilic linkers 3 are hydrophilic, water molecules are fixed between the hydrophilic linkers 3 (that is, the oligonucleotide conjugate 100 is hydrated) in an aqueous solution, thereby forming a layer of water molecules, namely a hydration layer 20, is formed around the hydrophilic linkers 3. Although depending on the type of hydrophilic linker, a thickness h of the hydration layer 20 formed around a given hydrophilic linker 3 may be equal or substantially equal to the linear distance between the ends of that hydrophilic linker 3 in some cases. Therefore, in such a case (for example, when the hydrophilic linker 3 is PEG), the average linear distance between the ends of each hydrophilic linker 3 can be defined as the average value of the thickness h of the hydration layer 20. The average value of the thickness h of the hydration layer 20 may be determined by multi-angle dynamic light scattering, for example. More specifically, first, a series of nanoparticle compounds each containing a core 10 of a dendritic polymer and a plurality of hydrophilic linkers 3 bonded to the core 10, wherein the molecular weight of the hydrophilic linker 3 of each nanoparticle compound is different from that of the hydrophilic linker 3 of other nanoparticle compounds, are prepared. Next, the average particle diameter of each nanoparticle compound is measured by multi-angle dynamic light scattering, and from the difference in the average particle diameter and the difference in the molecular weight of the hydrophilic linker 3, the correlation function between the molecular weight of the hydrophilic linker 3 and the thickness of the hydration layer is determined. Based on this correlation function, the thickness h of the hydration layer 20 of the oligonucleotide conjugate 100 can be calculated from the molecular weight of the hydrophilic linker 3 in the oligonucleotide conjugate 100.

The oligonucleotide conjugate may be a free body or a pharmaceutically acceptable salt. The oligonucleotide conjugate may be either a solvate (for example, hydrates, ethanol solvates, or propylene glycol solvates) or a non-solvate. Pharmaceutically acceptable salts may be acid addition salts or base addition salts. Examples of acid addition salts include salts with organic acids such as formate, acetate, trifluoroacetic acid (TFA), propionate, succinate, lactate, malate, adipate, citrate, tartrate, methanesulfonate, fumarate, maleate, p-toluenesulfonate, or ascorbate; and salts with inorganic acids such as hydrochloride, hydrobromide, sulfate, nitrate, or phosphate. Examples of base addition salts include alkali metal salts such as sodium salts or potassium salts; alkaline earth metal salts such as calcium salts or magnesium salts; ammonium salts; trimethylamine salts; triethylamine salts; aliphatic amine salts such as dicyclohexylamine salts, ethanolamine salts, diethanolamine salts, triethanolamine salts, or brocaine salts; aralkylamine salts such as N,N-dibenzylethylenediamine; heterocyclic aromatic amine salt such as pyridine salts, picoline salts, quinoline salts, or isoquinoline salts; quaternary ammonium salts such as tetramethylammonium salts, tetraethylammonium salts, benzyltrimethylammonium salts, benzyltriethylammonium salts, benzyltributylammonium salts, methyltrioctylammonium salts, or tetrabutylammonium salts; and basic amino acid salts such as arginine salts or lysine salts.

The present invention also provides a method for producing the oligonucleotide conjugate according to the above aspect. Namely, one aspect of the present invention is a method for producing the oligonucleotide conjugate including steps of bonding a plurality of oligonucleotides and one or more hydrophilic linkers to a dendritic polymer; and bonding a cellular internalization enhancer to each hydrophilic linker. Oligonucleotides may be bonded to the dendritic polymer either directly or through linkers. In one embodiment, the method for producing an oligonucleotide conjugate may further include a step of bonding a capping agent to the dendritic polymer. This allows for the production of an oligonucleotide conjugate in which at least some of the reactive functional groups of the dendritic polymer are capped with a capping agent.

Any of the above steps may be performed using a methodcommonly used in this field. Examples of such methods include a method in which an amino group and a carboxy group are allowed to react using an activating group to form an amide bond, a method in which thiol groups are allowed to react with each other using an activating group to form a disulfide bond, a method in which a thiol group and a maleimide group are allowed to react to form a thioether bond, a method in which a click chemistry using a catalyst or an activating group is used to form a triazole bond from an azide group and an alkynyl group, and a method in which an inverse electron demand Diels-Alder reaction is used to form a bond from an highly electron-deficient heterocycle such as a tetrazine or triazine and a compound with strained carbon multiple bonds such as norbornene, trans-cyclooctene, or cyclooctyne.

The oligonucleotide conjugate according to the above aspect may be produced by a known method other than the method according to the above aspect.

One aspect of the present invention is a pharmaceutical composition containing the oligonucleotide conjugate according to the above aspect as an active ingredient. The pharmaceutical composition contains a pharmaceutically acceptable additive. In the present specification, "pharmaceutically acceptable" refers to being acceptable to mammals from a pharmacological or toxicological point of view. That is, a "pharmaceutically acceptable" substance refers to a substance that is physiologically acceptable and that typically does not cause an allergic or other adverse or toxic reaction when administered to a mammal. A "pharmaceutically acceptable" substance means a substance which is approved by a generally recognized regulatory agency or listed in a generally recognized pharmacopoeia for use in mammals, more particularly humans, "pharmaceutically acceptable additive" means a pharmacologically inert material that is used with the oligonucleotide conjugate to formulate a pharmaceutical composition.

Additives may be liquid or solid. The additives are selected with the planned administration method in mind so as to obtain a pharmaceutical composition with the desired dosage, consistency, and the like. Additives are not particularly limited, and examples thereof include water, physiological saline, other aqueous solvents, various carriers such as aqueous or oily bases, excipients, binders, pH adjusters, disintegrants, absorption promoters, lubricants, coloring agents, corrigents, and fragrances. The blending ratio of the additive may be appropriately set based on the range normally employed in the pharmaceutical field.

A pharmaceutical composition may, for example, be a sterile composition for injection. Sterile compositions for injection may be prepared according to normal pharmaceutical practice (for example, dissolving or suspending the active ingredient in a solvent such as water for injection or natural vegetable oil). As aqueous solutions for injection, for example, isotonic solutions containing physiological saline, glucose, or other adjuvants (for example, D-sorbitol, D-mannitol, lactose, sucrose, or sodium chloride) are used. Aqueous solutions for injection may, for example, further contain suitable solubilizers such as alcohols (for example, ethanol), polyalcohols (for example, propylene glycol or polyethylene glycol), or nonionic surfactants (for example, polysorbate 80TM or HCO-50). In addition, aqueous solutions for injection may contain buffers (for example, phosphate buffer solution or sodium acetate buffer solution), soothing agents (for example, benzalkonium chloride or procaine hydrochloride), stabilizers (for example, human serum albumin or polyethylene glycol), preservatives (for example, benzyl alcohol or phenol), antimicrobial agents, dispersants, antioxidants, and various other materials known in the related art. Injections may be, for example, lyophilized formulations.

The oligonucleotide conjugate or pharmaceutical composition according to the above aspects of the present invention can be used to treat and/or prevent diseases associated with specific gene products. Examples of diseases associated with specific gene products include inborn errors of metabolism, a congenital endocrine disease, a single gene disorder, a neurodegenerative disease, a neurologic disease, a myopathy, a meningitis, an encephalitis, an encephalopathy, a lysosome disease, a malignant neoplasm, a fibrosis, an inflammatory disease, an immunodeficiency disease, an autoimmune disease, or an infectious disease, but the diseases are not limited to them. Therefore, one aspect of the present invention is a therapeutic agent or a preventive agent for the above diseases, which contains the oligonucleotide conjugate as an active ingredient.

Another aspect of the present invention is a method for treating and/or preventing the above diseases including administering a therapeutically effective amount of the oligonucleotide conjugate to a human or non-human animal. The human may be a human in need of treatment, namely a patient. Non-human animals include animals such as warm-blooded mammals such as primates; birds; domestic or livestock animals such as cats, dogs, sheep, goats, cows, horses, or pigs; laboratory animals such as mice, rats, or guinea pigs; fish; reptiles; zoo animals; or wild animals. Administration methods include, but are not limited to, oral, sublingual, intravenous, intraarterial, subcutaneous, intradermal, intraperitoneal, intramuscular, intrathecal, intracerebroventricular, intranasal, transmucosal, rectal, ophthalmic, intraocular, transpulmonary, transdermal, intra-articular, topical (cutaneous), intrafollicular, intravaginal, intrauterine, intratumoral, or intralymphatic administration, or combinations thereof.

Another aspect of the present invention is the oligonucleotide conjugate for use in the treatment and/or prevention of the diseases described above. Another aspect of the present invention is the use of oligonucleotide conjugate for producing a therapeutic agent and/or a preventive agent for the above diseases.

The oligonucleotide conjugate or pharmaceutical composition according to the above aspects of the present invention may also be used in combination with one or more other drugs. Other drugs may be one or more therapeutic agents and/or preventive agents for diseases associated with the specific gene products described above. For example, when the disease of interest is a malignant neoplasm, examples of other drugs include drugs that can be used in chemotherapy. That is, one aspect of the present invention is the oligonucleotide conjugate for treating diseases in combination with one or more therapeutic agents and/or preventive agents for the above diseases. Another aspect of the present invention is a medicament containing a combination of the oligonucleotide conjugate or pharmaceutical composition and one or more therapeutic agents and/or preventive agents for the above diseases. However, the present invention is a platform technology that can efficiently transport oligonucleotides into a cell, and can be used for any disease as long as the oligonucleotides can be applied to the diseases as a therapeutic agent or preventive agent, and thus, other drugs are not limited to specific drugs.

The timing of administration of the oligonucleotide conjugate or pharmaceutical composition and other drugs above used in combination therewith is not limited, and these may be administered to humans or animals other than humans at the same time or at appropriate intervals. Alternatively, the pharmaceutical composition according to the above aspect may be blended with other drugs above to prepare a combination drug. The administration dosage and blending amount of other drugs above may be appropriately determined based on the doses used clinically. The blending ratio of the oligonucleotide conjugate or pharmaceutical composition and other drugs above may be appropriately determined according to the administration target, administration route, target disease, symptom, combination of other drugs, and the like.

### Examples

The present invention will be described in detail below with reference to examples and test examples, but the present invention is not limited to these examples. In addition, "%" in the following description means % by weight unless otherwise specified.

### <Synthesis of oligonucleotide>

siRNAs and antisense oligonucleotides shown in Table 2 were prepared. A thiol group was bonded to the 3' end of the sense strand RNA of the siRNAs and the 5' end of the antisense oligonucleotide through Spacer18 (hexaethylene glycol). These nucleic acids were produced by GeneDesign, Inc.

**[Table 2]**

| | |
|---|---|
| Atp5b-siRNA | 5'- |
| sense strand (SEQ ID NO: 7) | U(F)^G(M)^U(F)C(M)A(F)U(M)U(F)G(F)G(F)A(M)G(F)A( M)A(F)C(M)C(F)U(M)A(F)U(M)U(F)tt-3'. 18. SH |
| Atp5b-siRNA antisense strand (SEQ ID NO: 8) | 5'- p_A(M)^A(F)^U(M)A(F)G(M)G(F)U(M)U(F)C(M)U(F)C(M) C(M)A(M)A(F)U(M)G(F)A(M)C(F)A(M)^t^t-3' |
| scramble-siRNA sense strand (SEQ ID NO: 9) | 5'- G(F)^C(M)^U(F)A(M)G(F)A(M)C(F)U(F)G(F)U(M)U(F)U( M)A(F)A(M)C(F)U(M)G(F)A(M)U(F)tt-3' 18 SH |
| scramble-siRNA antisense strand (SEQ ID NO: 10) | 5'- p_A(M)^U(F)^C(M)A(F)G(M)U(F)U(M)A(F)A(M)A(F)C(M) A(M)G(M)U(F)C(M)U(F)A(M)G(F)C(M)^t^t-3' |
| Malatl-ASO (SEQ ID NO: 11) | SH_18_5'- mC(L)^T(L)^A(L)^g^t^t^c^a^c^t^g^a^a^T(L)^G(L)^mC(L)-3' |

| | |
|---|---|
| In the table, upper case = RNA, lower case = DNA, mC = 5-methylcytosine, (M) = 2'-O-CH₃ substitution, (F) = 2'-F substitution, (L) = Locked nucleic acid, ^ = phosphorothioate linkage, p = PO₄, 18 = spacer18 | |

siRNA and ethylenediaminetetraacetic acid trisodium salt (EDTA 3Na) were dissolved in 10 mM phosphate buffered saline (PBS) at pH 7.4, and dithiothreitol (DTT) was added (final concentration: EDTA 0.5 mM, DTT 40 mM). After heating this solution at 25°C for 6 hours, this solution was purified 6 times by ultrafiltration (molecular weight cut-off 10 kDa) using PBS. The nucleic acid concentration of the obtained solution was determined from the absorbance measurement values at 260 nm using an ultraviolet-visible spectrophotometer (manufactured by Tecan Group Ltd., Infinite M200 PRO).

### <Example 1. Production of AF5-labeled cRGD-functionalized oligonucleotide conjugate using fourth generation polylysine dendrigraft as core>

### (A) Synthesis of azide-PEG5k SPDP AF5 DGL G4

As the dendritic polymer, a dendri-grafted poly-L-lysine G4 (DGL G4) having amino groups on the surface manufactured by COLCOM Group was used. To 10 µL of 50 mg/mL dimethyl sulfoxide (DMSO) solution of DGL G4, 1.53 µL of 300 mM DMSO solution of PEG12-SPDP (manufactured by Thermo Fisher Scientific Inc.), 1.17 µL of 50% v/v dimethylformamide (DMF) solution of triethylamine (TEA), and 1.28 µL of 30 mM DMSO solution of AlexaFluor (registered trademark) 546 NHS ester (manufactured by Thermo Fisher Scientific Inc.) were added, and the mixture was stirred at room temperature for 4.5 hours. Next, to this reaction solution, 68.9 µL of 20 mM DMSO solution of Azide-PEG5k-NHS (manufactured by Nanocs Inc., number average molecular weight of PEG: 5000) was added, and the mixture was further stirred at room temperature for 18 hours. Next, 15.3 µL of 200 mM DMSO solution of Methyl-PEG12-NHS (manufactured by Thermo Fisher Scientific Inc.) was added, and the mixture was further stirred at room temperature for 6 hours. By allowing the amino groups of DGL G4 and the N-hydroxysuccinimide (NHS) groups of Azide-PEG5k-NHS, PEG12-SPDP, the anionic fluorescent dye AlexaFluor 546 NHS ester, and Methyl-PEG12-NHS to react as described above, a nanoparticle compound azide-PEG5k SPDP AF5 DGL G4 was obtained. After adding 400 µL of pure water to the reaction solution and mixing, the mixture was purified 6 times by ultrafiltration (manufactured by Merck & Co., Amicon Ultra, molecular weight cut-off: 10 kDa) using pure water. Pure water was added to the collected aqueous solution to adjust the volume of the liquid to 100 µL.

### (B) Synthesis of azide-PEG5k siRNAAF5 DGL G4

To 20 µL of aqueous solution of azide-PEG5k SPDP AF5 DGL G4 shown in (A), 12 µL of 3 M sodium chloride aqueous solution and 72.1 µL of 5.1 mM PBS solution of siRNA were added, and the mixture was stirred at 25°C for 15 hours to alllow the pyridyl disulfide group of azide-PEG5k SPDP AF5 DGL G4 and the SH group of siRNA to react. The reaction solution was purified by gel filtration using Hiprep 16/60 Sephacryl S-200 HR (manufactured by GE HealthCare Technologies Inc.) (eluent: PBS). Fractions containing siRNA-bonded DGL G4 were collected and concentrated by ultrafiltration (manufactured by Merck & Co., Amicon Ultra, molecular weight cut-off 30 kDa), and the volume of the liquid was adjusted to 170 µL.

### (C) Synthesis of cRGD-DBCO

To 33.1 µL of 100 mM DMSO solution of Cyclo(-RGDfK) (manufactured by ChemScence), 33.1 µL of 300 mM DMSO solution of DBCO-NHCO-PEG4-NHS (manufactured by BroadPharm) and 1.39 µL of TEA were added, and the mixture was stirred at 25°C for 16 hours to allow the amino group of Cyclo(-RGDfK) and the NHS ester of DBCO-NHCO-PEG4-NHS to react. The reaction solution was concentrated by removing the solvent under reduced pressure while heating at 45°C, and purified by reversed-phase HPLC (column: manufactured by Waters Corporation, Xbridge Peptide BEH C18, 300 Å, 4.6 × 100 mm, eluent A: 0.1% v/v TFA, eluent B: 0.1% v/v TFA/acetonitrile (10/90; v/v)). After removing the solvent under reduced pressure while heating at 45°C, DMSO was added to adjust the concentration to 50 mM.

### (D) Synthesis of cRGD-PEG5k siRNAAF5 DGL G4

To 30 µL of aqueous solution of azide-PEG5k siRNAAF5 DGL G4 obtained in (B), 4.1 µL of DMSO solution of cRGD-DBCO obtained in (C) was added, and the mixture was stirred at 25°C for 20 hours to allow the azide group of azide-PEG5k siRNA AF5 DGL G4 and the DBCO group (dibenzocyclooctyne group) of cRGD-DBCO to react. Then, after adding 65 µL of PBS, purification was performed using NAP-5 Columns (manufactured by GE HealthCare Technologies Inc.). The collected solution was concentrated using ultrafiltration (molecular weight cut-off 30 kDa), and the volume of the solution was adjusted to 95 µL to obtain a solution of oligonucleotide conjugate cRGD-PEG5k siRNAAF5 DGL G4.

### <Example 2. Production of AF5-labeled GE11-functionalized oligonucleotide conjugate using fourth generation polylysine dendrigraft as core>

To 30 µL of azide-PEG5k siRNA AF5 DGL G4 aqueous solution obtained in (B) of Example 1, 4.1 µL of 50 mM DMSO solution of GE11-DBCO (manufactured by GeneDesign, Inc.) and 32.8 µL of DMSO were added, and the mixture was stirred at 25°C for 20 hours to allow the azide group of azide-PEG5k siRNA AF5 DGL G4 and the DBCO group of GE11-DBCO to react. Then, when 130 µL of PBS was added, a precipitate was obtained, and thus this precipitate was subjected to centrifugal sedimentation to remove the supernatant. After dissolving the obtained precipitate with 100 µL of DMSO, when 400 µL of 2-propanol was added, a precipitate was obtained. Thus, this precipitate was subjected to centrifugal sedimentation again to remove the supernatant. After the obtained precipitate was dissolved in 500 µL of PBS, the solution was purified using NAP-5 Columns (manufactured by GE HealthCare Technologies Inc.). The collected solution was concentrated using ultrafiltration (molecular weight cut-off 30 kDa), and the volume of the solution was adjusted to 95 µL to obtain a solution of oligonucleotide conjugate GE11-PEGSk siRNAAF5 DGL G4.

### <Example 3. Production of TF7-labeled cRGD-functionalized oligonucleotide conjugate using PAMAM G5 as core>

### (A) Synthesis of azide-PEG5k SPDP TF7 PAMAM G5

As the dendritic polymer, a fifth generation PAMAM dendrimer (PAMAM G5) having amino groups on the surface manufactured by Sigma-Aldrich was used. To 3.0 µL of 5% wt methanol solution of PAMAM G5, 1.24 µL of 100 mM DMSO solution of PEG12-SPDP, 1.66 µL of 10 mM DMSO solution of Tide Fluor (trademark) 7WS, succinimidyl ester (manufactured by AAT Bioquest, Inc.), and 1.48 µL of 10% v/v DMF solution of TEA were added, and the mixture was stirred at room temperature for 5 hours. Next, to this reaction solution, 12.4 µL of 30 mM DMSO solution of Azide-PEG5k-NHS, 1.03 µL of 400 mM DMSO solution of NHS (manufactured by FuJIFILM Wako Pure Chemical Corporation), and 1.03 µL of 400 mM DMSO solution of 1-ethyl-3-(3-dimethylaminopropyl)carbodiimide hydrochloride (EDC-HCl, manufactured by Nacalai Tesque, Inc.) was added, and the mixture was further stirred at room temperature for 18.5 hours. Next, 2.65 µL of 200 mM DMSO solution of Methyl-PEG12-NHS was added, and the mixture was further stirred at room temperature for 6 hours. By allowing the amino groups of PAMAM G5 and the NHS groups of Azide-PEG5k-NHS, PEG12-SPDP, Tide Fluor 7WS, succinimidyl ester, and Methyl-PEG12-NHS to react as described above, a nanoparticle compound azide-PEG5k SPDP TF7 PAMAM G5 was obtained. After adding 400 µL of pure water to the reaction solution and mixing, the mixture was purified 6 times by ultrafiltration (manufactured by Merck & Co., Amicon Ultra, molecular weight cut-off: 10 kDa) using pure water. Pure water was added to the collected aqueous solution to adjust the volume of the liquid to 80 µL.

### (B) Synthesis of azide-PEG5k siRNA TF7 PAMAM G5

To 10.0 µL of aqueous solution of azide-PEG5k SPDP TF7 PAMAM G5 shown in (A), 3.2 µL of 3 M sodium chloride aqueous solution and 12.4 µL of 5.0 mM PBS solution of siRNA were added, and the mixture was stirred at 25°C for 15 hours to allow the pyridyl disulfide group of azide-PEG5k SPDP TF7 PAMAM G5 and the SH group of siRNA to react. The reaction solution was purified by gel filtration using Hiprep 16/60 Sephacryl S-200 HR (eluent: PBS). Fractions containing siRNA-bonded PAMAM G5 were collected and concentrated by ultrafiltration (manufactured by Merck & Co., Amicon Ultra, molecular weight cut-off 30 kDa), and the volume of the liquid was adjusted to 100 µL.

### (C) Synthesis of cRGD-PEG5k siRNA TF7 PAMAM G5

To 500 µL of aqueous solution of azide-PEG5k siRNA TF7 PAMAM G5 obtained in (B), 3.70 µL of DMSO solution of cRGD-DBCO obtained in (C) of Example 1 was added, and the mixture was stirred at 25°C for 16 hours to allow the azide group of azide-PEG5k siRNA TF7 PAMAM G5 and the DBCO group of cRGD-DBCO to react. Then, after adding PBS to adjust the liquid volume to 100 µL, the solution was purified using NAP-5 Columns. The collected solution was concentrated using ultrafiltration (molecular weight cut-off 30 kDa), and the volume of the solution was adjusted to 110 µL to obtain a solution of oligonucleotide conjugate cRGD-PEG5k siRNA TF7 PAMAM G5.

### <Example 4. Production of TF7-labeled cRGD-functionalized oligonucleotide conjugate using PAMAM G6 as core>

### (A) Synthesis of azide-PEG5k SPDP TF7 PAMAM G6

As the dendritic polymer, a sixth generation PAMAM dendrimer (PAMAM G6) having amino groups on the surface manufactured by Sigma-Aldrich was used. To 5.0 µL of 5% wt methanol solution of PAMAM G6, 1.39 µL of 100 mM DMSO solution of PEG12-SPDP, 2.78 µL of 10 mM DMSO solution of Tide Fluor 7WS, succinimidyl ester, and 2.48 µL of 10% v/v DMF solution of TEA were added, and the mixture was stirred at room temperature for 5 hours. Next, to this reaction solution, 12.4 µL of 30 mM DMSO solution of Azide-PEGSk-NHS, 1.72 µL of 400 mM DMSO solution of NHS, and 1.72 µL of 400 mM DMSO solution of EDC-HCl were added, and the mixture was further stirred at room temperature for 18.5 hours. Next, 4.44 µL of 200 mM DMSO solution of Methyl-PEG12-NHS was added, and the mixture was further stirred at room temperature for 6 hours. By allowing the amino groups of PAMAM G6 and the NHS groups of Azide-PEG5k-NHS, PEG12-SPDP, Tide Fluor 7WS, succinimidyl ester, and Methyl-PEG12-NHS to react as described above, a nanoparticle compound azide-PEG5k SPDP TF7 PAMAM G6 was obtained. After adding 400 µL of pure water to the reaction solution and mixing, the mixture was purified 6 times by ultrafiltration (manufactured by Merck & Co., Amicon Ultra, molecular weight cut-off: 10 kDa) using pure water. Pure water was added to the collected aqueous solution to adjust the volume of the liquid to 80 µL.

### (B) Synthesis of azide-PEG5k siRNA TF7 PAMAM G6

To 9.0 µL of aqueous solution of azide-PEG5k SPDP TF7 PAMAM G6 shown in (A), 3.1 µL of 3 M sodium chloride aqueous solution and 12.5 µL of 5.0 mM PBS solution of siRNA were added, and the mixture was stirred at 25°C for 15 hours to allow the pyridyl disulfide group of azide-PEG5k SPDP TF7 PAMAM G6 and the SH group of siRNA to react. The reaction solution was purified by gel filtration using Hiprep 16/60 Sephacryl S-200 HR (eluent: PBS). Fractions containing siRNA-bonded PAMAM G6 were collected and concentrated by ultrafiltration (manufactured by Merck & Co., Amicon Ultra, molecular weight cut-off 30 kDa), and the volume of the liquid was adjusted to 100 µL.

### (C) Synthesis of cRGD-PEG5k siRNA TF7 PAMAM G6

To 500 µL of aqueous solution of azide-PEG5k siRNA TF7 PAMAM G6 obtained in (B), 4.51 µL of DMSO solution of cRGD-DBCO obtained in (C) of Example 1 was added, and the mixture was stirred at 25°C for 16 hours to allow the azide group of azide-PEG5k siRNA TF7 PAMAM G6 and the DBCO group of cRGD-DBCO to react. Then, after adding PBS to adjust the liquid volume to 100 µL, the solution was purified using NAP-5 Columns. The collected solution was concentrated using ultrafiltration (molecular weight cut-off 30 kDa), and the volume of the solution was adjusted to 110 µL to obtain a solution of oligonucleotide conjugate cRGD-PEG5k siRNA TF7 PAMAM G6.

### <Example 5. Production of TF7-labeled cRGD-functionalized oligonucleotide conjugate using fourth generation polylysine dendrigraft as core>

Oligonucleotide conjugate cRGD-PEGSk siRNA TF7 DGL G4 was synthesized according to the synthesis of cRGD-PEGSk siRNA AF5 DGL G4 in Example 1. However, Tide Fluor 7WS, succinimidyl ester was used instead of AlexaFluor 546 NHS ester.

### <Example 6. Production of AF5-labeled cRGD peptide-functionalized oligonucleotide conjugate 2 using fourth generation polylysine dendrigraft as core>

### (A) Synthesis of azide-PEG5k SPDP AF5 DGL G4

According to (A) of Example 1, azide-PEG5k SPDP AF5 DGL G4 was synthesized.

### (B) Synthesis of azide-PEG5k siRNA AF5 DGL G4

To 20 µL of aqueous solution of azide-PEG5k SPDP AF5 DGL G4 obtained in (A), 12 µL of 3 M sodium chloride aqueous solution and 76.7 µL of 6.0 mM PBS solution of siRNA were added, and the mixture was stirred at 25°C for 18 hours to allow the pyridyl disulfide group of azide-PEG5k SPDP AF5 DGL G4 and the SH group of siRNA to react. The reaction solution was purified by gel filtration using Hiprep 16/60 Sephacryl S-200 HR (manufactured by GE HealthCare Technologies Inc.) (eluent: PBS). Fractions containing siRNA-bonded DGL G4 were collected and concentrated by ultrafiltration (manufactured by Merck & Co., Amicon Ultra, molecular weight cut-off 30 kDa), and the volume of the liquid was adjusted to 200 µL.

### (C) Synthesis of cRGD-PEGSk siRNA AF5 DGL G4

To 30 µL of aqueous solution of azide-PEG5k siRNAAF5 DGL G4 obtained in (B), 5.8 µL of 5 mM DMSO solution of cRGD-DBCO obtained in (C) of Example 1 was added, and the mixture was stirred at 25°C for 20 hours to allow the azide group of azide-PEG5k siRNA AF5 DGL G4 and the DBCO group of cRGD-DBCO to react. Then, after adding 65 µL of PBS, the solution was purified using NAP-5 Columns (manufactured by GE HealthCare Technologies Inc.). The collected solution was concentrated using ultrafiltration (molecular weight cut-off 30 kDa), and the volume of the solution was adjusted to 95 µL to obtain a solution of oligonucleotide conjugate cRGD-PEGSk siRNAAF5 DGL G4.

### <Example 7. Production of AF5-labeled cRGD peptide-functionalized oligonucleotide conjugate 3 using fourth generation polylysine dendrigraft as core>

According to the synthesis of Example 6, an oligonucleotide conjugate similar to that of Example 6 was synthesized. However, instead of adding 5.8 µL of 5 mM DMSO solution of cRGD-DBCO, 1.4 µL of 5 mM DMSO solution of cRGD-DBCO and 4.3 µL of DMSO were added.

### <Example 8. Production of AF5-labeled cRGD peptide-functionalized oligonucleotide conjugate 4 using fourth generation polylysine dendrigraft as core>

According to the synthesis of Example 6, an oligonucleotide conjugate similar to that of Example 6 was synthesized. However, instead of adding 5.8 µL of 5 mM DMSO solution of cRGD-DBCO, 1.2 µL of 5 mM DMSO solution of cRGD-DBCO and 4.6 µL of DMSO were added.

### <Example 9. Production of AF5-labeled cRGD peptide-functionalized oligonucleotide conjugate 5 using fourth generation polylysine dendrigraft as core>

According to the synthesis of Example 6, an oligonucleotide conjugate similar to that of Example 6 was synthesized. However, instead of adding 5.8 µL of 5 mM DMSO solution of cRGD-DBCO, 0.9 µL of 5 mM DMSO solution of cRGD-DBCO and 4.9 µL of DMSO were added.

### <Example 10. Production of AF5-labeled cRGD peptide-functionalized oligonucleotide conjugate 6 using fourth generation polylysine dendrigraft as core>

According to the synthesis of Example 6, an oligonucleotide conjugate similar to that of Example 6 was synthesized. However, instead of adding 5.8 µL of 5 mM DMSO solution of cRGD-DBCO, 0.6 µL of 5 mM DMSO solution of cRGD-DBCO and 5.2 µL of DMSO were added.

### <Example 11. Production of AF5-labeled c(avb6) peptide-functionalized oligonucleotide conjugate using fourth generation polylysine dendrigraft as core>

According to the synthesis of Example 1, oligonucleotide conjugate c(avb6)-PEG5k siRNA AF5 DGL G4 was synthesized. However, instead of cRGD-DBCO, c(avb6)-DBCO (manufactured by GeneDesign, Inc.) obtained by allowing the amino group of the lysine side chain of c(avb6) and the NHS ester of DBCO-NHCO-PEG4-NHS to react was used.

### <Example 12. Production of AF5-labeled folic acid-functionalized oligonucleotide conjugate using fourth generation polylysine dendrigraft as core>

According to the synthesis of Example 1, oligonucleotide conjugate FA-PEGSk siRNAAF5 DGL G4 was synthesized. However, Folic acid-PEG2 DBCO (manufactured by Nanocs Inc.) was used instead of cRGD-DBCO.

### <Example 13. Production of AF6-labeled indatraline-functionalized oligonucleotide conjugate using fourth generation polylysine dendrigraft as core>

### (A) Synthesis of azide-PEG5k SPDP AF6 DGL G4

To 18 µL of 50 mg/mL DMSO solution of DGL G4, 13.8 µL of 60 mM DMSO solution of PEG12-SPDP, 8.9 µL of 8 mM DMSO solution of an anionic fluorescent dye AlexaFluor (registered trademark) 647 NHS ester (manufactured by Thermo Fisher Scientific Inc.), and 14.0 µL of 10% v/v DMSO solution of TEA were added, and the mixture was stirred at room temperature for 11 hours. Next, to this reaction solution, 1417.6 µL of 1.8 mM DMSO solution of N3-PEG-NHS (manufactured by Biopharma PEG Scientific Inc., number average molecular weight of PEG: 5000), 24.8 µL of 200 mM DMSO solution of EDC-HCl, and 24.8 µL of 200 mM DMSO solution of NHS were added, and the mixture was further stirred at room temperature for 13 hours. Next, 37.8 µL of 200 mM DMSO solution of Methyl-PEG12-NHS was added, and the mixture was further stirred at room temperature for 6 hours. By allowing the amino groups of DGL G4 and the NHS groups of N3-PEG-NHS, PEG12-SPDP, AlexaFluor 647 NHS ester, and Methyl-PEG12-NHS to react as described above, a nanoparticle compound azide-PEG5k SPDP AF6 DGL G4 was obtained. After adding 700 µL of pure water to the reaction solution and mixing, the mixture was purified 6 times by ultrafiltration (manufactured by Merck & Co., Amicon Ultra, molecular weight cut-off 30 kDa) using pure water. Pure water was added to the collected aqueous solution to adjust the volume of the liquid to 420 µL.

### (B) Synthesis of azide-PEG5k siRNAAF6 DGL G4

To 410.0 µL of aqueous solution of azide-PEG5k SPDP AF6 DGL G4 obtained in (A), 127.1 µL of 3 M sodium chloride aqueous solution, 465.0 µL of 5.0 mM PBS solution of siRNA, and 111.3 µL of DMSO were added, and the mixture was stirred at 25°C for 13 hours to allow the pyridyl disulfide group of azide-PEG5k SPDP AF6 DGL G4 and the SH group of siRNA to react. The reaction solution was purified by gel filtration using Hiprep 16/60 Sephacryl S-200 HR (eluent: PBS). Fractions containing siRNA-bonded DGL G4 were collected and concentrated by ultrafiltration (manufactured by Merck & Co., Amicon Ultra, molecular weight cut-off 30 kDa), and the volume of the liquid was adjusted to 700 µL.

### (C) Synthesis of IND-DBCO

To 15.5 µL of 800 mM DMSO solution of indatraline (manufactured by Sigma-Aldrich), 16.5 µL of 300mM DMSO solution of DBCO-NHCO-PEG4-NHS, 3.62 µL of TEA, and 20 µL of acetonitrile were added, and the mixture was stirred at 25°C for 18 hours to allow the amino group of indatraline and the NHS ester of DBCO-NHCO-PEG4-NHS to react. The reaction solution was purified by gel filtration using Sephadex (registered trademark) LH-20 (manufactured by Cytiva) (eluent: ethanol/acetonitrile (50/50; v/v)). After fractions containing the desired product, IND-DBCO, were collected, and the solvent was removed under reduced pressure while heating at 45°C, DMSO was added to adjust the concentration to 20 mM.

### (D) Synthesis of IND-PEGSk siRNA AF6 DGL G4

To 370 µL of PBS solution of azide-PEG5k siRNAAF6 DGL G4 obtained in (B), 42.7 µL of 20 mM DMSO solution of IND-DBCO obtained in (C) and 49.8 µL of DMSO were added, and the mixture was stirred at 25°C for 22 hours to allow the azide group of azide-PEG5k siRNA AF6 DGL G4 and the DBCO group of IND-DBCO to react. Then, the reaction solution was purified using NAP-10 Columns (manufactured by GE HealthCare Technologies Inc.). The collected solution was concentrated using ultrafiltration (molecular weight cut-off 10 kDa) and the volume of the solution was adjusted to 95 µL to obtain a solution of oligonucleotide conjugate IND-PEGSk siRNAAF6 DGL G4.

### <Example 14. Production of AF6-labeled aptamer-functionalized oligonucleotide conjugate 1 using fourth generation polylysine dendrigraft as core>

### (A) Synthesis of azide-PEG5k SPDP AF6 DGL G4

To 8 µL of 50 mg/mL DMSO solution of DGL G4, 6.1 µL of 60 mM DMSO solution of PEG12-SPDP, 3.8 µL of 8 mM DMSO solution of AlexaFluor 647 NHS ester, and 6.5 µL of 10% v/v DMSO solution of TEA were added, and the mixture was stirred at room temperature for 7 hours. Next, to this reaction solution, 700.1 µL of 1.8 mM DMSO solution of N3-PEG-NHS (manufactured by Biopharma PEG Scientific Inc., number average molecular weight of PEG: 5000), 12.3 µL of 200 mM DMSO solution of EDC-HCl, and 12.3 µL of 200 mM DMSO solution of NHS were added, and the mixture was further stirred at room temperature for 16 hours. Next, 16.8 µL of 200 mM DMSO solution of Methyl-PEG12-NHS was added, and the mixture was further stirred at room temperature for 6 hours. By allowing the amino groups of DGL G4 and the NHS groups of N3-PEG-NHS, PEG 12-SPDP, AlexaFluor 647 NHS ester, and Methyl-PEG12-NHS to react as described above, a nanoparticle compound azide-PEG5k SPDP AF6 DGL G4 was obtained. After adding 700 µL of pure water to the reaction solution and mixing, the mixture was purified 6 times by ultrafiltration (manufactured by Merck & Co., Amicon Ultra, molecular weight cut-off 30 kDa) using pure water. Pure water was added to the collected aqueous solution to adjust the volume of the liquid to 190 µL.

### (B) Synthesis of azide-PEG5k siRNAAF6 DGL G4

To 120 µL of aqueous solution of azide-PEG5k SPDP AF6 DGL G4 obtained in (A), 33.9 µL of 3 M sodium chloride aqueous solution, 108.3 µL of 6.0 mM PBS solution of siRNA, and 29.4 µL of DMSO were added, and the mixture was stirred at 25°C for 14 hours to allow the pyridyl disulfide group of azide-PEG5k SPDP AF6 DGL G4 and the SH group of siRNA to react. The reaction solution was purified by gel filtration using Hiprep 16/60 Sephacryl S-200 HR (eluent: PBS). Fractions containing siRNA-bonded DGL G4 were collected and concentrated by ultrafiltration (manufactured by Merck & Co., Amicon Ultra, molecular weight cut-off 30 kDa), and the volume of the liquid was adjusted to 800 µL.

### (C) Synthesis of NU1-PEG5k siRNAAF6 DGL G4

DBCO-NHCO-PEG4-NHS was allowed to react with the 3' end of an aptamer AS 1411 having the nucleotide sequence shown in SEQ ID NO: 1 through an Amino C6 linker to obtain NU1-DBCO (manufactured by GeneDesign, Inc.). 22.7 µL of 2.3 mM PBS solution of NU1 -DBCO, 95 µL of PBS solution of azide-PEG5k siRNAAF6 DGL G4 obtained in (B), and 14.1 µL of DMSO were mixed and stirred at 25°C for 15 hours to allow the azide group of azide-PEG5k siRNA AF6 DGL G4 and the DBCO group of NU1-DBCO to react. The reaction solution was purified by gel filtration using Hiprep 16/60 Sephacryl S-200 HR (eluent: PBS). Fractions containing the desired product were collected and concentrated by ultrafiltration (manufactured by Merck & Co., Amicon Ultra, molecular weight cut-off 30 kDa). Furthermore, purification was performed by gel filtration using two Superose 6 Increase (manufactured by Cytiva, eluent: PBS) connected in series. Fractions containing the desired product were collected and concentrated by ultrafiltration (manufactured by Merck & Co., Amicon Ultra, molecular weight cut-off 30 kDa). The volume of the liquid was adjusted to 194 µL to obtain a solution of oligonucleotide conjugate NU1-PEGSk siRNA AF6 DGL G4.

### <Example 15. Production of AF6-labeled aptamer-functionalized oligonucleotide conjugate 2 using fourth generation polylysine dendrigraft as core>

According to the synthesis of Example 14, NU2-PEG5k siRNA AF6 DGL G4 was synthesized. However, instead of NU1-DBCO, NU2-DBCO (manufactured by GeneDesign, Inc.) obtained by allowing DBCO-NHCO-PEG4-NHS to react with the 3' end of an aptamer FAN-1524dI having the nucleotide sequence shown in SEQ ID NO: 2 through an Amino C6 linker was used.

### <Example 16. Production of AF6-labeled aptamer-functionalized oligonucleotide conjugate 3 using fourth generation polylysine dendrigraft as core>

According to the synthesis of Example 14, EP1-PEGSk siRNA AF6 DGL G4 was synthesized. However, instead of NU1 -DBCO, EP1-DBCO (manufactured by GeneDesign, Inc.) obtained by allowing DBCO-NHCO-PEG4-NHS to react with the 3' end of an aptamer having the nucleotide sequence shown in SEQ ID NO: 3 through an Amino C6 linker was used.

### <Example 17. Production of AF6-labeled aptamer-functionalized oligonucleotide conjugate 4 using fourth generation polylysine dendrigraft as core>

According to the synthesis of Example 14, EP2-PEGSk siRNA AF6 DGL G4 was synthesized. However, instead of NU1-DBCO, EP2-DBCO (manufactured by GeneDesign, Inc.) obtained by allowing DBCO-NHCO-PEG4-NHS to react with the 5' end of an aptamer having the nucleotide sequence shown in SEQ ID NO: 4 through an Amino C6 linker was used.

### <Example 18. Production of AF5-labeled cRGD-functionalized oligonucleotide conjugate using third generation polylysine dendrigraft as core>

### (A) Synthesis of azide-PEG5k SPDP AF5 DGL G3

As the dendritic polymer, a dendri-grafted poly-L-lysine G3 (DGL G3) having amino groups on the surface manufactured by COLCOM Group was used. To 10 µL of 50 mg/mL DMSO solution of DGL G3, 1.5 µL of 300 mM DMSO solution of PEG12-SPDP, 3.8 µL of 10 mM DMSO solution of AlexaFluor 546 NHS ester, and 1.0 µL of 20% v/v DMF solution of TEA were added, and the mixture was stirred at room temperature for 7 hours. Next, to this reaction solution, 45.9 µL of 30 mM DMSO solution of Azide-PEGSk-NHS (manufactured by Nanocs Inc., number average molecular weight of PEG: 5000), 6.9 µL of 400 mM DMSO solution of EDC-HCl, and 6.9 µL of 400 mM DMSO solution of NHS were added, and the mixture was further stirred at room temperature for 16 hours. Next, 4.7 µL of 200 mM DMSO solution of Methyl-PEG12-NHS was added, and the mixture was further stirred at room temperature for 8 hours. By allowing the amino groups of DGL G3 and the NHS groups of Azide-PEGSk-NHS, PEG12-SPDP, AlexaFluor 546 NHS ester, and Methyl-PEG12-NHS to react as described above, a nanoparticle compound azide-PEG5k SPDP AF5 DGL G3 was obtained. After adding 700 µL of pure water to the reaction solution and mixing, the mixture was purified 6 times by ultrafiltration (manufactured by Merck & Co., Amicon Ultra, molecular weight cut-off: 10 kDa) using pure water. Pure water was added to the collected aqueous solution to adjust the volume of the liquid to 100 µL.

### (B) Synthesis of azide-PEG5k siRNAAF5 DGL G3

To 4.0 µL of aqueous solution of azide-PEG5k SPDP AF5 DGL G3 obtained in (A), 2.4 µL of 3 M sodium chloride aqueous solution and 14.8 µL of 5.0 mM PBS solution of siRNA were added, and the mixture was stirred at 25°C for 18 hours to allow the pyridyl disulfide group of azide-PEG5k SPDP AF5 DGL G3 and the SH group of siRNA to react. The reaction solution was purified by gel filtration using Hiprep 16/60 Sephacryl S-200 HR (eluent: PBS). Fractions containing siRNA-bonded DGL G3 were collected and concentrated by ultrafiltration (manufactured by Merck & Co., Amicon Ultra, molecular weight cut-off 30 kDa), and the volume of the liquid was adjusted to 100 µL.

### (C) Synthesis of cRGD-PEGSk siRNAAF5 DGL G3

To 40 µL of PBS solution of azide-PEG5k siRNA AF5 DGL G3 obtained in (B), 3.1 µL of 5 mM DMSO solution of cRGD-DBCO obtained in (C) of Example 1 was added, and the mixture was stirred at 25°C for 18 hours to allow the azide group of azide-PEG5k siRNA AF5 DGL G3 and the DBCO group of cRGD-DBCO to react. Then, after adding PBS to adjust the liquid volume to 100 µL, the solution was purifiedusing NAP-5 Columns. The collected solution was concentrated using ultrafiltration (manufactured by Merck & Co., Amicon Ultra, molecular weight cut-off 30 kDa) and the volume of the solution was adjusted to 110 µL to obtain a solution of oligonucleotide conjugate cRGD-PEGSk siRNA AF5 DGL G3.

### <Example 19. Production of AF5-labeled cRGD-functionalized oligonucleotide conjugate using fifth generation polylysine dendrigraft as core>

### (A) Synthesis of azide-PEG5k SPDP AF5 DGL G5

As the dendritic polymer, a dendri-grafted Poly-L-Lysine G5 (DGL G5) having amino groups on the surface manufactured by COLCOM Group was used. To 3.0 µL of 50 mg/mL DMSO solution of DGL G5, 0.46 µL of 300 mM DMSO solution of PEG12-SPDP, 1.1 µL of 10 mM DMSO solution of AlexaFluor 546 NHS ester, and 2.3 µL of 20% v/v DMF solution of TEA were added, and the mixture was stirred at room temperature for 7 hours. Next, to this reaction solution, 13.8 µL of 30 mM DMSO solution of Azide-PEGSk-NHS (manufactured by Nanocs Inc., number average molecular weight of PEG: 5000), 2.1 µL of 400 mM DMSO solution of EDC-HCl, and 2.1 µL of 400 mM DMSO solution of NHS were added, and the mixture was further stirred at room temperature for 16 hours. Next, 11.1 µL of 200 mM DMSO solution of Methyl-PEG12-NHS was added, and the mixture was further stirred at room temperature for 8 hours. By allowing the amino groups of DGL G5 and the NHS groups of Azide-PEGSk-NHS, PEG12-SPDP, AlexaFluor 546 NHS ester, and Methyl-PEG12-NHS to react as described above, a nanoparticle compound azide-PEG5k SPDP AF5 DGL G5 was obtained. After adding 700 µL of pure water to the reaction solution and mixing, the mixture was purified 6 times by ultrafiltration (manufactured by Merck & Co., Amicon Ultra, molecular weight cut-off: 10 kDa) using pure water. Pure water was added to the collected aqueous solution to adjust the volume of the liquid to 100 µL.

### (B) Synthesis of azide-PEG5k siRNA AF5 DGL G5

To 13.0 µL of aqueous solution of azide-PEG5k SPDP AF5 DGL G5 obtained in (A), 4.0 µL of 3 M sodium chloride aqueous solution and 14.8 µL of 5.0 mM PBS solution of siRNA were added, and the mixture was stirred at 25°C for 18 hours to allow the pyridyl disulfide group of azide-PEG5k SPDP AF5 DGL G5 and the SH group of siRNA to react. The reaction solution was purified by gel filtration using Hiprep 16/60 Sephacryl S-200 HR (eluent: PBS). Fractions containing siRNA-bonded DGL G5 were collected and concentrated by ultrafiltration (manufactured by Merck & Co., Amicon Ultra, molecular weight cut-off 30 kDa), and the volume of the liquid was adjusted to 100 µL.

### (C) Synthesis of cRGD-PEGSk siRNA AF5 DGL G5

To 40 µL of PBS solution of azide-PEG5k siRNA AF5 DGL G5 obtained in (B), 3.0 µL of 5 mM DMSO solution of cRGD-DBCO obtained in (C) of Example 1 was added, and the mixture was stirred at 25°C for 18 hours to allow the azide group of azide-PEG5k siRNA AF5 DGL G5 and the DBCO group of cRGD-DBCO to react. Then, after adding PBS to adjust the liquid volume to 100 µL, the solution was purifiedusing NAP-5 Columns. The collected solution was concentrated using ultrafiltration (manufactured by Merck & Co., Amicon Ultra, molecular weight cut-off 30 kDa), and the volume of the solution was adjusted to 110 µL to obtain a solution of oligonucleotide conjugate cRGD-PEGSk siRNA AF5 DGL G5.

### <Example 20. Production of AF6-labeled cRGD-functionalized oligonucleotide conjugate using sixth generation Bis-MPA dendrimer as core>

### (A) Synthesis of PFD-G6-TMP-NH2

5.25 mg of PFD-G6-TMP-NHBoc (manufactured by Polymer Factory) was dissolved in 200 µL of TFA and stirred at room temperature for 3 hours. Then, diethyl ether was added to this reaction solution, and the precipitated solid was subjected to centrifugal sedimentation. The supernatant was removed and the solid was washed 3 times with diethyl ether. After removing the supernatant, 300 µL of DMSO was added to dissolve the solid.

### (B) Synthesis of azide-PEG5k SPDP AF6 MPA G6

To 20 µL of DMSO solution of PFD-G6-TMP-NH2 obtained in (A), 6.4 µL of 60 mM DMSO solution of PEG12-SPDP, 3.4 µL of 8 mM DMSO solution of AlexaFluor 647 NHS ester, 384.0 µL of 2.5 mM DMSO solution of N3-PEG-NHS (manufactured by Biopharma PEG Scientific Inc., number average molecular weight of PEG: 5000), 9.6 µL of 200 mM DMSO solution of EDC-HCl, 9.6 µL of 200 mM DMSO solution of NHS, 6.1 µL of 200 mM DMSO solution of Methyl-PEG12-NHS, and 6.1 µL of 10% v/v DMSO solution of TEA were added, and the mixture was stirred at room temperature for 12 hours. By allowing the amino group of PFD-G6-TMP-NH2 and the NHS groups of N3-PEG-NHS, PEG12-SPDP, AlexaFluor 647 NHS ester, and Methyl-PEG12-NHS to react as described above, a nanoparticle compound azide-PEG5k SPDP AF6 MPA G6 was obtained. After adding 700 µL of pure water to the reaction solution and mixing, the mixture was purified 6 times by ultrafiltration (manufactured by Sartorius AG, Vivaspin, molecular weight cut-off: 50 kDa) using pure water. Pure water was added to the collected aqueous solution to adjust the volume of the liquid to 310 µL.

### (C) Synthesis of azide-PEG5k siRNA AF6 MPA G6

To 6.0 µL of aqueous solution of azide-PEG5k SPDP AF6 MPA G6 obtained in (B), 1.9 µL of 3 M sodium chloride aqueous solution, 7.4 µL of 6.0 mM PBS solution of siRNA, and 3.8 µL of DMSO were added, and the mixture was stirred at 25°C for 16 hours to allow the pyridyl disulfide group of azide-PEG5k SPDP AF6 MPA G6 and the SH group of siRNA to react. The reaction solution was purified by gel filtration using Hiprep 16/60 Sephacryl S-200 HR (eluent: PBS). Fractions containing siRNA-bonded MPA G6 was collected and concentrated by ultrafiltration (manufactured by Sartorius, Vivaspin, molecular weight cut-off 50 kDa), and the volume of the liquid was adjusted to 80 µL.

### (D) Synthesis of cRGD-PEGSk siRNA AF6 MPA G6

To 50 µL of PBS solution of azide-PEG5k siRNAAF6 MPA G6 obtained in (C), 1.5 µL of 10 mM DMSO solution of cRGD-DBCO obtained in (C) of Example 1 and 4.1 µL of DMSO were added, and the mixture was stirred at 25°C for 16 hours to allow the azide group of azide-PEG5k siRNA AF6 MPA G6 and the DBCO group of cRGD-DBCO to react. The reaction solution was purified using Zeba (registered trademark) Spin Desalting Column (manufactured by Thermo Fisher Scientific Inc., molecular weight cut-off 40 kDa). Next, purification was performed 3 times using PBS by ultrafiltration (manufactured by Merck & Co., Amicon Ultra, molecular weight cut-off 50 kDa), and the volume of the liquid was adjusted to 70 µL to obtain a solution of oligonucleotide conjugate cRGD-PEGSk siRNA AF6 MPA G6.

### <Example 21. Production of AF6-labeled cRGD-functionalized oligonucleotide conjugate with small number of oligonucleotide modifications, using fourth generation polylysine dendrigraft as core>

### (A) Synthesis of azide-PEG5k SPDP-C3 AF6 DGL G4

According to (A) of Example 14, azide-PEG5k SPDP-C3 AF6 DGL G4 was synthesized. However, instead of SPDP-PEG12, N-Succinimidyl 3-(2-pyridyldithio)propionate (manufactured by Tokyo Chemical Industry Co., Ltd.) was used.

### (B) Synthesis of azide-PEG5k siRNA-C3 AF6 DGL G4

To 10.0 µL of aqueous solution of azide-PEG5k SPDP-C3 AF6 DGL G4 obtained in (A), 0.79 µL of 6.5 mM PBS solution of siRNA, 1.9 µL of 3 M NaCl aqueous solution, and 1.4 µL of DMSO were added, and the mixture was stirred at room temperature for 13 hours to allow the pyridyl disulfide group of azide-PEG5k SPDP-C3 AF6 DGL G4 and the SH group of siRNA to react. The reaction solution was purified by gel filtration using Hiprep 16/60 Sephacryl S-200 HR (eluent: PBS). Fractions containing siRNA-bonded DGL G4 was collected and concentrated by ultrafiltration (manufactured by Sartorius, Vivaspin, molecular weight cut-off 30 kDa), and the volume of the liquid was adjusted to 95 µL.

### (C) Synthesis of cRGD-PEGSk siRNA-C3 AF6 DGL G4

To 60 µL of PBS solution of azide-PEG5k siRNA-C3 AF6 DGL G4 obtained in (B), 4.1 µL of 5 mM DMSO solution of cRGD-DBCO obtained in (C) of Example 1 and 2.6 µL of DMSO were added, and the mixture was stirred at 25°C for 14 hours to allow the azide group of azide-PEG5k siRNA-C3 AF6 DGL G4 and the DBCO group of cRGD-DBCO to react. The reaction solution was purified using Zeba (registered trademark) Spin Desalting Column. Next, purification was performed 3 times using PBS by ultrafiltration (manufactured by Merck & Co., Amicon Ultra, molecular weight cut-off 50 kDa), and the volume of the liquid was adjusted to 70 µL to obtain a solution of oligonucleotide conjugate cRGD-PEGSk siRNA-C3 AF6 DGL G4.

### <Example 22. Production of AF6-labeled c(avb6)-functionalized oligonucleotide conjugate using fourth generation polylysine dendrigraft as core and using antisense oligonucleotide >

### (A) Synthesis of azide-PEG5k ASO AF6 DGL G4

According to (B) of Example 14, azide-PEG5k ASO AF6 DGL G4 was synthesized. However, Malat1-ASO shown in Table 2 was used instead of siRNA.

### (B) Synthesis of c(avb6)-PEG5k ASO AF6 DGL G4

To 60 µL of PBS solution of azide-PEG5k ASO AF6 DGL G4 obtained in (A), 1.6 µL of 1 mM DMSO solution of c(avb6)-DBCO and 5.1 µL of DMSO were added, and the mixture was stirred at 25°C for 15 hours to allow the azide group of azide-PEG5k ASO AF6 DGL G4 and the DBCO group of c(avb6)-DBCO to react. The reaction solution was purified using Zeba (registered trademark) Spin Desalting Column. Next, purification was performed 3 times using PBS by ultrafiltration (manufactured by Merck & Co., Amicon Ultra, molecular weight cut-off 50 kDa), and the volume of the liquid was adjusted to 100 µL to obtain a solution of oligonucleotide conjugate c(avb6)-PEG5k ASO AF6 DGL G4.

### <Example 23. Production of AF5-labeled cRGD-functionalized oligonucleotide conjugate modified with PEG2k, using fourth generation polylysine dendrigraft as core>

### (A) Synthesis of azide-PEG2k SPDP AF5 DGL G4

To 2.0 µL of 50 mg/mL DMSO solution of DGL G4, 0.9 µL of 100 mM DMSO solution of PEG12-SPDP, 1.5 µL of 5 mM DMSO solution of AlexaFluor 546 NHS ester, and 2.5 µL of 10% v/v DMF solution of TEA were added, and the mixture was stirred at room temperature for 6 hours. Next, to this reaction solution, 6.1 µL of 100 mM DMSO solution of Azide-PEG2k-NHS (manufactured by Nanocs Inc., number average molecular weight of PEG: 2000), 3.1 µL of 400 mM DMSO solution of EDC-HCl, and 3.1 µL of 400 mM DMSO solution of NHS were added, and the mixture was further stirred at room temperature for 14 hours. Next, 2.8 µL of 200 mM DMSO solution of Methyl-PEG12-NHS was added, and the mixture was further stirred at room temperature for 6 hours. By allowing the amino groups of DGL G4 and the NHS groups of Azide-PEG2k-NHS, PEG12-SPDP, AlexaFluor 546 NHS ester, and Methyl-PEG12-NHS to react as described above, a nanoparticle compound azide-PEG2k SPDP AF5 DGL G4 was obtained. After adding 700 µL of pure water to the reaction solution and mixing, the mixture was purified 6 times by ultrafiltration (manufactured by Merck & Co., Amicon Ultra, molecular weight cut-off 30 kDa) using pure water. Pure water was added to the collected aqueous solution to adjust the volume of the liquid to 230 µL.

### (B) Synthesis of azide-PEG2k siRNAAF5 DGL G4

To 50 µL of aqueous solution of azide-PEG2k SPDP AF5 DGL G4 obtained in (A), 7.3 µL of 3 M sodium chloride aqueous solution and 12.0 µL of 5.0 mM PBS solution of siRNA were added, and the mixture was stirred at 25°C for 18 hours to allow the pyridyl disulfide group of azide-PEG2k SPDP AF5 DGL G4 and the SH group of siRNA to react. The reaction solution was purified by gel filtration using Hiprep 16/60 Sephacryl S-200 HR (eluent: PBS). Fractions containing siRNA-bonded DGL G4 were collected and concentrated by ultrafiltration (manufactured by Merck & Co., Amicon Ultra, molecular weight cut-off 30 kDa), and the volume of the liquid was adjusted to 185 µL.

### (C) Synthesis of cRGD-PEG2k siRNAAF5 DGL G4

To 90 µL of PBS solution of azide-PEG2k siRNA AF5 DGL G4 obtained in (B), 4.0 µL of 5.0 mM DMSO solution of cRGD-DBCO obtained in (C) of Example 1 and 6.0 µL of DMSO were added, and the mixture was stirred at 25°C for 18 hours to allow the azide group of azide-PEG2k siRNA AF5 DGL G4 and the DBCO group of cRGD-DBCO to react. Then, the reaction solution was purified using NAP-5 Columns. The collected solution was concentrated using ultrafiltration (manufactured by Merck & Co., Amicon Ultra, molecular weight cut-off 30 kDa), and the volume of the solution was adjusted to 110 µL to obtain a solution of oligonucleotide conjugate cRGD-PEG2k siRNAAF5 DGL G4.

### <Example 24. Production of AF5-labeled cRGD-functionalized oligonucleotide conjugate modified with PEG3.4k, using fourth generation polylysine dendrigraft as core>

According to the synthesis of Example 23, oligonucleotide conjugate cRGD-PEG3.4k siRNA AF5 DGL G4 was synthesized. However, instead of Azide-PEG2k-NHS, Azide-PEG3.4k-NHS (manufactured by Nanocs Inc., number average molecular weight of PEG: 3400) was used.

### <Example 25. Production of AF5-labeled cRGD-functionalized oligonucleotide conjugate modified with PEG5k, using fourth generation polylysine dendrigraft as core>

### (A) Synthesis of azide-PEG5k SPDP AF5 DGL G4

To 2 µL of 50 mg/mL DMSO solution of DGL G4, 3.1 µL of 30 mM DMSO solution of PEG12-SPDP, 1.5 µL of 4 mM DMSO solution of AlexaFluor 546 NHS ester, and 1.6 µL of 10% v/v DMSO solution of TEA were added, and the mixture was stirred at room temperature for 8 hours. Next, to this reaction solution, 306.3 µL of 1.0 mM DMSO solution of N3-PEG-NHS (manufactured by Biopharma PEG Scientific Inc., number average molecular weight of PEG: 5000), 3.1 µL of 200 mM DMSO solution of EDC-HCl, and 3.1 µL of 200 mM DMSO solution of NHS were added, and the mixture was further stirred at room temperature for 15 hours. Next, 4.2 µL of 200 mM DMSO solution of Methyl-PEG12-NHS was added, and the mixture was further stirred at room temperature for 8 hours. By allowing the amino groups of DGL G4 and the NHS groups of N3-PEG-NHS, PEG12-SPDP, AlexaFluor 546 NHS ester, and Methyl-PEG12-NHS to react as described above, a nanoparticle compound azide-PEG5k SPDP AF5 DGL G4 was obtained. After adding 700 µL of pure water to the reaction solution and mixing, the mixture was purified 6 times by ultrafiltration (manufactured by Merck & Co., Amicon Ultra, molecular weight cut-off 30 kDa) using pure water. Pure water was added to the collected aqueous solution to adjust the volume of the liquid to 50 µL.

### (B) Synthesis of azide-PEG5k siRNAAF5 DGL G4

To 18.0 µL of aqueous solution of azide-PEG5k SPDP AF5 DGL G4 obtained in (A), 4.1 µL of 3 M sodium chloride aqueous solution and 7.9 µL of 6.0 mM PBS solution of siRNA were added, and the mixture was stirred at 25°C for 14 hours to allow the pyridyl disulfide group of azide-PEG5k SPDP AF5 DGL G4 and the SH group of siRNA to react. The reaction solution was purified by gel filtration using Hiprep 16/60 Sephacryl S-200 HR (eluent: PBS). Fractions containing siRNA-bonded DGL G4 were collected and concentrated by ultrafiltration (manufactured by Merck & Co., Amicon Ultra, molecular weight cut-off 30 kDa), and the volume of the liquid was adjusted to 100 µL.

### (C) Synthesis of cRGD-PEGSk siRNA AF5 DGL G4

To 70 µL of PBS solution of azide-PEG5k siRNA AF5 DGL G4 obtained in (B), 6.9 µL of 2.0 mM DMSO solution of cRGD-DBCO obtained in (C) of Example 1 and 0.9 µL of DMSO were added, and the mixture was stirred at 25°C for 14 hours to allow the azide group of azide-PEG5k siRNA AF5 DGL G4 and the DBCO group of cRGD-DBCO to react. Then, the reaction solution was purified using NAP-5 Columns. The collected solution was concentrated using ultrafiltration (manufactured by Merck & Co., Amicon Ultra, molecular weight cut-off 30 kDa). The volume of the solution was adjusted to 70 µL, and filter filtration (Ultrafree manufactured by Merck & Co.; -MC, GV, 0.22 µm) was performed to obtain a solution of oligonucleotide conjugate cRGD-PEG5k siRNAAF5 DGL G4.

### <Example 26. Production of AF5-labeled cRGD-functionalized oligonucleotide conjugate modified with PEG10k, using fourth generation polylysine dendrigraft as core>

According to the synthesis of Example 25, oligonucleotide conjugate cRGD-PEG10k siRNA AF5 DGL G4 was synthesized. However, instead of adding 306.3 µL of 1.0 mM DMSO solution of N3-PEG-NHS (manufactured by Biopharma PEG Scientific Inc., number average molecular weight of PEG: 5000), 1531 µL of 0.2 mM DMSO solution of N3-PEG-NHS (manufactured by Biopharma PEG Scientific Inc., number average molecular weight of PEG: 10000) was added.

### <Example 27. Production of TF7-labeled cRGD-functionalized oligonucleotide conjugatemodified with pMeOx10k, using fourth generation polylysine dendrigraft as core>

### (A) Synthesis of azide-pMeOxlOk SPDP TF7 DGL G4

To 4.0 µL of 50 mg/mL DMSO solution of DGL G4, 1.2 µL of 150 mM DMSO solution of PEG12-SPDP, 1.5 µL of 10 mM DMSO solution of Tide Fluor7WS, and succinimidyl ester, and 1.3 µL of a 25% v/v DMF solution of TEA were added, and the mixture was stirred at room temperature for 4 hours. Next, to this reaction solution, a mixed solution obtained by stirring 76.6 µL of 8 mM DMSO solution of Poly(2-methyl-2-oxazoline), carboxy initiated, azide terminated (manufactured by Ultroxa, number average molecular weight of pMeOx: 10000), 4.9 µL of 500 mM DMSO solution of EDC-HCl, and 4.9 µL of 500 mM DMSO solution of NHS at room temperature for 0.5 hours was added, and the mixture was further stirred for 18 hours. Next, 5.6 µL of 200 mM DMSO solution of Methyl-PEG12-NHS was added, and the mixture was further stirred at room temperature for 6 hours. By allowing the amino groups of DGL G4 to react with the COOH group of Poly(2-methyl-2-oxazoline), carboxy initiated, azide terminated, and the NHS groups of PEG12-SPDP, Tide Fluor7WS, and Methyl-PEG12-NHS as described above, a nanoparticle compound azide-pMeOx10k SPDP TF7 DGL G4 was obtained. After adding 700 µL of pure water to the reaction solution and mixing, the mixture was purified 6 times by ultrafiltration (manufactured by Merck & Co., Amicon Ultra, molecular weight cut-off 30 kDa) using pure water. Pure water was added to the collected aqueous solution to adjust the volume of the liquid to 230 µL.

### (B) Synthesis of azide-pMeOx10k siRNA TF7 DGL G4

To 8.0 µL of aqueous solution of azide-pMeOx10k SPDP TF7 DGL G4 obtained in (A), 2.9 µL of 3 M sodium chloride aqueous solution and 13.1 µL of 5.0 mM PBS solution of siRNA were added, and the mixture was stirred at 25°C for 14 hours to allow the pyridyl disulfide group of azide-pMeOx10k SPDP TF7 DGL G4 and the SH group of siRNA to react. The reaction solution was purified by gel filtration using Hiprep 16/60 Sephacryl S-200 HR (eluent: PBS). Fractions containing siRNA-bonded DGL G4 were collected and concentrated by ultrafiltration (manufactured by Merck & Co., Amicon Ultra, molecular weight cut-off 30 kDa), and the volume of the liquid was adjusted to 100 µL.

### (C) Synthesis of cRGD-pMeOxlOk siRNA TF7 DGL G4

To 40 µL of PBS solution of azide-pMeOx10k siRNA TF7 DGL G4 obtained in (B), 1.6 µL of 10.0 mM DMSO solution of cRGD-DBCO obtained in (C) of Example 1 was added, and the mixture was stirred at 25°C for 19 hours to allow the azide group of azide-pMeOx10k siRNA TF7 DGL G4 and the DBCO group of cRGD-DBCO to react. Then, the reaction solution was purified using NAP-5 Columns. The collected solution was concentrated using ultrafiltration (manufactured by Merck & Co., Amicon Ultra, molecular weight cut-off 30 kDa), and the volume of the solution was adjusted to 110 µL to obtain a solution of oligonucleotide conjugate cRGD-pMeOxlOk siRNA TF7 DGL G4.

### <Example 28. Production of AF5-labeled cRGD-functionalized oligonucleotide conjugatemodified with pSar10k, using fourth generation polylysine dendrigraft as core>

### (A) Synthesis of azide-pSar10k SPDP AF5 DGL G4

To 4.0 µL of 50 mg/mL DMSO solution of DGL G4, 1.2 µL of 150 mM DMSO solution of PEG12-SPDP, 1.0 µL of 15 mM DMSO solution of AlexaFluor 546 NHS ester, and 1.6 µL of 20% v/v DMF solution of TEA were added, and the mixture was stirred at room temperature for 8 hours. Next, to this reaction solution, a mixed solution obtained by stirring 61.3 µL of 10 mM DMSO solution of N3-pSar(150)-COOH (manufactured by Iris Biotech GmbH, number average molecular weight of pSar: 11400), 2.5 µL of 500 mM DMSO solution of EDC-HCl, and 2.5 µL of 500 mM DMSO solution of NHS at room temperature for 0.5 hours was added, and the mixture was further stirred for 19 hours. Next, 5.6 µL of 200 mM DMSO solution of Methyl-PEG12-NHS was added, and the mixture was further stirred at room temperature for 9 hours. By allowing the amino groups of DGL G4 to react with the COOH group of N3-pSar(150)-COOH and the NHS groups of PEG12-SPDP, AlexaFluor 546 NHS ester, and Methyl-PEG12-NHS as described above, a nanoparticle compound azide-pSar10k SPDP AF5 DGL G4 was obtained. After adding 700 µL of pure water to the reaction solution and mixing, the mixture was purified 6 times by ultrafiltration (manufactured by Merck & Co., Amicon Ultra, molecular weight cut-off 30 kDa) using pure water. Pure water was added to the collected aqueous solution to adjust the volume of the liquid to 90 µL.

### (B) Synthesis of azide-pSar10k siRNAAF5 DGL G4

To 12.0 µL of aqueous solution of azide-pSar10k SPDP AF5 DGL G4 obtained in (A), 4.4 µL of 3 M sodium chloride aqueous solution and 19.7 µL of 5.0 mM PBS solution of siRNA were added, and the mixture was stirred at 25°C for 18 hours to allow the pyridyl disulfide group of azide-pSar10k SPDP AF5 DGL G4 and the SH group of siRNA to react. The reaction solution was purified by gel filtration using Hiprep 16/60 Sephacryl S-200 HR (eluent: PBS). Fractions containing siRNA-bonded DGL G4 were collected and concentrated by ultrafiltration (manufactured by Merck & Co., Amicon Ultra, molecular weight cut-off 30 kDa), and the volume of the liquid was adjusted to 100 µL.

### (C) Synthesis of cRGD-pSar10k siRNAAF5 DGL G4

To 40 µL of PBS solution of azide-pSar10k siRNAAF5 DGL G4 obtained in (B), 1.6 µL of 10.0 mM DMSO solution of cRGD-DBCO obtained in (C) of Example 1 was added, and the mixture was stirred at 25°C for 19 hours to allow the azide group of azide-pSar10k siRNAAF5 DGL G4 and the DBCO group of cRGD-DBCO to react. Then, the reaction solution was purified using NAP-5 Columns. The collected solution was concentrated using ultrafiltration (manufactured by Merck & Co., Amicon Ultra, molecular weight cut-off 30 kDa), and the volume of the solution was adjusted to 110 µL to obtain a solution of oligonucleotide conjugate cRGD-pSar10k siRNAAF5 DGL G4.

### <Example 29. Production of AF5-labeled cRGD-functionalized oligonucleotide conjugate modified with PEG5k through disulfide bond, using fourth generation polylysine dendrigraft as core>

### (A) Synthesis of SPDP AF5 DGL G4

To 3 µL of 50 mg/mL DMSO solution of DGL G4, 2.8 µL of 100 mM DMSO solution of PEG12-SPDP, 2.3 µL of 5 mM DMSO solution of AlexaFluor 546 NHS ester, and 1.8 µL of 10% v/v DMF solution of TEA were added, and the mixture was stirred at room temperature for 19 hours. Next, 4.2 µL of 200 mM DMSO solution of Methyl-PEG12-NHS was added, and the mixture was further stirred at room temperature for 6 hours. By allowing the amino groups of DGL G4 and the NHS groups of PEG12-SPDP, AlexaFluor 546 NHS ester, and Methyl-PEG12-NHS to react as described above, a nanoparticle compound SPDP AF5 DGL G4 was obtained. After adding 700 µL of pure water to the reaction solution and mixing, the mixture was purified 6 times by ultrafiltration (manufactured by Merck & Co., Amicon Ultra, molecular weight cut-off 30 kDa) using pure water. Ethanol was added to the collected aqueous solution to adjust to 100 µL of 40% v/v ethanol/aqueous solution.

### (B) Synthesis of azide-PEG5k-SS siRNAAF5 DGL G4

To 8.0 µL of SPDP AF5 DGL G4 solution obtained in (A), 10.6 µL of 5.0 mM PBS solution of siRNA and 8.7 µL of 3 M sodium chloride aqueous solution were added, and the mixture was stirred at 25°C for 8 hours. Next, to this reaction solution, 79.4 µL of 25 mM 30% v/v DMSO/aqueous solution of Azide-PEG5k-Thiol (manufactured by Nanocs Inc., number average molecular weight of PEG: 5000) was added, and the mixture was further stirred for 15 hours. As such, the pyridyl disulfide groups of SPDP AF5 DGL G4 and the SH groups of siRNA and Azide-PEG5k-Thiol were allowed to react. The reaction solution was purified by gel filtration using Hiprep 16/60 Sephacryl S-200 HR (eluent: PBS). Fractions containing siRNA-bonded DGL G4 were collected and concentrated by ultrafiltration (manufactured by Merck & Co., Amicon Ultra, molecular weight cut-off 30 kDa). The obtained solution was purified by gel filtration using TSKgel (registered trademark) G2000swxl (manufactured by Tosoh Corporation). The collected solution was concentrated by ultrafiltration (manufactured by Merck & Co., Amicon Ultra, molecular weight cut-off 30 kDa), and the volume of the liquid was adjusted to 120 µL.

### (C) Synthesis of cRGD-PEGSk-SS siRNAAF5 DGL G4

To 80 µL of PBS solution of azide-PEGSk-SS siRNAAF5 DGL G4 obtained in (B), 7.4 µL of 5 mM DMSO solution of cRGD-DBCO obtained in (C) of Example 1 and 1.5 µL of DMSO were added, and the mixture was stirred at 25°C for 19 hours to allow the azide group of azide-PEGSk-SS siRNAAF5 DGL G4 and the DBCO group of cRGD-DBCO to react. Then, after adding PBS to adjust the liquid volume to 100 µL, purification was performed using NAP-5 Columns. The collected solution was concentrated using ultrafiltration (manufactured by Merck & Co., Amicon Ultra, molecular weight cut-off 30 kDa), and the volume of the solution was adjusted to 110 µL to obtain a solution of oligonucleotide conjugate cRGD-PEGSk-SS siRNAAF5 DGL G4.

### <Example 30. Production of AF6-labeled cRGD-functionalized oligonucleotide conjugate modified with EK peptide, using fourth generation polylysine dendrigraft as core>

### (A) Synthesis of azide-PEG500 SPDP AF6 DGL G4

To 5 µL of 50 mg/mL DMSO solution of DGL G4, 1.5 µL of 150 mM DMSO solution of PEG12-SPDP, 1.5 µL of 10 mM DMSO solution of AlexaFluor 647 NHS ester, 3.8 µL of 100 mM DMSO solution of Azide-PEG12-NHS, and 2.3 µL of 10% v/v DMF solution of TEA were added, and the mixture was stirred at room temperature for 9 hours. Next, 4.2 µL of 200 mM DMSO solution of Methyl-PEG12-NHS was added, and the mixture was further stirred at room temperature for 15 hours. By allowing the amino groups of DGL G4 and the NHS groups of PEG12-SPDP, AlexaFluor 647 NHS ester, Azide-PEG12-NHS, and Methyl-PEG12-NHS to react as described above, a nanoparticle compound azide-PEG500 SPDP AF6 DGL G4 was obtained. After adding 700 µL of pure water to the reaction solution and mixing, the mixture was purified 6 times by ultrafiltration (manufactured by Merck & Co., Amicon Ultra, molecular weight cut-off 30 kDa) using pure water. Pure water was added to the collected aqueous solution to adjust to 100 µL of 40% v/v ethanol/aqueous solution.

### (B) Synthesis of azide-PEG500 siRNA AF6 DGL G4

To 7.0 µL of azide-PEG500 SPDP AF6 DGL G4 solution obtained in (A), 16.1 µL of 5.0 mM PBS solution of siRNA, 2.7 µL of 3 M sodium chloride aqueous solution, and 5.2 µL of DMSO were added, and the mixture was stirred at 25°C for 17 hours. As such, the pyridyl disulfide group of azide-PEG500 SPDP AF6 DGL G4 and the SH group of siRNA were allowed to react. The reaction solution was purified by gel filtration using Hiprep 16/60 Sephacryl S-200 HR (eluent: PBS). Fractions containing siRNA-bonded DGL G4 were collected and concentrated by ultrafiltration (manufactured by Merck & Co., Amicon Ultra, molecular weight cut-off 30 kDa), and the volume of the liquid was adjusted to 80 µL.

### (C) Synthesis of cRGD-EK-maleimide

To 50.0 µL of 20 mM PBS solution of the peptide cRGD-EK-SH (manufactured by GeneDesign, Inc.) shown in the following Formula (XIII), 6.7 µL of 300 mM 50% v/v DMSO/DMF solution of DBCO-maleimide (manufactured by Tokyo Chemical Industry Co., Ltd.) and 20.0 µL of DMF were added, and the mixture was stirred at 4°C for 15 hours to allow the SH group of cRGD-EK-SH and the maleimide group of DBCO-maleimide to react. The solvent was removed under reduced pressure while heating at 45°C. 10 µL of DMF was added to dissolve the sample, 400 µL of pure water was further added, filter filtration (Ultrafree manufactured by Merck & Co.; -MC, GV, 0.22 µm) was performed, and the solvent was removed under reduced pressure while heating at 45°C. The obtained solid was dissolved with 30 µL of DMSO.

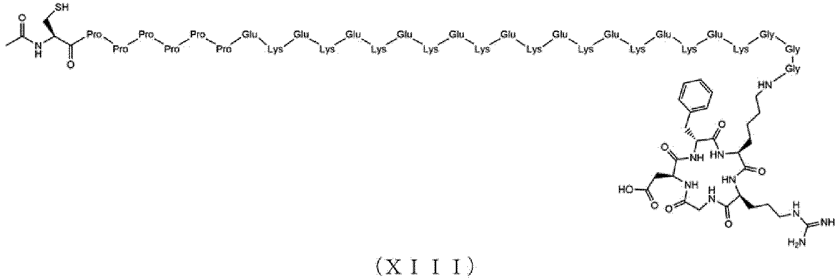

### (D) Synthesis of cRGD-EK siRNA AF6 DGL G4

To 20 µL of PBS solution of azide-PEG500 siRNAAF6 DGL G4 obtained in (B), 4.8 µL of DMSO solution of cRGD-EK-DBCO obtained in (C) and 20 µL of 1 M magnesium chloride aqueous solution (manufactured by Nippon Gene) were added, and the mixture was stirred at 25°C for 18 hours to allow the azide group of azide-PEG500 siRNA AF6 DGL G4 and the DBCO group of cRGD-EK-DBCO to react. After subjecting the precipitated solid to centrifugal sedimentation, the supernatant was removed, 100 µL of PBS was added, and the solution was purified using NAP-5 Columns. The collected solution was concentrated using ultrafiltration (manufactured by Merck & Co., Amicon Ultra, molecular weight cut-off 30 kDa), and the volume of the solution was adjusted to 110 µL to obtain a solution of oligonucleotide conjugate cRGD-EK siRNA AF6 DGL G4.

### <Example 31. Production of AF5-labeled cRGD-functionalized oligonucleotide conjugate modified with mPEG4, using fourth generation polylysine dendrigraft as core>

According to the synthesis of Example 1, oligonucleotide conjugate cRGD-PEGSk siRNA AF5 mPEG4 DGL G4 was synthesized. However, instead of Methyl-PEG12-NHS, m-dPEG, (registered trademark) 4-NHS Ester (manufactured by Quanta Biodesig) was used.

### <Example 32. Production of AF5-labeled cRGD-functionalized oligonucleotide conjugate modified with glycolic acid, using fourth generation polylysine dendrigraft as core>

According to the synthesis of Example 1, oligonucleotide conjugate cRGD-PEGSk siRNA AF5 GA DGL G4 was synthesized. However, Glicolic Acid (manufactured by Sigma-Aldrich) was used instead of Methyl-PEG12-NHS, and at the same time that Glicolic Acid was added, 9.3 µL of 400 mM DMSO solution of EDC-HCl and 9.3 µL of 400 mM DMSO solution of NHS were added.

### <Example 33. Production of AF5-labeled cRGD-functionalized oligonucleotide conjugate modified with sulfobetaine, using fourth generation polylysine dendrigraft as core>

According to the synthesis of Example 1, oligonucleotide conjugate cRGD-PEGSk siRNA AF5 sbeta DGL G4 was synthesized. However, instead of adding Methyl-PEG12-NHS and stirring at 25°C, 3-[[2-(Methacryloyloxy)ethyl]dimethylammonio]propane-1-sulfonate (manufactured by Sigma-Aldrich) was added and stirred at 60°C.

### <Example 34. Production of AF5-labeled cRGD-functionalized oligonucleotide conjugate in which dimethylamine is introduced and fourth generation polylysine dendrigraft is used as core>

According to the synthesis of Example 32, oligonucleotide conjugate cRGD-PEGSk siRNA AF5 tN DGL G4 was synthesized. However, dimethylamino propionic acid hydrochloride (manufactured by Tokyo Chemical Industry Co., Ltd.) was used instead of Glicolic Acid.

### <Example 35. Production of AF5-labeled cRGD-functionalized oligonucleotide conjugate in which butyl group is introduced and fourth generation polylysine dendrigraft is used as core>

According to the synthesis of Example 32, oligonucleotide conjugate cRGD-PEGSk siRNA AF5 nBu DGL G4 was synthesized. However, n-Valeric Acid (manufactured by Kanto Chemical Industry Co., Ltd.) was used instead of Glicolic Acid.

### <Example 36. Production of AF5-labeled cRGD-functionalized oligonucleotide conjugate in which isobutyl group is introduced and fourth generation polylysine dendrigraft is used as core>

According to the synthesis of Example 32, oligonucleotide conjugate cRGD-PEGSk siRNA AF5 iBu DGL G4 was synthesized. However, Isovaleric Acid (manufactured by Tokyo Chemical Industry Co., Ltd.) was used instead of Glicolic Acid.

### <Example 37. Production of AF5-labeled cRGD-functionalized oligonucleotide conjugate in which morpholino group is introduced and fourth generation polylysine dendrigraft is used as core>

According to the synthesis of Example 32, oligonucleotide conjugate cRGD-PEGSk siRNA AF5 MP DGL G4 was synthesized. However, 3-morpholin-4-yl-propionic acid (manufactured by Santa Cruz Biotechnology, Inc.) was used instead of Glicolic Acid.

### <Example 38. Production of AF5-labeled cRGD-functionalized oligonucleotide conjugate in which thiomorpholino group is introduced and fourth generation polylysine dendrigraft is used as core>

According to the synthesis of Example 32, oligonucleotide conjugate cRGD-PEG5k siRNA AF5 TP DGL G4 was synthesized. However, 4-thiomorpholinylacetic acid hydrochloride (manufactured by Fluorochem) was used instead of Glicolic Acid.

### <Comparative Example 1. Production of non-cell-recognizing oligonucleotide conjugate>

### (A) Synthesis of mPEG5k SPDP AF5 DGL G4

To 10 µL of 50 mg/mL DMSO solution of DGL G4, 1.53 µL of 300 mM DMSO solution of PEG12-SPDP, 1.17 µL of 50% v/v DMF solution of TEA, and 1.28 µL of 30 mM DMSO solution of AlexaFluor 546 NHS ester were added, and the mixture was stirred at room temperature for 4.5 hours. Next, to this reaction solution, 68.9 µL of 20 mM DMSO solution of mPEG5k-NHS (manufactured by Iris Biotech GmbH, number average molecular weight of PEG: 5000, lot number: 1217558) was added, and the mixture was further stirred at room temperature for 18 hours. Next, 15.3 µL of 200 mM DMSO solution of Methyl-PEG12-NHS ester was added, and the mixture was further stirred at room temperature for 6 hours. By allowing the amino groups of DGL G4 and the NHS groups of mPEG5k-NHS, PEG12-SPDP, AlexaFluor 546 NHS ester, and Methyl-PEG12-NHS to react as described above, a nanoparticle compound mPEG5k SPDP AF5 DGL G4 was obtained. After adding 400 µL of pure water to the reaction solution and mixing, the mixture was purified 6 times by ultrafiltration (molecular weight cut-off 10 kDa) using pure water. Pure water was added to the collected aqueous solution to adjust the volume of the liquid to 100 µL.

### (B) Synthesis of mPEG5k siRNA AF5 DGL G4

To 10 µL of aqueous solution of mPEG5k SPDP AF5 DGL G4 shown in (A), 6.0 µL of 3 M sodium chloride aqueous solution and 36.0 µL of 5.1 mM PBS solution of siRNA were added, and the mixture was stirred at 25°C for 15 hours to allow the pyridyl disulfide group of mPEG5k SPDP AF5 DGL G4 and the SH group of siRNA to react. The reaction solution was purified by gel filtration using Hiprep 16/60 Sephacryl S-200 HR (eluent: PBS), and fractions containing siRNA-bonded DGL G4 were collected. The collected solution was concentrated using ultrafiltration (molecular weight cut-off 30 kDa), and the volume of the liquid was adjusted to 95 µL to obtain a solution of oligonucleotide conjugate mPEG5k siRNAAF5 DGL G4.

### <Reference Example 1. Production of PEG2000-modified fourth generation polylysine dendrigraft>

### (A) Synthesis of azide-PEG2k AF5 DGL G4

To 3.5 µL of 50 mg/mL DMSO solution of DGL G4, 2.68 µL of 5 mM DMSO solution of AlexaFluor 546 NHS ester and 2.05 µL of 10% v/v DMF solution of TEA were added, and the mixture was stirred at room temperature for 6.5 hours. Next, to this reaction solution, 12.9 µL of 75 mM DMSO solution of Azide-PEG2k-NHS (manufactured by Nanocs Inc., number average molecular weight of PEG: 2000, lot number: 190220), 4.82 µL of 400 mM DMSO solution of NHS, and 4.82 µL of 400 mM DMSO solution of EDC-HCl were added, and the mixture was further stirred at room temperature for 17.5 hours. Next, 4.9 µL of 200 mM DMSO solution of Methyl-PEG12-NHS was added, and the mixture was further stirred at room temperature for 6 hours. By allowing the amino groups of DGL G4 and the NHS groups of Azide-PEG2k-NHS, AlexaFluor 546 NHS ester, and Methyl-PEG12-NHS to react as described above, a nanoparticle compound azide-PEG2k AF5 DGL G4 was obtained. After adding 3 mL of pure water to the reaction solution and mixing, the mixture was purified 6 times by ultrafiltration (molecular weight cut-off 30 kDa) using pure water. The collected aqueous solution was purified by reverse phase HPLC (column: XBridge peptide BEH C18 manufactured by Waters Corporation, 4.6 × 180 mm, eluent A: 0.1% TFA aqueous solution/acetonitrile (90/10; v/v), eluent B: 0.1% TFA aqueous solution/acetonitrile (10/90; v/v)) to separate and collect the target fraction. The solvent in the collected fraction was exchanged with PBS using ultrafiltration (manufactured by Merck & Co., Amicon Ultra, molecular weight cut-off 10 kDa), and the volume of the solution was adjusted to 110 µL.

### (B) Synthesis of Cy7-PEG2k AF5 DGL G4

To 40 µL of azide-PEG2k AF5 DGL G4 obtained in (A), 8.8 µL of 20 mM aqueous solution of Cy7-DBCO (manufactured by Click Chemistry Tools) and 5.4 µL of DMSO were added, and the mixture was stirred at 40°C for 13.5 hours to allow the azide group of azide-PEG2k AF5 DGL G4 and the DBCO group of Cy7-DBCO to react. Then, after adding 47 µL of PBS, the solution was purified using NAP-5 Columns. The collected solution was concentrated using ultrafiltration (molecular weight cut-off 10 kDa), and the volume of the solution was adjusted to 110 µL to obtain a solution of nanoparticle compound Cy7-PEG2k AF5 DGL G4. The concentration of AlexaFluor 546 and the concentration of Cy7 in the obtained solutions were determined from absorabnces at 554 nm and 754 nm, respectively, using an ultraviolet-visible spectrophotometer. In addition, the concentration of DGL G4 was determined by quantitative amino acid analysis using the AQC method described later. From these concentrations, the number of Cy7 bonded to one DGL G4 was calculated. The number of PEG2k determined from the number of Cy7 was 21. Therefore, it can be said that the number of PEG2k in the azide-PEG2k AF5 DGL G4 synthesized in (A) is also 21.

### <Reference Example 2. Production of PEG5000-modified fourth generation polylysine dendrigraft>

### (A) Synthesis of azide-PEG5k AF5 DGL G4

To 7.0 µL of 25 mg/mL DMSO solution of DGL G4, 1.34 µL of 10 mM DMSO solution of AlexaFluor 546 NHS ester and 1.03 µL of 20% v/v DMF solution of TEA were added, and the mixture was stirred at room temperature for 7 hours. Next, to this reaction solution, 16.1 µL of 30 mM DMSO solution of Azide-PEGSk-NHS (manufactured by Nanocs Inc., number average molecular weight of PEG: 5000, lot number: 2005EC), 2.41 µL of 400 mM DMSO solution of NHS, and 2.41 µL of 400 mM DMSO solution of EDC-HCl were added, and the mixture was further stirred at room temperature for 16 hours. Next, 4.9 µL of 200 mM DMSO solution of Methyl-PEG12-NHS was added, and the mixture was further stirred at room temperature for 6 hours. By allowing the amino groups of DGL G4 and the NHS groups of Azide-PEGSk-NHS, AlexaFluor 546 NHS ester, and Methyl-PEG12-NHS to react as described above, a nanoparticle compound azide-PEG5k AF5 DGL G4 was obtained. After adding 3 mL of pure water to the reaction solution and mixing, the mixture was purified 6 times by ultrafiltration (molecular weight cut-off 30 kDa) using pure water. The collected aqueous solution was purified by reverse phase HPLC (column: XBridge peptide BEH C18 manufactured by Waters Corporation, 4.6 × 180 mm, eluent A: 0.1% TFA aqueous solution/acetonitrile (90/10; v/v), eluent B: 0.1% TFA aqueous solution/acetonitrile (10/90; v/v)) to separate and collect the target fraction. The solvent in the collected fraction was exchanged with PBS using ultrafiltration (molecular weight cut-off 10 kDa), and the volume of the solution was adjusted to 200 µL.

### (B) Synthesis of AF405-PEGSk AF5 DGL G4

To 100 µL of azide-PEG5k AF5 DGL G4 obtained in (A), 5.12 µL of 1 mM DMSO solution of AFDye (registered trademark) 405 DBCO (manufactured by Click Chemistry Tools) was added, and the mixture was stirred at room temperature for 30 hours to allow the azide group of azide-PEG5k AF5 DGL G4 and the DBCO group of AFDye 405 DBCO to react. Then, the reaction solution was purified using NAP-5 Columns. The collected solution was concentrated using ultrafiltration (molecular weight cut-off 30 kDa), and the volume of the solution was adjusted to 110 µL to obtain a solution of nanoparticle compound AF405-PEG5k AF5 DGL G4. The concentration of AFDye 405 and the concentration of AlexaFluor 546 in the obtained solutions were determined from absorbances at 405 nm and 554 nm, respectively, using an ultraviolet-visible spectrophotometer. In addition, the concentration of DGL G4 was determined by quantitative amino acid analysis using the AQC method described later. From these concentrations, the number of AFDye 405 bonded to one DGL G4 was calculated. The number of PEG5k determined from the number of AFDye 405 was 26. Therefore, it can be said that the number of PEG5k in the azide-PEG5k AF5 DGL G4 synthesized in (A) is also 26.

### <Reference Example 3. Production of fourth generation polylysine dendrigraft with morpholino group introduced>

To 50 µL of 50 mg/mL DMSO solution of DGL G4, 2.6 µL of 300 mM DMSO solution of PEG12-SPDP and 0.26 µL of 50% v/v DMF solution of TEA were added, and the mixture was stirred at room temperature for 2 hours. 38.3 µL of 20 mM DMSO solution of mPEG2k-NHS (manufactured by Iris Biotech GmbH, number average molecular weight of PEG: 2000) and 1.3 µL of 10% v/v DMF solution of TEA were added, and the mixture was stirred at room temperature for 2 hours. Then, 187.2 µL of 100 mM DMSO solution of 3-morpholin-4-yl-propionic acid, 93.6 µL of 400 mM DMSO solution of EDC-HCl, and 93.6 µL of 400 mM DMSO solution of NHS were mixed, 31.3 µL of 10% v/v DMF solution of TEA was added, and the mixture was stirred at room temperature overnight. By allowing the amino groups of DGL G4 to react with the COOH group of 3-morpholin-4-yl-propionic acid and the NHS groups of PEG12-SPDP and mPEG2k-NHS as described above, a nanoparticle compound mPEG2k SPDP MP DGL G4 was obtained. After adding 700 µL of pure water to the reaction solution and mixing, the mixture was purified 6 times by ultrafiltration (manufactured by Merck & Co., Amicon Ultra, molecular weight cut-off: 10 kDa) using pure water. Pure water was added to the collected aqueous solution to adjust the volume of the liquid to 100 µL.

### <Reference Example 4. Production of fourth generation polylysine dendrigraft with thiomorpholino group introduced>

According to the synthesis of Reference Example 3, a nanoparticle compound mPEG2k SPDP TP DGL G4 was synthesized. However, 4-thiomorpholinylacetic acid hydrochloride was used instead of 3-morpholin-4-yl-propionic acid.

### <Reference Example 5. Production of fourth generation polylysine dendrigraft with mPEG12 introduced>

According to the synthesis of Reference Example 3, a nanoparticle compound mPEG2k SPDP mPEG12 DGL G4 was synthesized. However, Methyl-PEG12-NHS was used instead of 3-morpholin-4-yl-propionic acid.

### <Reference Example 6. Productionof nanoparticle compound for evaluation of oligonucleotide conjugate of Example 13>

According to the synthesis of Example 13, a nanoparticle compound AF4-PEGSk siRNA AF6 DGL G4 was synthesized. However, instead of IND-DBCO, AFDye405 DBCO (manufactured by Click Chemistry Tools) was used. It can be said that the number of fluorescent molecules AFDye405 in the obtained nanoparticle compound is equal to the number of indatraline in the oligonucleotide conjugate obtained in Example 13.

### <Reference Example 7. Production of nanoparticle compound for evaluation of oligonucleotide conjugates of Examples 14 to 17>

According to the synthesis of Example 14, a nanoparticle compound AF4-PEGSk siRNA AF6 DGL G4 was synthesized. However, instead of NU1-DBCO, AFDye405 DBCO (manufactured by BroadPharm) was used. It can be said that the number of fluorescent molecules AFDye405 in the obtained nanoparticle compound is equal to the number of hydrophilic linkers (that is, the number of azide groups) in the nanoparticle compound azide-PEG5k siRNA AF6 DGL G4 obtained in Example 14(B).

### <Reference Example 8. Production of oligonucleotide conjugate for evaluation of oligonucleotide conjugate of Example 14>

To 30 µL of PBS solution of oligonucleotide conjugate obtained in Example 14, 1.6 µL of PBS solution of AFDye405 DBCO and 3.5 µL of DMSO were added, and the mixture was stirred at 25°C for 14 hours. The reaction solution was purified using Zeba (registered trademark) Spin Desalting Column (manufactured by Thermo Fisher Scientific Inc., molecular weight cut-off 40 kDa). Next, purification was performed 3 times using PBS by ultrafiltration (manufactured by Merck & Co., Amicon Ultra, molecular weight cut-off 30 kDa), and the volume of the liquid was adjusted to 70 µL to obtain a solution of oligonucleotide conjugate AF4-NU1-PEGSk siRNA AF6 DGL G4. It can be said that the number of fluorescent molecules AFDye405 in the obtained oligonucleotide conjugate is equal to the number of unreacted hydrophilic linkers (that is, unreacted azide groups) in the oligonucleotide conjugate obtained in Example 14. Therefore, the number of aptamers AS 1411 in the obtained oligonucleotide conjugate AF4-NU1-PEGSk siRNA AF6 DGL G4 can be calculated by subtracting the number of fluorescent molecules AFDye405 in the oligonucleotide conjugate AF4-NLT1-PEGSk siRNA AF6 DGL G4 from the number of fluorescent molecules AFDye405 in the nanoparticle compound AF4-PEG5k siRNA AF6 DGL G4 obtained in Reference Example 7, and it can be said that this value is equal to the number of aptamers AS 1411 in the oligonucleotide conjugate obtained in Example 14.

### <Reference Example 9. Production of oligonucleotide conjugate for evaluation of oligonucleotide conjugate of Example 15>

According to the synthesis of Reference Example 8, a solution of oligonucleotide conjugate AF4-NU2-PEGSk siRNA AF6 DGL G4 was obtained. However, instead of the oligonucleotide conjugate obtained in Example 14, the oligonucleotide conjugate obtained in Example 15 was used.

### <Reference Example 10. Production of oligonucleotide conjugate for evaluation of oligonucleotide conjugate of Example 16>

According to the synthesis of Reference Example 8, a solution of oligonucleotide conjugate AF4-EP1-PEGSk siRNA AF6 DGL G4 was obtained. However, instead of the oligonucleotide conjugate obtained in Example 14, the oligonucleotide conjugate obtained in Example 16 was used.

### <Reference Example 11. Production of oligonucleotide conjugate for evaluation of oligonucleotide conjugate of Example 17>

According to the synthesis of Reference Example 8, a solution of oligonucleotide conjugate AF4-EP2-PEGSk siRNA AF6 DGL G4 was obtained. However, instead of the oligonucleotide conjugate obtained in Example 14, the oligonucleotide conjugate obtained in Example 17 was used.

### <Test Example 1. Evaluation of oligonucleotide conjugate>

The oligonucleotide conjugates of Examples and Comparative Examples were evaluated as follows.

### (A) Evaluation of number of oligonucleotides, cellular internalization enhancers, and fluorescent molecules

The concentrations of oligonucleotide, cellular internalization enhancer, and fluorescent molecule in the oligonucleotide conjugate samples of Examples and Comparative Examples were determined as follows. The concentrations of oligonucleotides, folic acids, and fluorescent molecules were obtained from absorbances at the following wavelengths using an ultraviolet-visible spectrophotometer: 260 nm for oligonucleotides (siRNA and antisense oligonucleotides), 300 nm for folic acid, 402 nm for AFDye405, 554 nm for AlexaFluor 546, 651 nm for AlexaFluor 647, and 749 nm for Tide Fluor 7WS. In addition, the concentrations of the dendritic polymer and the polypeptide-based cellular internalization enhancer in the sample were quantified by the quantitative amino acid analysis (PTC method or AQC method) shown below. The PTC method was used for the samples of Example 1 and Comparative Example 1, and the AQC method was used for the samples of other Examples.

Regarding the number of indatraline in the oligonucleotide conjugate sample of Example 13, a nanoparticle compound (Reference Example 6) in which indatraline in the oligonucleotide conjugate of Example 13 is substituted with a fluorescent molecule AFDye405 was synthesized, and the number of AFDye405 in the nanoparticle compound was determined and deemed as the number of indatraline in the oligonucleotide conjugate of Example 13.

Regarding the oligonucleotide conjugate samples of Examples 14 to 17 having an aptamer as a cellular internalization enhancer, the number of oligonucleotides in the sample was determined by determining the number of oligonucleotides in the synthetic intermediate (nanoparticle compound) before the aptamer was allowed to react. In addition, the number of aptamers in the sample was determined as follows. First, a nanoparticle compound (Reference Example 7) in which the aptamers in the oligonucleotide conjugates of Examples 14 to 17 were substituted with a fluorescent molecule AFDye405 was synthesized, and the number of fluorescent molecules in the nanoparticle compound (which is equal to the number of hydrophilic linkers in the synthetic intermediate before the aptamer was allowed to react) was determined. Separately, oligonucleotide conjugates (Reference Examples 8 to 11) in which the oligonucleotide conjugates of Examples 14 to 17 were further allowed to react with a fluorescent molecule AFDye405 were prepared, and the number of AFDye405 in the oligonucleotide conjugates (which is equal to the number of unreacted hydrophilic linkers left after being allowed to react with the aptamer) was determined. Then, the difference in the number of these AFDye405 was calculated and deemed as the number of aptamers in the oligonucleotide conjugates of Examples 14 to 17.

From these concentrations, the number of oligonucleotides, cellular internalization enhancers, and fluorescent molecules bonded to one dendritic polymer was calculated. The calculation results are shown in each Test Example or Table 3 below.

**[Table 3]**

| Sample | IND-PEG5k siRNA AF6 DGLG4 | EP 1-PEGSk siRNA AF6 DGLG4 | EP2-PEG5k siRNA AF6 DGL G4 | cRGD-PEG5k siRNA AF6 MPA G6 |
|---|---|---|---|---|
| Corresponding Example | 13 | 16 | 17 | 20 |
| Dendritic polymer | DGL G4 | DGL G4 | DGL G4 | Bis-MPA G6 |

| **Oligonucleotide** | Atp5b-siRNA | Atp5b-siRNA | Atp5b-siRNA | Atp5b-siRNA |
|---|---|---|---|---|
| **Number of oligonucleotides** | 20.3 | 15.8 | 15.8 | 5 |
| **Hydrophilic linker** | PEG5k | PEG5k | PEG5k | PEG5k |
| **Cellular internalization enhancer** | **Indatraline** | **EpCAM Aptamer represented by SEQ ID NO: 3** | **EpCAM Aptamer represented by SEQ ID NO:** 4 | **cRGDfK** |
| **Number of cellular internalization enhancers** | 28.1 | 18.8 | 15 | 11.9 |
| **Fluorescent molecule** | AlexaFluor 647 | AlexaFluor 647 | AlexaFluor 647 | AlexaFluor 647 |
| **Number of fluorescent molecules** | 4 | 1.9 | 2.3 | 2.8 |
| **Capping agent** | Methyl-PEG 12 | Methyl-PEG12 | Methyl-PEG 12 | Methyl-PEG 12 |

| **Sample** | cRGD-PEG5k siRNA-C3 AF6 DGL G4 | c(avb6)-PEG5k ASO AF6 DGL G4 | cRGD-PEG5k-SS siRNA AF5 DGL G4 | cRGD-EK siRNA AF6 DGL G4 |
|---|---|---|---|---|
| **Corresponding Example** | 21 | 22 | 29 | 30 |
| **Dendritic polymer** | DGL G4 | DGL G4 | DGL G4 | DGLG4 |
| **Oligonucleotide** | scramble-siRNA | Malatl-ASO | scramble-siRNA | AtpSb-siRNA |
| **Number** of **oligonucleotides** | 2.6 | 28.4 | 32.7 | 28.4 |
| **Hydrophilic linker** | PEG5k | PEG5k | PEG5k | EK peptide |
| **Cellular internalization enhancer** | cRGDfK | c(avb6) | cRGDfK | cRGDfK |
| **Number of cellular internalization enhancers** | 26 | 25.7 | 10.2 | 20.6 |
| **Fluorescent molecule** | AlexaFluor 647 | AlexaFluor 647 | AlexaFluor 546 | AlexaFluor 647 |
| **Number of fluorescent molecules** | 2.9 | 3.6 | 4.4 | 3.1 |
| **Capping agent** | Methyl-PEG12 | Methyl-PEG12 | Methyl-PEG12 | Methyl-PEG12 |

### (A-1) Quantitative amino acid analysis (PTC method)

30 µL of aqueous solution of the oligonucleotide conjugate sample and 300 µL of constant boiling hydrochloric acid were added to a sealable glass bottle, sealed, and hydrolyzed by heating at 105°C for 24 hours. After removing the solvent under reduced pressure while heating at 45°C, 150 µL of mixed solution of acetonitrile/pyridine/TEA/pure water (10/5/2/3; v/v/v/v) was added, and the solvent was removed under reduced pressure while heating at 45°C. 150 µL of mixed solution of acetonitrile/pyridine/TEA/pure water/PITC (10/5/2/3/1; v/v/v/v/v) was added and stirred at 25°C for 30 minutes, and then the solvent was removed under reduced pressure while heating at 45°C. The PITC used is manufactured by FUJIFILM Wako Pure Chemical Corporation. The obtained solid was analyzed by reverse phase HPLC (column: Wakopak Wakosil-PTC manufactured by FuJIFILM Wako Pure Chemical Corporation, 4.0 × 250 mm, eluent A: PTC-Amino Acids Mobile Phase A, eluent B: PTC-Amino Acids Mobile Phase B), the concentration of DGL G4 was quantified from the peak area of the lysine residue peak, and the concentration of cRGDfK was quantified from the peak area of the phenylalanine residue peak.

### (A-2) Quantitative amino acid analysis (AQC method)

30 µL of aqueous solution of the oligonucleotide conjugate sample and 300 µL of constant boiling hydrochloric acid were added to a sealable glass bottle, sealed, and hydrolyzed by heating at 110°C for 24 hours. After hydrolysis, the solvent was removed under reduced pressure while heating at 45°C. AQC (manufactured by Adipogen Life Sciences) was dissolved in acetonitrile (super dehydrated) at 60°C and adjusted to 3 mg/mL. To the glass bottle containing the dried oligonucleotide conjugate sample, 30 µL of 20 mM hydrochloric acid, 90 µL of 0.2 M borate buffer solution (pH 8.8), and 30 µL of 3 mg/mL AQC acetonitrile solution were added, stirred, and allowed to stand at 60°C. After incubation for 10 minutes, the solvent was removed under reduced pressure while heating at 45°C. The obtained solid was dissolved in 150 µL of eluent A, filter filtration (Ultrafree manufactured by Merck & Co.; -MC, GV, 0.22 µm) was performed. The obtained filtrate was analyzed by reverse phase HPLC (column: AccQ-Tag Column, 60 Å, 4 µm 3.9 × 150 mm, eluent A: AccQ-Tag Eluent A/water (1/9; v/v), eluent B: water/acetonitrile (1/1; v/v)), the concentrations of DGL G3, DGL G4, and DGL G5 were quantified from the peak areas of the lysine residue peaks, and the concentrations of PAMAM G5, PAMAM G6, Bis-MPA dendrimer, cRGDfK, c(avb6), and GE11 were quantified from the peak areas of each unique peak. AccQ-Tag Column and AccQ-Tag Eluent A were purchased from Waters Corporation.

### (B) Evaluation of particle size distribution of oligonucleotide conjugate

The average particle diameter of mPEG5k siRNA AF5 DGL G4 obtained in Comparative Example 1 was measured using a particle size analyzer (Zetasizer Nano ZS manufactured by Malvern Panalytical). ZEN0040 manufactured by Malvern Panalytical was used as a cell, and the measurement was performed by a dynamic light scattering method. Table 4 shows the results obtained.

**[Table 4]**

| Sample | Average particle diameter (based on scattering intensity) |
|---|---|
| mPEG siRNA AF5 DGL G4 | 36.2 nm |

### (C) Evaluation of thickness h of hydration layer of oligonucleotide conjugate

As described above, in order to obtain the average linear distance between the ends of hydrophilic linkers bonded to a dendritic polymer, a method using a plurality of types of dendritic polymers to which the hydrophilic linkers of different lengths are bonded, can be used. Specifically, by measuring these particle diameters and creating a calibration curve between the molecular weight of the hydrophilic linker and the distance between the ends of the hydrophilic linker, the distance between the ends of the hydrophilic linkers of experimentally unused molecular weights can be deduced. As an example, the case where PEG was used as a hydrophilic linker and two types of samples were used to create a calibration curve is shown below.

The thickness h of the hydration layer (that is, the average linear distance between the ends of PEG5k) of the oligonucleotide conjugate of Example was obtained as follows. First, the average particle diameters of the nanoparticle compound azide-PEG2k AF5 DGL G4 in Reference Example 1 and the nanoparticle compound azide-PEG5k AF5 DGL G4 in Reference Example 2 were measured by a multi-angle dynamic light scattering method using a particle size analyzer (Zetasizer Ultra manufactured by Malvern Panalytical). As a cell, a Sarstedt cuvette (product number: 67.754) was used. The results are shown in Table 5. Next, from the difference between the average particle diameter of azide-PEG5k AF5 DGL G4 and the average particle diameter of azide-PEG2k AF5 DGL G4, the thickness of the hydration layer per unit molecular weight of PEG was calculated. Specifically, the thickness of the hydration layer per PEG with molecular weight of 1000 was calculated as (32.9 - 21.5)/[2 × (5000-2000)] × 1000 = 1.9 nm. From this result, the thickness h of the hydration layer of the oligonucleotide conjugate of the Examples having PEG with a molecular weight of 5000 as the hydrophilic linker was calculated as 5000/1000 × 1.9 = 9.5 nm. In other words, it can be said that the average linear distance between the ends of the hydrophilic linker PEG5k is 9.5 nm. On the other hand, in Examples, siRNA (molecular length: approximately 5 nm) was bonded to the dendritic polymer through PEG12 (PEG with a molecular weight of approximately 500) and Spacer18 (PEG with a molecular weight of approximately 250), and thus the average linear distance from the core surface to the free end of siRNA is calculated as 5 + (500 + 250)/1000 × 1.9 = 6.4 nm. Therefore, in the oligonucleotide conjugates of Examples, it can be said that the average linear distance between the ends of the hydrophilic linker PEG5k is longer than the length of siRNA and longer than the average linear distance from the core surface to the free end of the siRNA. However, this analysis result and this discussion are limited to cases where the nanoparticle compounds of Reference Examples 1 and 2 are used as samples and it is assumed that the PEG molecular weight and the thickness of the hydration layer are in a linear relationship. Although two-point approximation is shown as an example, multi-point approximation is desirable for more accurate analysis.

**[Table 5]**

| Sample | Molecular weight of PEG | Average particle diameter (based on scattering intensity) |
|---|---|---|
| azide-PEG2k AF5 DGL G4 | 2000 | 21.5 nm |
| azide-PEG5k AF5 DGL G4 | 5000 | 32.9 nm |

### (D) Comparison of average linear distance between ends of hydrophilic linkers and length of oligonucleotide

The average particle diameters of the nanoparticle compound azide-PEG5k SPDP AF5 DGL G4 obtained in (A) of Example 25 and the nanoparticle compound azide-PEG5k siRNAAF5 DGL G4 (synthesized in (B) of Example 25) obtained by bonding siRNA to the nanoparticle compound were measured by a multi-angle dynamic light scattering method using a particle size analyzer (Zetasizer Ultra manufactured by Malvern Panalytical). As a cell, a Sarstedt cuvette (product number: 67.754) was used. Similarly, the average particle diameters of the nanoparticle compound azide-PEGlOk SPDP AF5 DGL G4 obtained in the synthesis process of Example 26 and the nanoparticle compound azide-PEGlOk siRNAAF5 DGL G4 (acquired in the synthesis process of Example 26) obtained by bonding siRNA to the nanoparticle compound were measured. The results are shown in Table 6.

**[Table 6]**

| Sample | azide-PEG5k AF5 DGLG4 | azide-PEG5k siRNAAF5 DGLG4 | azide-PEG10k AF5 DGLG4 | azide-PEG10k siRNAAF5 DGL G4 |
|---|---|---|---|---|
| Corresponding Example | Synthetic intermediate obtained in 25(A) | Synthetic intermediate obtained in 25(B) | Synthetic intermediate obtained in 26 | Synthetic intermediate obtained in 26 |
| Dendritic polymer | DGL G4 | DGL G4 | DGL G4 | DGL G4 |
| Oligonucleotide | scramble-siRNA | scramble-siRNA | scramble-siRNA | scramble-siRNA |
| Number of oligonucleotide s | - | 16.7 | - | 15.3 |
| Hydrophilic linker | PEG5k | PEG5k | PEG10k | PEG10k |
| Number of hydrophilic linkers | 31.0 | 31.0 | 21.8 | 21.8 |
| Capping agent | Methyl-PEG 12 | Methyl-PEG12 | Methyl-PEG12 | Methyl-PEG12 |
| Fluorescent molecule | AlexaFluor 546 | AlexaFluor 546 | AlexaFluor 546 | AlexaFluor 546 |
| Number of fluorescent molecules | 1.8 | 1.8 | 4.5 | 4.5 |
| Average particle diameter (based on scattering intensity) | 23.5 nm | 28.9 nm | 33.9 nm | 32.7 nm |

When PEG5k (PEG with a molecular weight of 5000) was used as the hydrophilic linker, the average particle diameter of the nanoparticle compound increased by 5.4 nm by bonding scramble-siRNAs to the core. From this result, it can be said that the average linear distance from the core surface to the free end of the siRNA is 2.7 nm longer than the average linear distance between the ends of the hydrophilic linker PEG5k. Therefore, contrary to the results in (C) of Test Example 1, in the oligonucleotide conjugates of Examples having a hydrophilic linker PEG5k, it was found that the average linear distance between the ends of the hydrophilic linker PEG5k was shorter than the average linear distance from the core surface to the free end of siRNA.

On the other hand, when PEG10k (PEG with a molecular weight of 10000) was used as the hydrophilic linker, the average particle diameter of the nanoparticle compound with scramble-siRNAs conjugated to the core was almost the same as that without scramble-siRNAs. From this result, it can be said that the average linear distance between the ends of the hydrophilic linker PEG5k is longer than the average linear distance from the core surface to the free end of the siRNA.

Note that, since the average linear distance from the core surface to the free end of the siRNA is 2.7 nm longer than the average linear distance between the ends of the hydrophilic linker PEG5k, if the average linear distance between the ends of the hydrophilic linker PEG5k is 1.35 nm or more and less than 2.7 nm, it can be said that the average linear distance is 1/3 or more and less than half the length from the core surface to the free end of the oligonucleotide. Further, if the average linear distance between the ends of the hydrophilic linker PEG5k is 2.7 nm or more, it can be said that the average linear distance is half or more of the length from the core surface to the free end of the oligonucleotide.

However, this analysis result is limited to this synthesis lot, and are not generalized when materials of the same molecular weight are used. A similar analysis should be conducted for each synthetic lot to compare the average linear distance between the ends of the hydrophilic linkers and the length of the oligonucleotide.

### <Test Example 2. in vitro evaluation of cRGD ligand-functionalized oligonucleotide conjugate>

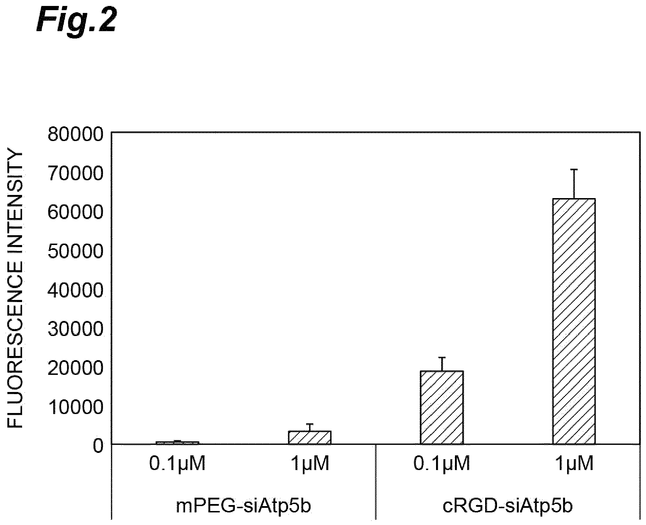

U-87MG cells (human glioblastoma cell line) were seeded in a 96-well plate and cultured in a DMEM medium containing 10% FBS at 37°C with 5% CO₂. The next day, the medium was exchanged, and the sample was added to each well to transfect the cells, which were then cultured at 37°C with 5% CO₂. The concentrations of siRNA for transfection were 0.1 µM or 1 µM. 48 hours after transfection, the cells were washed with PBS and then fluorescence intensity was measured (excitation wavelength 540 nm, fluorescence wavelength 585 nm). Furthermore, the siRNA concentration was converted to the concentration of the fluorescent molecules from the ratio of the number of siRNAs and the number of fluorescent molecules (number of siRNAs/number of fluorescent molecules), and after approximating that the fluorescence intensity and the concentration of the fluorescent molecules were in a direct proportional relationship, the fluorescence intensity when the concentration of the fluorescent molecules was 0.1 µM or 1 µM was calculated. The used samples are shown in Table 7 and the obtained results are shown in FIG. 2.

In Table 7, mPEG-siAtp5b corresponds to Comparative Example 1 and cRGD-siAtp5b corresponds to Example 1.

**[Table 7]**

| Sample | mPEG-siAtp5b | \| cRGD-siAtp5b |
|---|---|---|
| Corresponding Example/ Comparative Example | Comparative Example 1 | Example 1 |
| Dendritic polymer | DGL G4 | DGL G4 |
| Oligonucleotide | Atp5b-siRNA | Atp5b-siRNA |
| Number of oligonucleotides | 20.8 | 35.5 |
| Hydrophilic linker | PEG5k | PEG5k |
| Cellular internalization enhancer | | cRGDfk |
| Number of cellular internalization enhancers | - | 25.4 |
| Fluorescent molecule | AlexaFluor 546 | AlexaFluor 546 |
| Number of fluorescent molecules | 6.7 | 16.9 |
| Capping agent | Methyl-PEG12 | Methyl-PEG12 |

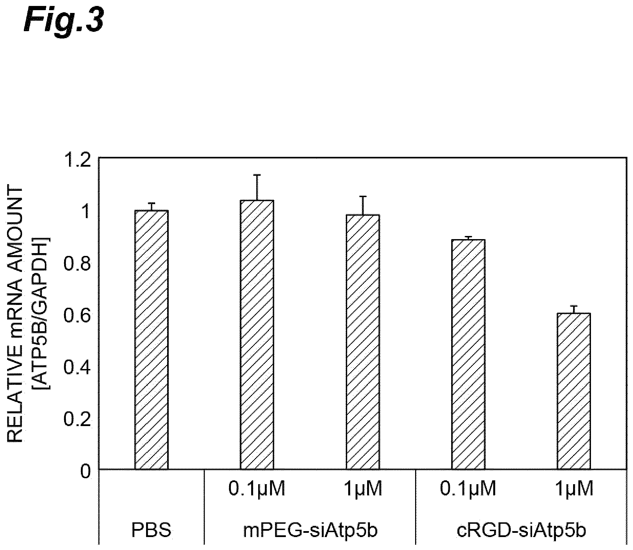

U-87MG cells (human glioblastoma cell line) were seeded in a 96-well plate and cultured in a DMEM medium containing 10% FBS at 37°C with 5% CO₂. The next day, the medium was exchanged, and the sample was added to each well to transfect the cells, which were then cultured at 37°C with 5% CO₂. The concentrations of siRNA for transfection were 0.1 µM or 1 µM. For the control group, PBS was added instead of sample. 48 hours after transfection, mRNA was extracted using RNeasy Mini Kit (manufactured by Qiagen), and cDNA was synthesized from a fixed amount of mRNA using High Capacity RNA-to-cDNA Kit (Applied Biosystems (registered trademark)). Subsequently, quantitative RT-PCR was performed using the obtained cDNA as a template and using PowerUp SYBR Green Master Mix (Applied Biosystems). As ATP5B primers, primers of SEQ ID NO: 12 and SEQ ID NO: 13 shown in Table 8 below were used, and as GAPDH primers, primers of SEQ ID NO: 14 and SEQ ID NO: 15 shown in Table 8 below were used. PCR conditions (temperature and time) were as follows. One cycle was designed to be 95°C for 1 second and 60°C for 30 seconds, and 40 cycles were performed. Based on the results of quantitative RT-PCR, the value of "hATP5B expression level/hGAPDH (internal standard gene) expression level" was calculated, and the calculation result for the control group and the calculation result for the sample addition group were compared. The used samples are shown in Table 7 and the obtained results are shown in FIG. 3.

**[Table 8]**

| | |
|---|---|
| ATP5B-forward direction (SEQ ID NO: 12) | 5'-GGTCCTGAGACTTTGGGCAGAA-3' |
| ATP5B-reverse direction (SEQ ID NO: 13) | 5'-CCTCAGCATGAATGGGAGCA-3' |
| GAPDH-forward direction (SEQ ID NO: 14) | 5'-GCACCGTCAAGGCTGAGAAC-3' |
| GAPDH-reverse direction (SEQ ID NO: 15) | 5'-TGGTGAAGACGCCAGTGGA-3' |

### <Test Example 3. Evaluation of siRNA sequence-specific knockdown efficiency using cRGD ligand-functionalized oligonucleotide conjugate>

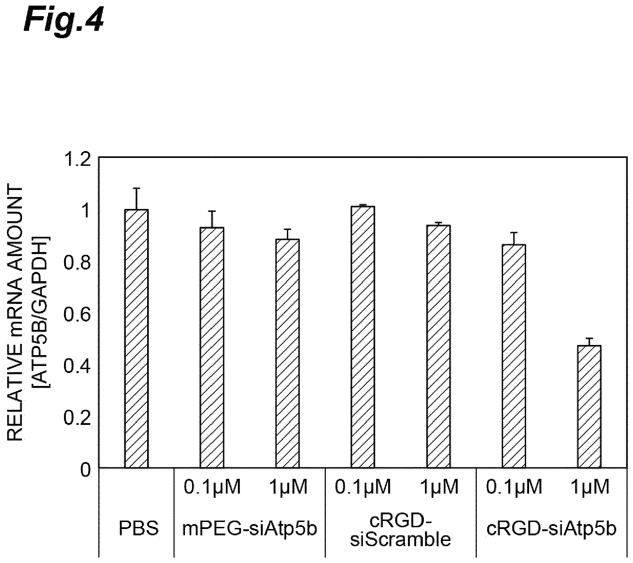

U-87MG cells (human glioblastoma cell line) were seeded in a 96-well plate and cultured in a DMEM medium containing 10% FBS at 37°C with 5% CO₂. The next day, the medium was exchanged, and the sample was added to each well to transfect the cells, which were then cultured at 37°C with 5% CO₂. The concentrations of siRNA for transfection were 0.1 µM or 1 µM. For the control group, PBS was added instead of sample. 48 hours after transfection, mRNA was extracted using RNeasy Mini Kit (manufactured by Qiagen), and cDNA was synthesized from a fixed amount of mRNA using High Capacity RNA-to-cDNA Kit (Applied Biosystems). Subsequently, quantitative RT-PCR was performed using the obtained cDNA as a template and using PowerUp SYBR Green Master Mix (Applied Biosystems). As ATP5B primers, primers of SEQ ID NO: 12 and SEQ ID NO: 13 shown in Table 8 above were used, and as GAPDH primers, primers of SEQ ID NO: 14 and SEQ ID NO: 15 shown in Table 8 above were used. PCR conditions (temperature and time) were as follows. One cycle was designed to be 95°C for 1 second and 60°C for 30 seconds, and 40 cycles were performed. Based on the results of quantitative RT-PCR, the value of "hATP5B expression level/hGAPDH (internal standard gene) expression level" was calculated, and the calculation result for the control group and the calculation result for the sample addition group were compared. The used samples are shown in Table 9 and the obtained results are shown in FIG. 4.

In Table 9, mPEG-siAtp5b corresponds to Comparative Example 1 and the remaining samples correspond to Example 1.

**[Table 9]**

| Sample | mPEG-siAtp5b | cRGD-siScramble | cRGD-siAtp5b |
|---|---|---|---|
| Corresponding Example/Comparative Example | Comparative Example 1 | Example 1 | Example 1 |
| Dendritic polymer | DGL G4 | DGL G4 | DGL G4 |
| Oligonucleotide | Atp5b-siRNA | scramble-siRNA | Atp5b-siRNA |
| Number of oligonucleotides | 20.8 | 18.0 | 28.0 |
| Hydrophilic linker | PEG5k | PEG5k | PEG5k |
| Cellular internalization enhancer | - | cRGDfK | cRGDfK |
| Number of cellular internalization enhancers | - | 26.2 | 25.6 |
| Fluorescent molecule | AlexaFluor 546 | AlexaFluor 546 | AlexaFluor 546 |
| Number of fluorescent molecules | 6.7 | 5.8 | 6.1 |
| Capping agent | Methyl-PEG12 | Methyl-PEG12 | Methyl-PEG12 |

### <Test Example 4. in vitro evaluation of GE11 ligand-functionalized oligonucleotide conjugate>

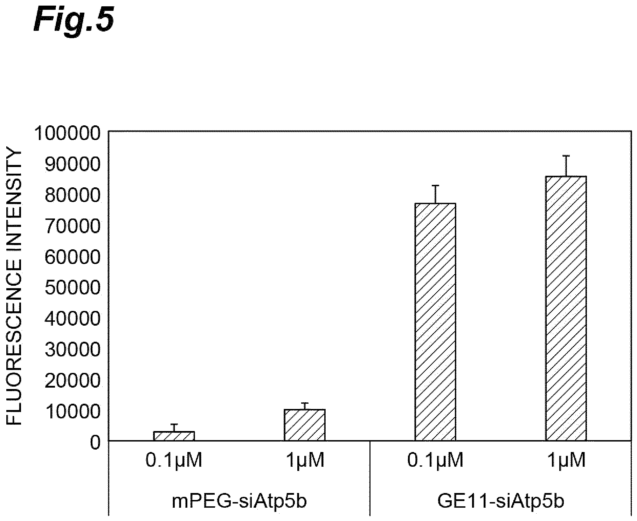

A431 cells (human squamous cell carcinoma cell line) were seeded in a 96-well plate and cultured in a DMEM medium containing 10% FBS at 37°C with 5% CO₂. The next day, the medium was exchanged, and the sample was added to each well to transfect the cells, which were then cultured at 37°C with 5% CO₂. The concentrations of siRNA for transfection were 0.1 µM or 1 µM. 48 hours after transfection, the cells were washed with PBS and then fluorescence intensity was measured (excitation wavelength 540 nm, fluorescence wavelength 585 nm). Furthermore, the siRNA concentration was converted to the concentration of the fluorescent molecules from the ratio of the number of siRNAs and the number of fluorescent molecules (number of siRNAs/number of fluorescent molecules), and after approximating that the fluorescence intensity and the concentration of the fluorescent molecules were in a direct proportional relationship, the fluorescence intensity when the concentration of the fluorescent molecules was 0.1 µM or 1 µM was calculated. The used samples are shown in Table 10 and the obtained results are shown in FIG. 5.

In Table 10, mPEG-siAtp5b corresponds to Comparative Example 1 and GE11-siAtp5b corresponds to Example 2.

**Table 10]**

| Sample | mPEG-siAtp5b | GE11-siAtp5b |
|---|---|---|
| Corresponding Example/Comparative Example | Comparative Example 1 | Example 2 |
| Dendritic polymer | DGL G4 | DGL G4 |
| Oligonucleotide | Atp5b-siRNA | Atp5b-siRNA |
| Number of oligonucleotides | 20.8 | 21.5 |
| Hydrophilic linker | PEG5k | PEG5k |
| Cellular internalization enhancer | - | GE11 |
| Number of cellular internalization enhancers | 0 | 19.1 |
| Fluorescent molecule | AlexaFluor 546 | AlexaFluor 546 |
| Number of fluorescent molecules | 6.7 | 8.2 |
| Capping agent | Methyl-PEG 12 | Methyl-PEG12 |

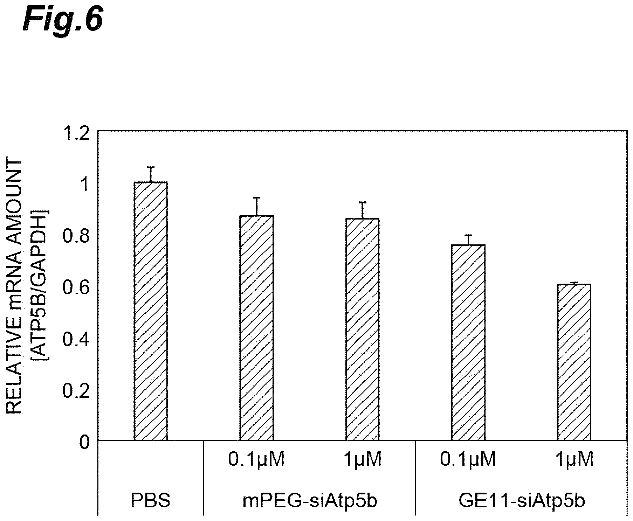

A431 cells (human squamous cell carcinoma cell line) were seeded in a 96-well plate and cultured in a DMEM medium containing 10% FBS at 37°C with 5% CO₂. The next day, the medium was exchanged, and the sample was added to each well to transfect the cells, which were then cultured at 37°C with 5% CO₂. The concentrations of siRNA for transfection were 0.1 µM or 1 µM. For the control group, PBS was added instead of sample. 48 hours after transfection, mRNA was extracted using RNeasy Mini Kit (manufactured by Qiagen), and cDNA was synthesized from a fixed amount of mRNA using High Capacity RNA-to-cDNA Kit (Applied Biosystems). Subsequently, quantitative RT-PCR was performed using the obtained cDNA as a template and using PowerUp SYBR Green Master Mix (Applied Biosystems). As ATP5B primers, primers of SEQ ID NO: 12 and SEQ ID NO: 13 shown in Table 8 above were used, and as GAPDH primers, primers of SEQ ID NO: 14 and SEQ ID NO: 15 shown in Table 8 above were used. PCR conditions (temperature and time) were as follows. One cycle was designed to be 95°C for 1 second and 60°C for 30 seconds, and 40 cycles were performed. Based on the results of quantitative RT-PCR, the value of "hATP5B expression level/hGAPDH (internal standard gene) expression level" was calculated, and the calculation result for the control group and the calculation result for the sample addition group were compared. The used samples are shown in Table 10 and the obtained results are shown in FIG. 6.

### <Test Example 5. in vitro evaluation of cRGD ligand-functionalized oligonucleotide conjugate using PAMAM as core>

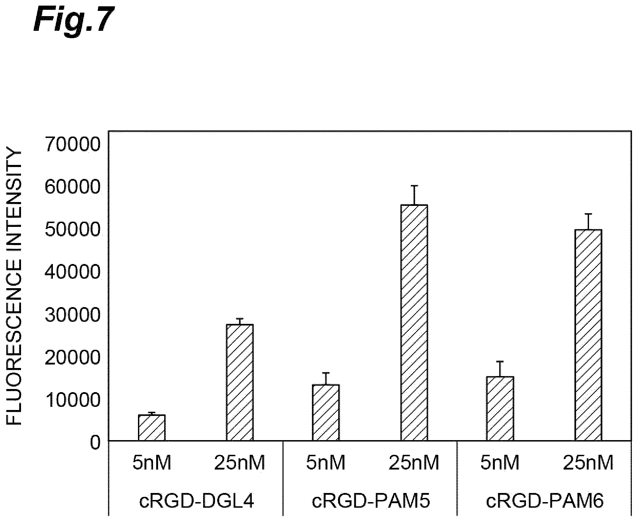

U-87MG cells (human glioblastoma cell line) were seeded in a 96-well plate and cultured in a DMEM medium containing 10% FBS at 37°C with 5% CO₂. The next day, the medium was exchanged, and the sample was added to each well to transfect the cells, which were then cultured at 37°C with 5% CO₂. The concentrations of dendrimer for transfection were 2 nM or 10 nM. 48 hours after transfection, the cells were washed with PBS and then fluorescence intensity was measured (excitation wavelength 740 nm, fluorescence wavelength 780 nm). Furthermore, the dendrimer concentration was converted to the concentration of the fluorescent molecules from the number of fluorescent molecules, and after approximating that the fluorescence intensity and the concentration of the fluorescent molecules were in a direct proportional relationship, the fluorescence intensity when the concentration of the fluorescent molecules was 5 nM or 25 nM was calculated. The used samples are shown in Table 11 and the obtained results are shown in FIG. 7.

In Table 11, cRGD-DGL4 corresponds to Example 5, cRGD-PAM5 corresponds to Example 3, and cRGD-PAM6 corresponds to Example 4.

**[Table 11]**

| Sample | cRGD-DGL4 | cRGD-PAM5 | cRGD-PAM6 |
|---|---|---|---|
| Corresponding Example | 5 | 3 | 4 |
| Dendritic polymer | DGL G4 | PAMAM G5 | PAMAM G6 |
| Oligonucleotide | Atp5b-siRNA | Atp5b-siRNA | Atp5b-siRNA |
| Number of oligonucleotides | 25.6 | 13.9 | 15.9 |
| Hydrophilic linker | PEG5k | PEG5k | PEG5k |
| Cellular internalization enhancer | cRGDfK | cRGDfK | cRGDfK |
| Number of cellular internalization enhancers | 19.9 | 10.5 | 20.8 |
| Fluorescent molecule | TideFluor 7WS | TideFluor 7WS | TideFluor 7WS |
| Number of fluorescent molecules | 2.9 | 1.0 | 2.0 |
| Capping agent | Methyl-PEG12 | Methyl-PEG12 | Methyl-PEG12 |

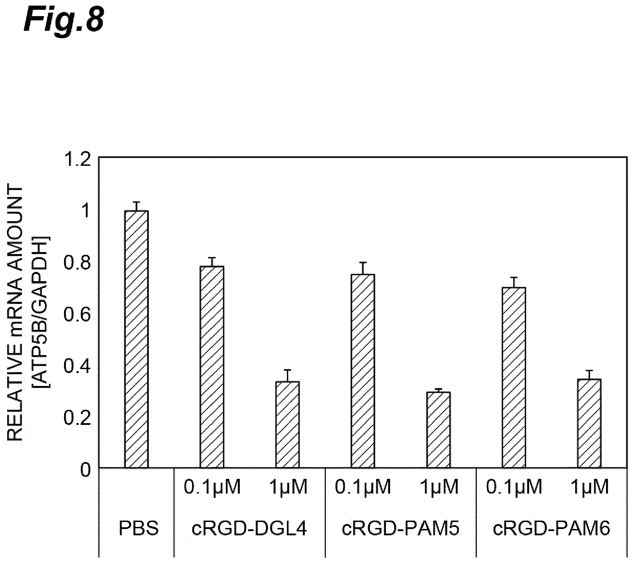

U-87MG cells (human glioblastoma cell line) were seeded in a 96-well plate and cultured in a DMEM medium containing 10% FBS at 37°C with 5% CO₂. The next day, the medium was exchanged, and the sample was added to each well to transfect the cells, which were then cultured at 37°C with 5% CO₂. The concentrations of siRNA for transfection were 0.1 µM or 1 µM. For the control group, PBS was added instead of sample. 48 hours after transfection, mRNA was extracted using RNeasy Mini Kit (manufactured by Qiagen), and cDNA was synthesized from a fixed amount of mRNA using High Capacity RNA-to-cDNA Kit (Applied Biosystems (registered trademark)). Subsequently, quantitative RT-PCR was performed using the obtained cDNA as a template and using PowerUp SYBR Green Master Mix (Applied Biosystems). As ATP5B primers, primers of SEQ ID NO: 12 and SEQ ID NO: 13 shown in Table 8 above were used, and as GAPDH primers, primers of SEQ ID NO: 14 and SEQ ID NO: 15 shown in Table 8 above were used. PCR conditions (temperature and time) were as follows. One cycle was designed to be 95°C for 1 second and 60°C for 30 seconds, and 40 cycles were performed. Based on the results of quantitative RT-PCR, the value of "hATP5B expression level/hGAPDH (internal standard gene) expression level" was calculated, and the calculation result for the control group and the calculation result for the sample addition group were compared. The used samples are shown in Table 11 and the obtained results are shown in FIG. 8.

### <Test Example 6. in vitro evaluation of cRGD ligand-functionalized oligonucleotide conjugate with various numbers of modifications>

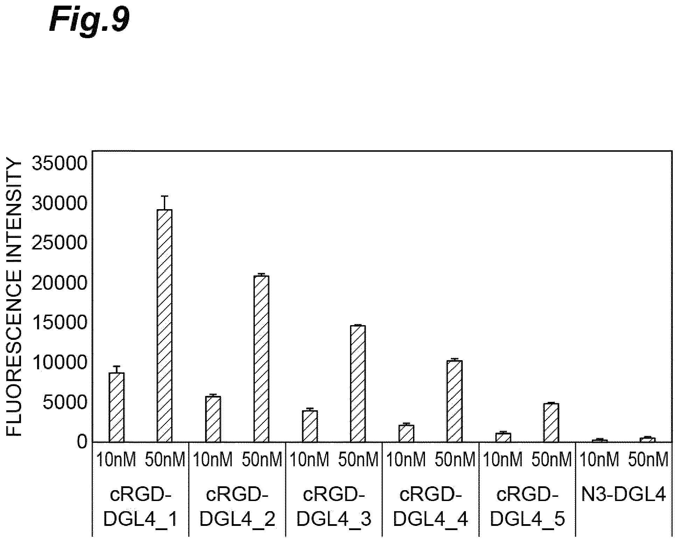

Evaluation of cellular uptake was performed according to the procedure of Test Example 5. The concentrations of dendrimer for transfection were 2 nM or 10 nM. Fluorescence intensity was measured at an excitation wavelength of 540 nm and a fluorescence wavelength of 580 nm. The dendrimer concentration was converted to the concentration of the fluorescent molecules from the number of fluorescent molecules, and after approximating that the fluorescence intensity and the concentration of the fluorescent molecules were in a direct proportional relationship, the fluorescence intensity when the concentration of the fluorescent molecules was 10 nM or 50 nM was calculated. The used samples are shown in Table 12 and the obtained results are shown in FIG. 9.

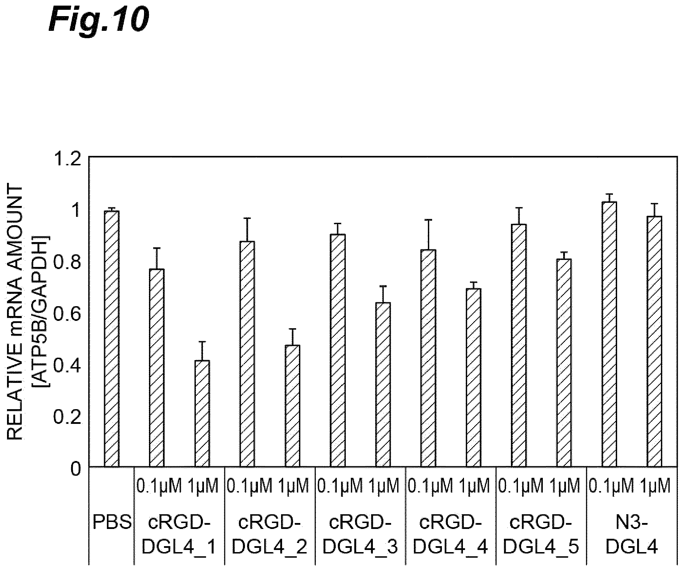

Evaluation of knockdown efficiency was performed according to the procedure of Test Example 5. The used samples are shown in Table 12 and the obtained results are shown in FIG. 10.

**[Table 12]**

| Sample | cRGD-DGL4_1 | cRGD-DGL4_2 | cRGD-DGL4_3 | cRGD-DGL4_4 | cRGD-DGL4_5 | N3-DGL4 |
|---|---|---|---|---|---|---|
| Corresponding Example | 6 | 7 | 8 | 9 | 10 | Synthetic intermediate obtained in 6(B) |
| Dendritic polymer | DGL G4 | DGL G4 | DGL G4 | DGL G4 | DGL G4 | DGL G4 |
| Oligonucleotid e | Atp5b-si RNA | Atp5b-siRNA | Atp5b-siRNA | Atp5b-siRNA | Atp5b-siRNA | Atp5b-siRNA |
| Number of oligonucleotid es | 26.7 | 27.2 | 27.3 | 29.0 | 29.9 | 27.5 |
| Hydrophilic linker | PEG5k | PEG5k | PEG5k | PEG5k | PEG5k | PEG5k |
| Cellular internalization enhancer | cRGDfK | cRGDfK | cRGDfK | cRGDfK | cRGDfK | cRGDfK |
| Number of cellular internalization enhancers | 25.6 | 20.7 | 16.2 | 13.1 | 8.8 | - |
| Fluorescent molecule | AlexaFlu or 546 | AlexaFlu or 546 | AlexaFlu or 546 | AlexaFlu or 546 | AlexaFlu or 546 | AlexaFluor 546 |
| Number of fluorescent molecules | 7.4 | 7.8 | 7.5 | 8.4 | 8.5 | 6.0 |
| Capping agent | Methyl-PEG12 | Methyl-PEG12 | Methyl-PEG12 | Methyl-PEG12 | Methyl-PEG12 | Methyl-PEG12 |

### <Test Example 7. in vitro evaluation of integrin αvβ6 targeted peptide ligand-functionalized oligonucleotide conjugate>

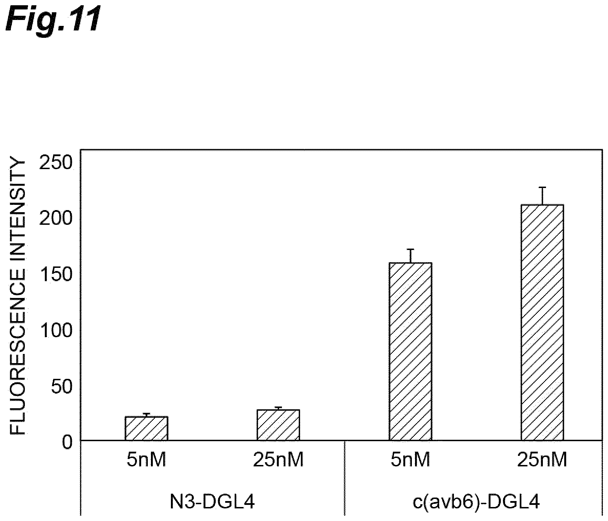

Evaluation of cellular uptake was performed according to the procedure of Test Example 6. H2009 cells (human lung adenocarcinoma cell line) were used as the cells. Fluorescence intensity was measured at an excitation wavelength of 540 nm and a fluorescence wavelength of 580 nm. The dendrimer concentration was converted to the concentration of the fluorescent molecules from the number of fluorescent molecules, and after approximating that the fluorescence intensity and the concentration of the fluorescent molecules were in a direct proportional relationship, the fluorescence intensity when the concentration of the fluorescent molecules was 5 nM or 25 nM was calculated. The used samples are shown in Table 13 and the obtained results are shown in FIG. 11.

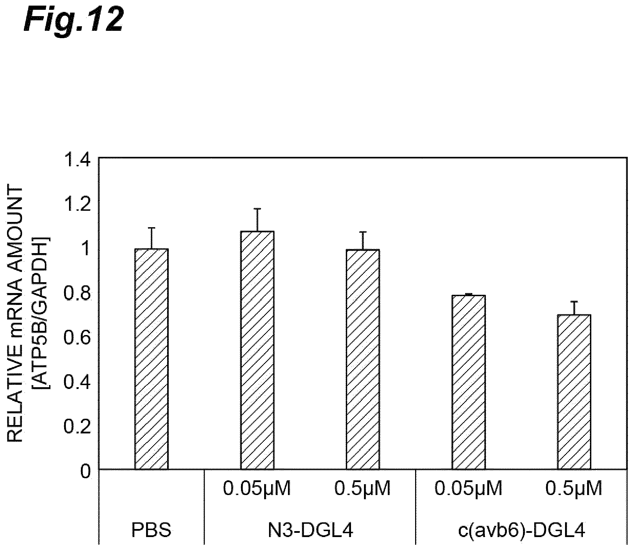

Evaluation of knockdown efficiency was performed according to the procedure of Test Example 6. The concentrations of siRNA for transfection were of 0.05 µM or 0.5 µM. The used samples are shown in Table 13 and the obtained results are shown in FIG. 12.

**[Table 13]**

| Sample | N3-DGL4 | c(avb6)-DGL4 |
|---|---|---|
| Corresponding Example | Synthetic intermediate obtained in 1(B) | 11 |
| Dendritic polymer | DGL G4 | DGL G4 |
| Oligonucleotide | Atp5b-siRNA | Atp5b-siRNA |
| Number of oligonucleotides | 30.2 | 21.9 |
| Hydrophilic linker | PEG5k | PEG5k |
| Cellular internalization enhancer | - | c(avb6) |
| Number of cellular internalization enhancers | - | 16.5 |
| Fluorescent molecule | AlexaFluor 546 | AlexaFluor 546 |
| Number of fluorescent molecules | 2.5 | 4.4 |
| Capping agent | Methyl-PEG12 | Methyl-PEG12 |

### <Test Example 8. in vitro evaluation of folic acid ligand-functionalized oligonucleotide conjugate>

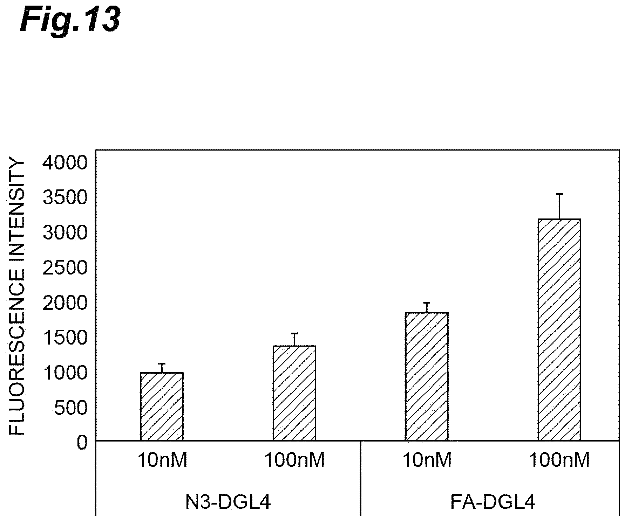

Evaluation of cellular uptake was performed according to the procedure of Test Example 6. KB cells (human oral epidermoid carcinoma cell line) were used as the cells. The fluorescence concentration of samples at the time of transfection was 10 nM or 100 nM. The used samples are shown in Table 14 and the obtained results are shown in FIG. 13.

**[Table 14]**

| Sample | N3-DGL4 | FA-DGL4 |
|---|---|---|
| Corresponding Example | Synthetic intermediate obtained in 1(B) | 12 |
| Dendritic polymer | DGL G4 | DGL G4 |
| Oligonucleotide | Atp5b-siRNA | Atp5b-siRNA |
| Number of oligonucleotides | 20.5 | 28.5 |
| Hydrophilic linker | PEG5k | PEG5k |
| Cellular internalization enhancer | - | Folic acid |
| Number of cellular internalization enhancers | - | 31.5 |
| Fluorescent molecule | AlexaFluor 546 | AlexaFluor 546 |
| Number of fluorescent molecules | 10.0 | 8.9 |
| Capping agent | Methyl-PEG12 | Methyl-PEG12 |

### <Test Example 9. in vitro evaluation of nucleolin-targeted aptamer ligand-functionalized oligonucleotide conjugate>

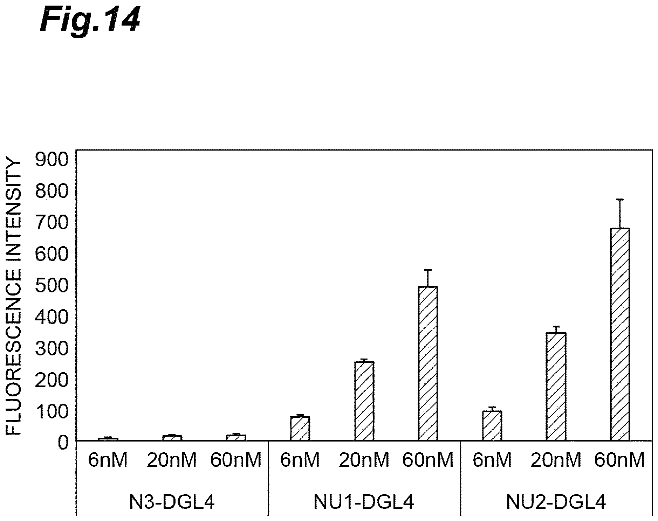

Evaluation of cellular uptake was performed according to the procedure of Test Example 6. MCF-7 (human breast cancer cell line) were used as the cells. The concentrations of dendrimer for transfection were 3 nM, 10 nM, or 30 nM. Fluorescence intensity was measured at an excitation wavelength of 650 nm and a fluorescence wavelength of 695 nm. The dendrimer concentration was converted to the concentration of the fluorescent molecules from the number of fluorescent molecules, and after approximating that the fluorescence intensity and the concentration of the fluorescent molecules were in a direct proportional relationship, the fluorescence intensity when the concentration of the fluorescent molecules was 6 nM, 20 nM, or 60 nM was calculated. The used samples are shown in Table 15 and the obtained results are shown in FIG. 14.

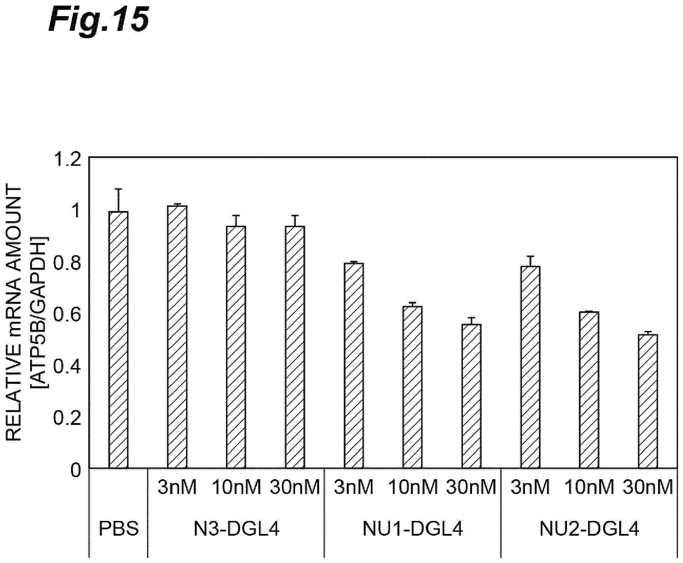

Evaluation of knockdown efficiency was performed according to the procedure of Test Example 6. The concentrations of dendrimer for transfection were 3 nM, 10 nM, or 30 nM. The used samples are shown in Table 15 and the obtained results are shown in FIG. 15.

**[Table 15]**

| Sample | N3-DGL4 | NU1-DGL4 | NU2-DGL4 |
|---|---|---|---|
| Corresponding Example | Synthetic intermediate obtained in 14(B) | 14 | 15 |
| Dendritic polymer | DGL G4 | DGL G4 | DGL G4 |
| Oligonucleotide | Atp5b-siRNA | Atp5b-siRNA | Atp5b-siRNA |
| Number of oligonucleotides | 14.3 | 15.3 | 15.3 |
| Hydrophilic linker | PEG5k | PEG5k | PEG5k |
| Cellular internalization enhancer | - | AS1411 | FAN-1524dT |
| Number of cellular internalization enhancers | - | 16.3 | 16.6 |
| Fluorescent molecule | AlexaFluor 647 | AlexaFluor 647 | AlexaFluor 647 |
| Number of fluorescent molecules | 2.4 | 2.0 | 1.8 |
| Capping agent | Methyl-PEG12 | Methyl-PEG 12 | Methyl-PEG12 |

### <Test Example 10. in vitro evaluation of cRGD ligand-bonded oligonucleotide conjugate using different generations of polylysine dendrigrafts>

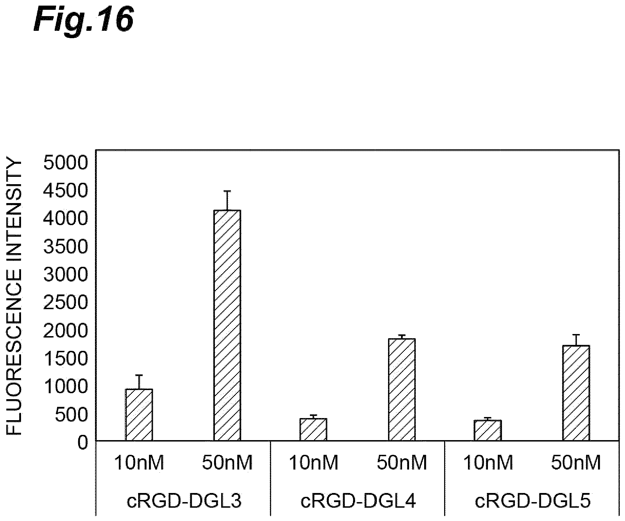

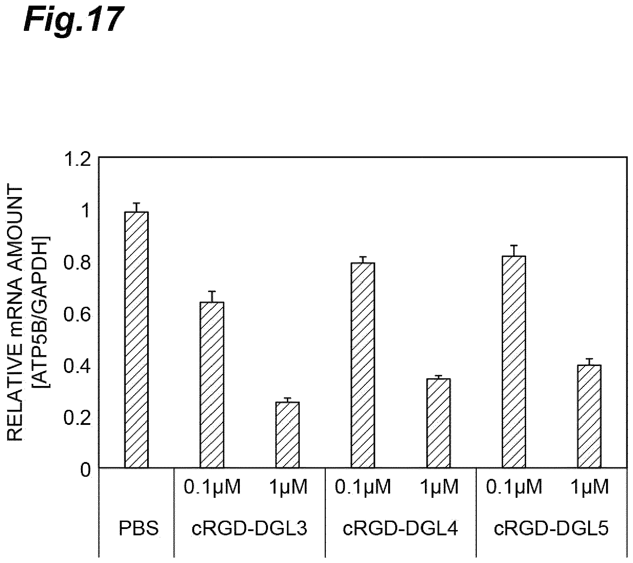

Evaluation of cellular uptake and knockdown efficiency were performed according to the procedure of Test Example 6. The used samples are shown in Table 16 and the obtained results are shown in FIGS. 16 and 17.

**[Table 16]**

| Sample | cRGD-DGL3 | cRGD-DGL4 | cRGD-DGL5 |
|---|---|---|---|
| Corresponding Example | 18 | 1 | 19 |
| Dendritic polymer | DGL G3 | DGL G4 | DGLG5 |
| Oligonucleotide | Atp5b-siRNA | Atp5b-siRNA | Atp5b-siRNA |
| Number of oligonucleotides | 10.9 | 25.3 | 44.8 |
| Hydrophilic linker | PEG5k | PEG5k | PEG5k |
| Cellular internalization enhancer | cRGDfK | cRGDfK | cRGDfK |
| Number of cellular internalization enhancers | 7.2 | 23.6 | 40.5 |
| Fluorescent molecule | AlexaFluor 546 | AlexaFluor 546 | AlexaFluor 546 |
| Number of fluorescent molecules | 1.5 | 6.5 | 14.0 |
| Capping agent | Methyl-PEG12 | Methyl-PEG12 | Methyl-PEG12 |

### <Test Example 11. in vitro evaluation 1 of cRGD ligand-functionalized oligonucleotide conjugate modified with PEG with different molecular weights>

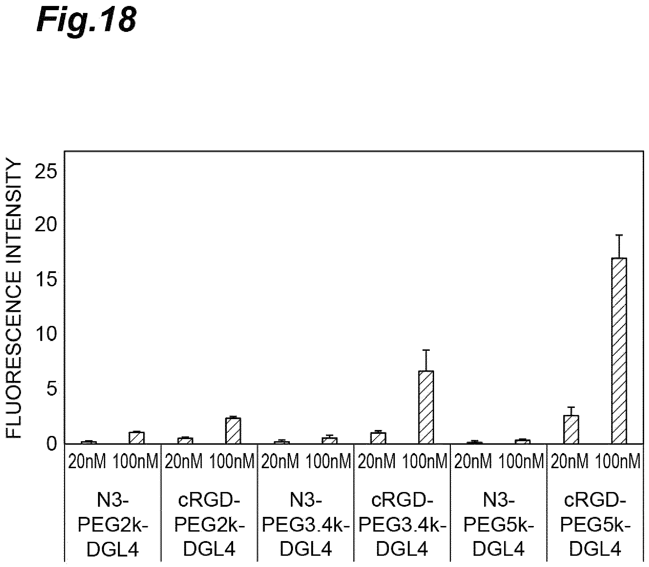

Evaluation of cellular uptake was performed according to the procedure of Test Example 6. The concentrations of dendrimer for transfection were 2 nM or 10 nM. The dendrimer concentration was converted to the concentration of the fluorescent molecules from the number of fluorescent molecules, and after approximating that the fluorescence intensity and the concentration of the fluorescent molecules were in a direct proportional relationship, the fluorescence intensity when the concentration of the fluorescent molecules was 20 nM or 100 nM was calculated. The used samples are shown in Table 17 and the obtained results are shown in FIG. 18.

**[Table 17]**

| Sample | N3-PEG2k-DGL4 | cRGD-PEG2k-DGL4 | N3-PEG3.4k-DGL4 | cRGD-PEG3.4k-DGL4 | N3-PEG5k-DGL4 | cRGD-PEG5k-DGL4 |
|---|---|---|---|---|---|---|
| Correspon ding Example | Synthetic intermedia te obtained in 23(B) | 23 | Synthetic intermedia te obtained in 24 | 24 | 25 | Synthetic intermedia te obtained in 25(B) |
| Dendritic polymer | DGL G4 | DGL G4 | DGL G4 | DGL G4 | DGL G4 | DGLG4 |
| Oligonucle otide | scramble -siRNA | scramble -siRNA | scramble -siRNA | scramble -siRNA | Atp5b-siRNA | Atp5b-siRNA |
| Number of oligonucle otides | 18.3 | 17.9 | 15.5 | 13.7 | 20.5 | 22.9 |
| Hydrophili c linker | PEG2k | PEG2k | PEG3.4k | PEG3.4k | PEG5k | PEG5k |
| Cellular internalizat ion enhancer | - | cRGDfK | - | cRGDfK | - | cRGDfK |
| Number of cellular internalizat ion enhancers | - | 30.2 | - | 31.8 | - | 34.9 |
| Fluorescen t molecule | AlexaFluo r 546 | AlexaFluo r 546 | AlexaFluo r 546 | AlexaFluo r 546 | AlexaFluo r 546 | AlexaFluo r 546 |
| Number of fluorescent molecules | 9.5 | 7.4 | 10.4 | 7.8 | 10.0 | 11.0 |
| Capping agent | Methyl-PEG12 | Methyl-PEG12 | Methyl-PEG12 | Methyl-PEG12 | Methyl-PEG12 | Methyl-PEG12 |

### <Test Example 12. in vitro evaluation 2 of cRGD ligand-functionalized oligonucleotide conjugate modified with PEG with different molecular weights>

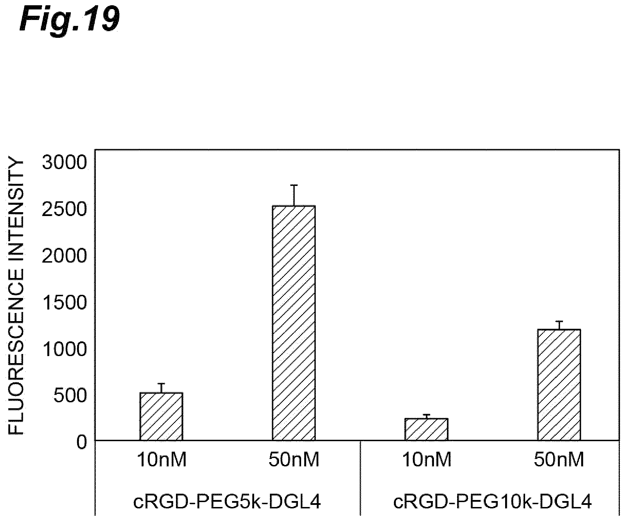

Evaluation of cellular uptake was performed according to the procedure of Test Example 6. The used samples are shown in Table 18 and the obtained results are shown in FIG. 19.

**[Table 18]**

| Sample | cRGD-PEG5k-DGL4 | cRGD-PEG10k-DGL4 |
|---|---|---|
| Corresponding Example | 25 | 26 |
| Dendritic polymer | DGL G4 | DGL G4 |
| Oligonucleotide | Atp5b-siRNA | scramble-siRNA |
| Number of oligonucleotides | 18.3 | 13.1 |
| Hydrophilic linker | PEG5k | PEG10k |
| Cellular internalization enhancer | cRGDfK | cRGDfK |
| Number of cellular internalization enhancers | 34.8 | 21.8 |
| Fluorescent molecule | AlexaFluor 546 | AlexaFluor 546 |
| Number of fluorescent molecules | 1.7 | 3.7 |
| Capping agent | Methyl-PEG12 | Methyl-PEG 12 |

### <Test Example 13. in vitro evaluation of cRGD ligand-functionalized oligonucleotide conjugate using pMeOx>

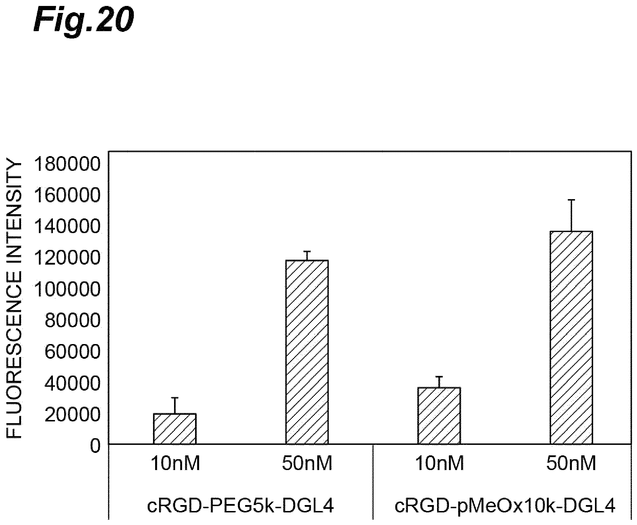

Evaluation of cellular uptake was performed according to the procedure of Test Example 6. The concentrations of dendrimer for transfection were 4 nM or 20 nM. Fluorescence intensity was measured at an excitation wavelength of 740 nm and a fluorescence wavelength of 780 nm. The dendrimer concentration was converted to the concentration of the fluorescent molecules from the number of fluorescent molecules, and after approximating that the fluorescence intensity and the concentration of the fluorescent molecules were in a direct proportional relationship, the fluorescence intensity when the concentration of the fluorescent molecules was 10 nM or 50 nM was calculated. The used samples are shown in Table 19 and the obtained results are shown in FIG. 20.

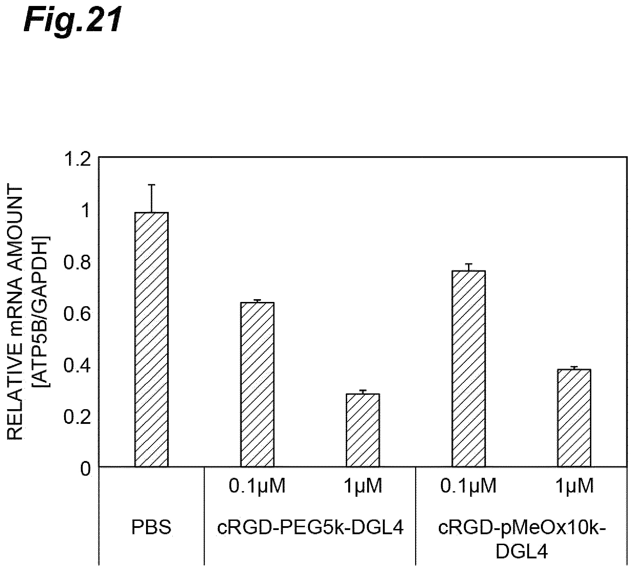

Evaluation of knockdown efficiency was performed according to the procedure of Test Example 6. The used samples are shown in Table 19 and the obtained results are shown in FIG. 21.

**[Table 19]**

| Sample | cRGD-PEG5k-DGL4 | cRGD-pMeOx10k-DGL4 |
|---|---|---|
| Corresponding Example | 5 | 27 |
| Dendritic polymer | DGL G4 | DGL G4 |
| Oligonucleotide | Atp5b-siRNA | Atp5b-siRNA |
| Number of oligonucleotides | 17.7 | 16.8 |
| Hydrophilic linker | PEG5k | pMeOx10k |
| Cellular internalization enhancer | cRGDfK | cRGDfK |
| Number of cellular internalization enhancers | 14.0 | 14.7 |
| Fluorescent molecule | TideFluor7WS | TideFluor7WS |
| Number of fluorescent molecules | 2.1 | 2.5 |
| Capping agent | Methyl-PEG12 | Methyl-PEG12 |

### <Test Example 14. in vitro evaluation of cRGD ligand-functionalized oligonucleotide conjugate using pSar>

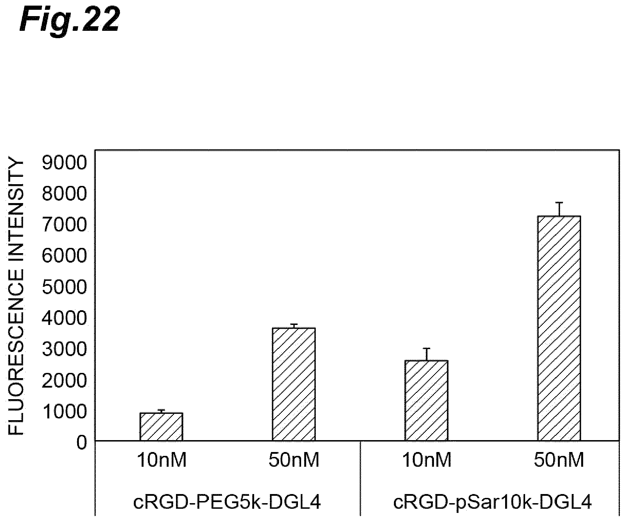

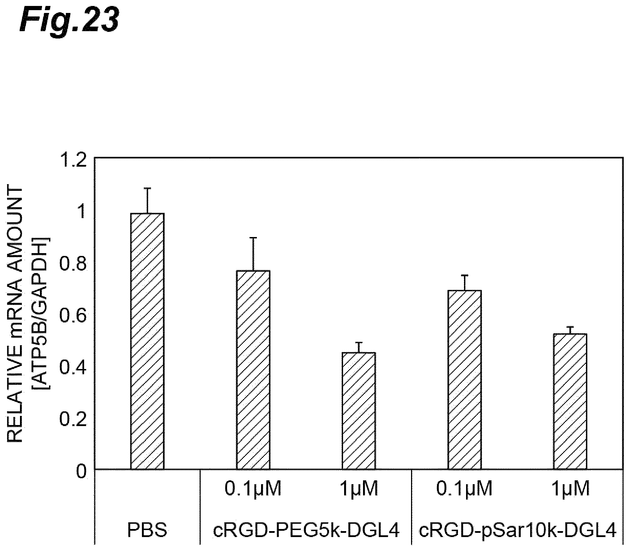

Evaluation of cellular uptake and knockdown efficiencywere performed according to the procedure of Test Example 6. The used samples are shown in Table 20 and the obtained results are shown in FIGS. 22 and 23.

**[Table 20]**

| Sample | cRGD-PEG5k-DGL4 | cRGD-pSar10k-DGL4 |
|---|---|---|
| Corresponding Example | 1 | 28 |
| Dendritic polymer | DGL G4 | DGL G4 |
| Oligonucleotide | Atp5b-siRNA | Atp5b-siRNA |
| Number of oligonucleotides | 27.2 | 21.5 |
| Hydrophilic linker | PEG5k | pSar10k |
| Cellular internalization enhancer | cRGDfK | cRGDfK |
| Number of cellular internalization enhancers | 20.7 | 22.9 |
| Fluorescent molecule | AlexaFluor 546 | AlexaFluor 546 |
| Number of fluorescent molecules | 7.8 | 3.5 |
| Capping agent | Methyl-PEG12 | Methyl-PEG12 |

### <Test Example 15. in vitro evaluation of cRGD ligand-functionalized oligonucleotide conjugate modified with various capping agents>

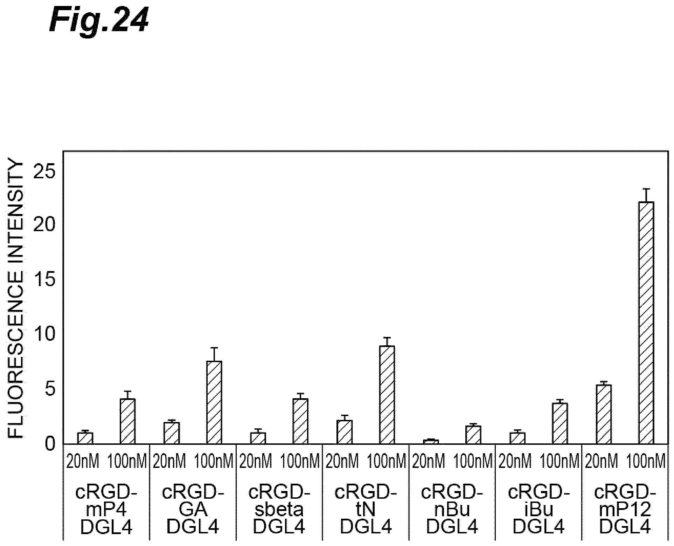

Evaluation of cellular uptake was performed according to the procedure of Test Example 6. The concentrations of dendrimer for transfection were 2 nM or 10 nM. The dendrimer concentration was converted to the concentration of the fluorescent molecules from the number of fluorescent molecules, and after approximating that the fluorescence intensity and the concentration of the fluorescent molecules were in a direct proportional relationship, the fluorescence intensity when the concentration of the fluorescent molecules was 20 nM or 100 nM was calculated. The used samples are shown in Table 21 and the obtained results are shown in FIG. 24.

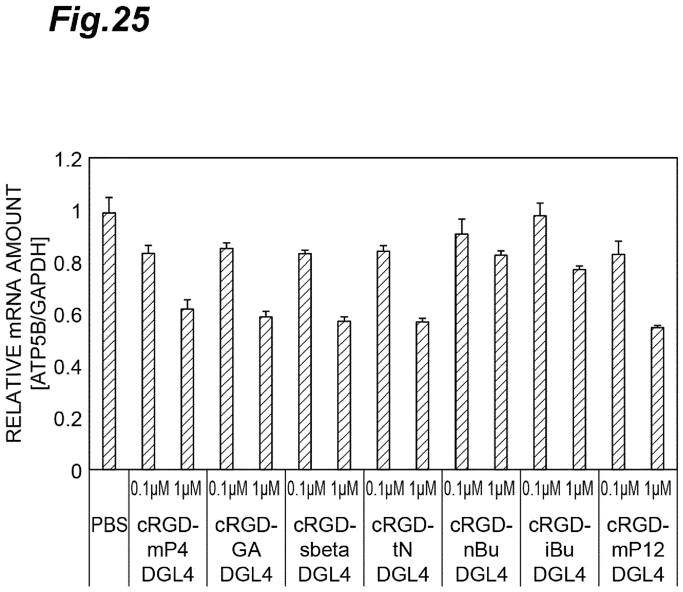

Evaluation of knockdown efficiency was performed according to the procedure of Test Example 6. The used samples are shown in Table 21 and the obtained results are shown in FIG. 25.

**[Table 21]**

| Sample | cRGD-mPEG 1 2-DGL4 | cRGD-mPEG4 -DGL4 | cRGD-GA-DGL4 | cRGD-tN-DGL4 | cRGD-sbeta-DGL4 | cRGD-nBu-DGL4 | cRGD-iBu-DGL4 |
|---|---|---|---|---|---|---|---|
| Corresponding Example | 1 | 31 | 32 | 33 | 34 | 35 | 36 |
| Dendritic polymer | DGL G4 | DGL G4 | DGL G4 | DGL G4 | DGL G4 | DGL G4 | DGL G4 |
| Oligonucleotid e | Atp5b -siRNA | Atp5b -siRNA | Atp5b -siRNA | Atp5b -siRNA | Atp5b -siRNA | Atp5b -siRNA | Atp5b -siRNA |
| Number of oligonucleotid es | 28.1 | 37.6 | 34.0 | 33.0 | 29.0 | 35.7 | 34.9 |
| Hydrophilic linker | PEG5k | PEG5k | PEG5k | PEG5k | PEG5k | PEG5k | PEG5k |
| Cellular internalization enhancer | cRGDf K | cRGDf K | cRGDf K | cRGDf K | cRGDf K | cRGDf K | cRGDf K |
| Number of cellular internalization enhancers | 22.3 | 24.9 | 27,6 | 25.6 | 26.1 | 13.1 | 13.9 |
| Fluorescent molecule | Alexa Fluor 546 | Alexa Fluor 546 | Alexa Fluor 546 | Alexa Fluor 546 | Alexa Fluor 546 | Alexa Fluor 546 | Alexa Fluor 546 |
| Number of fluorescent molecules | 4.3 | 14.4 | 8.2 | 7.6 | 9.3 | 9.1 | 8.4 |
| Capping agent | Methyl -PEG12 | Methyl -PEG4 | Glycolic acid | Dimeth vlamine | Sulfobet aine | n-Bu | i-Bu |

### <Test Example 16. in vitro evaluation of cRGD ligand-functionalized oligonucleotide conjugate modified with capping agent having protonation ability>

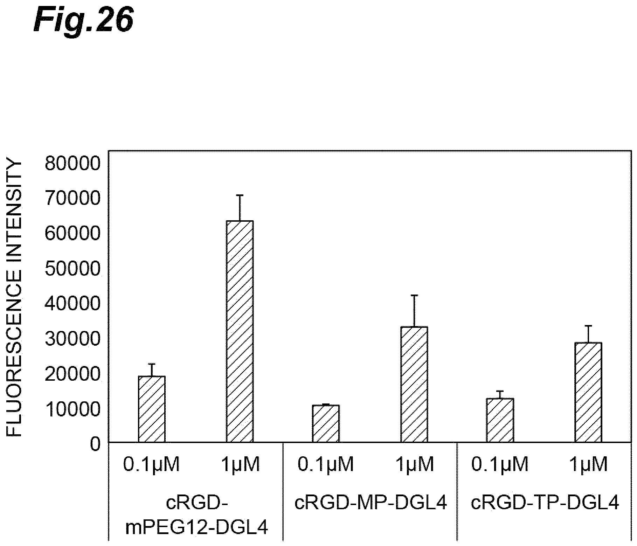

Evaluation of cellular uptake was performed according to the procedure of Test Example 6. The concentrations of siRNA for transfection were 0.1 µM or 1 µM, the dendrimer concentration was converted to the concentration of the fluorescent molecules from the number of fluorescent molecules, and after approximating that the fluorescence intensity and the concentration of the fluorescent molecules were in a direct proportional relationship, the fluorescence intensity when the concentration of the fluorescent molecules was 0.1 µM or 1 µM was calculated. The used samples are shown in Table 22 and the obtained results are shown in FIG. 26.

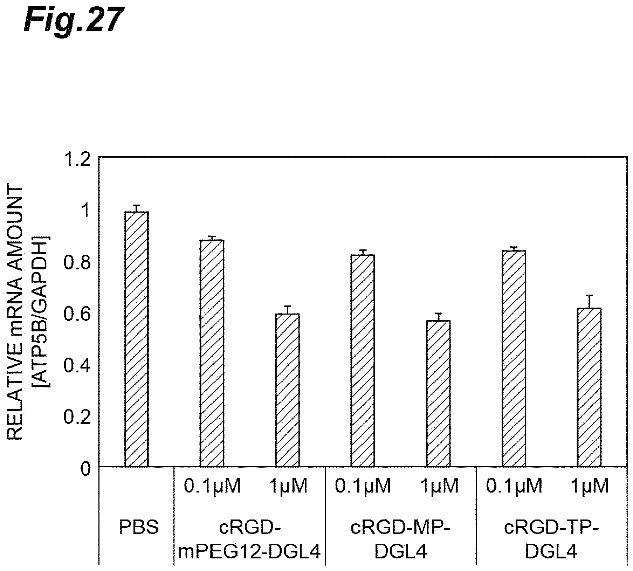

Evaluation of knockdown efficiency was performed according to the procedure of Test Example 6. The used samples are shown in Table 22 and the obtained results are shown in FIG. 27.

**[Table 22]**

| Sample | cRGD-mPEG12-DGL4 | cRGD-MP-DGL4 | cRGD-TP-DGL4 |
|---|---|---|---|
| Corresponding Example | 1 | 37 | 38 |
| Dendritic polymer | DGL G4 | DGL G4 | DGL G4 |
| Oligonucleotide | Atp5b-siRNA | Atp5b-siRNA | Atp5b-siRNA |
| Number of oligonucleotides | 35.5 | 14.2 | 16.8 |
| Hydrophilic linker | PEG5k | PEG5k | PEG5k |
| Cellular internalization enhancer | cRGDfK | cRGDfK | cRGDfK |
| Number of cellular internalization enhancers | 25.4 | 28.8 | 28.3 |
| Fluorescent molecule | AlexaFluor 546 | AlexaFluor 546 | AlexaFluor 546 |
| Number of fluorescent molecules | 16.9 | 10.1 | 11.4 |
| Capping agent | Methyl-PEG 12 | Morpholinyl group | Thiomorpholinyl group |

### <Test Example 17. Evaluation of pH sensitivity of dendritic polymer modified with capping agent having protonation ability>

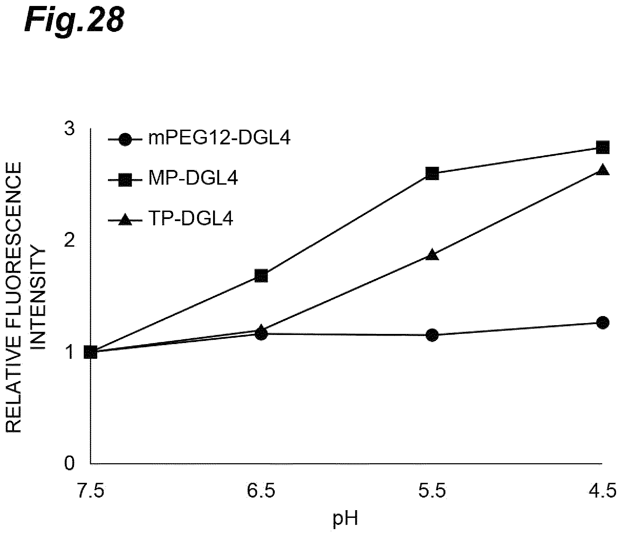

Pure water was added to samples to adjust the dendrimer concentration of each sample to 8.2 µM. In addition, 6-(p-toluidino)-2-naphthalenesulfonic acid sodium salt (TNS, manufactured by Sigma-Aldrich) was dissolved in DMSO to prepare a 2 mg/mL TNS solution. To 12.4 µL of each sample, 487 µL of 10 mM citrate-20 mM phosphate buffered saline at pH 4.5, 5.5, 6.5, or 7.5 was added, and then 5 µL of TNS solution was added and vigorously stirred. After adding 150 µL of each mixed solution to 3 wells in a 96-well plate, fluorescence intensity was measured (excitation wavelength 325 nm, fluorescence wavelength 435 nm). Relative fluorescence intensity was calculated by dividing the fluorescence intensity at each pH by the fluorescence intensity at pH 7.5. The used samples are shown in Table 23 and the obtained results are shown in FIG. 28.

**[Table 23]**

| Sample | mPEG12-DGL4 | MP-DGL4 | TP-DGL4 |
|---|---|---|---|
| Reference Example | 5 | 3 | 4 |
| Dendritic polymer | DGL G4 | DGL G4 | DGLG4 |
| Hydrophilic linker | mPEG2k | mPEG2k | mPEG2k |
| Capping agent | Methyl-PEG12 | Morpholinyl group | Thiomorpholinyl group |

### Industrial Applicability

Since the oligonucleotide conjugate according to one aspect of the present invention can improve the amount of oligonucleotides transported into cytoplasm, the oligonucleotide conjugate can be used as a pharmaceutical composition or medicament for treating or preventing diseases.

### Reference Signs List

1 Oligonucleotide
2 Cellular internalization enhancer
3 Hydrophilic linker
4 Capping agent
5 Linker
10 Core
20 Hydration layer
100 Oligonucleotide conjugate

## Claims

1. An oligonucleotide conjugate comprising: a dendritic polymer; a plurality of oligonucleotides; one or a plurality of cellular internalization enhancers; and one or a plurality of hydrophilic linkers, wherein
each oligonucleotide is bonded to the dendritic polymer directly or through a linker, and
each cellular internalization enhancer is bonded to the dendritic polymer through the hydrophilic linker.

2. The oligonucleotide conjugate according to claim 1, wherein bonds between the dendritic polymer and the oligonucleotides, bonds between the dendritic polymer and the hydrophilic linkers, bonds between the dendritic polymer and the linkers, bonds between the cellular internalization enhancers and the hydrophilic linkers, and bonds between the linkers and the oligonucleotides are covalent bonds, metal coordinations, or host-guest interactions.

3. The oligonucleotide conjugate according to claim 1, wherein bonds between the dendritic polymer and the oligonucleotides, bonds between the dendritic polymer and the hydrophilic linkers, bonds between the dendritic polymer and the linkers, bonds between the cellular internalization enhancers and the hydrophilic linkers, and bonds between the linkers and the oligonucleotides are covalent bonds or metal coordinations.

4. The oligonucleotide conjugate according to claim 1, wherein bonds between the dendritic polymer and the oligonucleotides, bonds between the dendritic polymer and the hydrophilic linkers, bonds between the dendritic polymer and the linkers, bonds between the cellular internalization enhancers and the hydrophilic linkers, and bonds between the linkers and the oligonucleotides are covalent bonds.

5. The oligonucleotide conjugate according to any one of claims 1 to 4, wherein at least some of reactive functional groups of the dendritic polymer are capped with a capping agent.

6. The oligonucleotide conjugate according to claim 5, wherein the capping agent is one or more molecules selected from the group consisting of a hydrophilic molecule and hydrophobic molecule.

7. The oligonucleotide conjugate according to claim 6, wherein the capping agent is a hydrophilic molecule.

8. The oligonucleotide conjugate according to claim 6, wherein the capping agent is one or more hydrophilic molecules selected from the group consisting of an electrically neutral hydrophilic molecule, polar molecule that protonates under acidic conditions, anionic molecule, and cationic molecule.

9. The oligonucleotide conjugate according to claim 6, wherein the capping agent is one or more hydrophilic molecules selected from the group consisting of an electrically neutral hydrophilic molecule, polar molecule that protonates under acidic conditions, and anionic molecule.

10. The oligonucleotide conjugate according to claim 6, wherein the capping agent is a hydrophobic molecule.

11. The oligonucleotide conjugate according to claim 6, wherein the capping agent is one or more molecules selected from the group consisting of an aliphatic compound, an aromatic compound, a trialkylamine, and a steroid.

12. The oligonucleotide conjugate according to claim 6, wherein the capping agent is an aliphatic compound.

13. The oligonucleotide conjugate according to any one of claims 1 to 12, wherein the dendritic polymer is a dendrigraft or a dendrimer.

14. The oligonucleotide conjugate according to any one of claims 1 to 12, wherein monomers in the dendritic polymer are bonded to each other by amide bonds, ester bonds, or glycosidic bonds.

15. The oligonucleotide conjugate according to any one of claims 1 to 12, wherein monomers in the dendritic polymer are bonded to each other by amide bonds or ester bonds.

16. The oligonucleotide conjugate according to any one of claims 1 to 12, wherein the dendritic polymer is a poly-L-lysine dendrigraft, a polyamidoamine dendrimer, or a 2,2-bis(hydroxyl-methyl)propionic acid dendrimer.

17. The oligonucleotide conjugate according to any one of claims 1 to 16, wherein the oligonucleotide is a gene expression modifier.

18. The oligonucleotide conjugate according to claim 17, wherein the gene expression modifier is a molecule that downregulates mRNA expression.

19. The oligonucleotide conjugate according to claim 17, wherein the gene expression modifier is an RNA interference inducer or an antisense oligonucleotide.

20. The oligonucleotide conjugate according to any one of claims 1 to 19, wherein an average linear distance between ends of each hydrophilic linker is 1/5 or more of a length of the oligonucleotide.

21. The oligonucleotide conjugate according to any one of claims 1 to 19, wherein an average linear distance between ends of each hydrophilic linker is 1/4 or more of a length of the oligonucleotide.

22. The oligonucleotide conjugate according to any one of claims 1 to 19, wherein an average linear distance between ends of each hydrophilic linker is 1/3 or more of a length of the oligonucleotide.

23. The oligonucleotide conjugate according to any one of claims 1 to 19, wherein an average linear distance between ends of each hydrophilic linker is 2/5 or more of a length of the oligonucleotide.

24. The oligonucleotide conjugate according to any one of claims 1 to 19, wherein an average linear distance between ends of each hydrophilic linker is half or more of a length of the oligonucleotide.

25. The oligonucleotide conjugate according to any one of claims 1 to 24, wherein the hydrophilic linker is one or more hydrophilic linkers selected from the group consisting of polyethylene glycol, poly(2-alkyl-2-oxazoline), polypeptide, and polypeptoid.

26. The oligonucleotide conjugate according to any one of claims 1 to 24, wherein the hydrophilic linker is one or more hydrophilic linkers selected from the group consisting of polyethylene glycol, poly(2-methyl-2-oxazoline), EK peptide, and polysarcosine.

27. The oligonucleotide conjugate according to any one of claims 1 to 26, wherein the cellular internalization enhancer is one or more cellular internalization enhancers selected from the group consisting of a small-molecule ligand, polypeptide, aptamer, antibody or fragment thereof, saccharide, and lipid.

28. The oligonucleotide conjugate according to any one of claims 1 to 26, wherein the cellular internalization enhancer is a small-molecule ligand.

29. The oligonucleotide conjugate according to any one of claims 1 to 26, wherein the cellular internalization enhancer is a polypeptide.

30. The oligonucleotide conjugate according to any one of claims 1 to 26, wherein the cellular internalization enhancer is an aptamer.

31. The oligonucleotide conjugate according to any one of claims 1 to 26, wherein the cellular internalization enhancer is an antibody or a fragment thereof.

32. The oligonucleotide conjugate according to any one of claims 1 to 26, wherein the cellular internalization enhancer is a saccharide.

33. The oligonucleotide conjugate according to any one of claims 1 to 26, wherein the cellular internalization enhancer is a lipid.

34. A pharmaceutical composition comprising the oligonucleotide conjugate according to any one of claims 1 to 33 as an active ingredient.

35. A therapeutic agent or a preventive agent comprising the oligonucleotide conjugate according to any one of claims 1 to 33 as an active ingredient,
wherein the therapeutic agent or the preventive agent is for a disease selected from the group consisting of inborn errors of metabolism, a congenital endocrine disease, a single gene disorder, a neurodegenerative disease, a neurologic disease, a myopathy, a meningitis, an encephalitis, an encephalopathy, a lysosome disease, a malignant neoplasm, a fibrosis, an inflammatory disease, an immunodeficiency disease, an autoimmune disease, and an infectious disease.

36. A method for treating and/or preventing a disease selected from the group consisting of inborn errors of metabolism, a congenital endocrine disease, a single gene disorder, a neurodegenerative disease, a neurologic disease, a myopathy, a meningitis, an encephalitis, an encephalopathy, a lysosome disease, a malignant neoplasm, a fibrosis, an inflammatory disease, an immunodeficiency disease, an autoimmune disease, and an infectious disease, the method comprising:
administering a therapeutically effective amount of the oligonucleotide conjugate according to any one of claims 1 to 33.

37. A use of the oligonucleotide conjugate according to any one of claims 1 to 33, for producing a therapeutic agent and/or a preventive agent for a disease selected from the group consisting of inborn errors of metabolism, a congenital endocrine disease, a single gene disorder, a neurodegenerative disease, a neurologic disease, a myopathy, a meningitis, an encephalitis, an encephalopathy, a lysosome disease, a malignant neoplasm, a fibrosis, an inflammatory disease, an immunodeficiency disease, an autoimmune disease, and an infectious disease.

38. The oligonucleotide conjugate according to any one of claims 1 to 33 for use in the treatment and/or prevention of a disease selected from the group consisting of inborn errors of metabolism, a congenital endocrine disease, a single gene disorder, a neurodegenerative disease, a neurologic disease, a myopathy, a meningitis, an encephalitis, an encephalopathy, a lysosome disease, a malignant neoplasm, a fibrosis, an inflammatory disease, an immunodeficiency disease, an autoimmune disease, and an infectious disease.

39. A medicament comprising a combination of
the oligonucleotide conjugate according to any one of claims 1 to 33; and
one or more therapeutic agents and/or one or more preventive agents for a disease,
wherein the disease is selected from the group consisting of inborn errors of metabolism, a congenital endocrine disease, a single gene disorder, a neurodegenerative disease, a neurologic disease, a myopathy, a meningitis, an encephalitis, an encephalopathy, a lysosome disease, a malignant neoplasm, a fibrosis, an inflammatory disease, an immunodeficiency disease, an autoimmune disease, and an infectious disease.

40. The oligonucleotide conjugate according to any one of claims 1 to 33 for treating a disease in combination with one or more therapeutic agents and/or one or more preventive agents for the disease,
wherein the disease is selected from the group consisting of inborn errors of metabolism, a congenital endocrine disease, a single gene disorder, a neurodegenerative disease, a neurologic disease, a myopathy, a meningitis, an encephalitis, an encephalopathy, a lysosome disease, a malignant neoplasm, a fibrosis, an inflammatory disease, an immunodeficiency disease, an autoimmune disease, and an infectious disease.

41. A method for producing the oligonucleotide conjugate according to any one of claims 1 to 33, the method comprising steps of
bonding a plurality of oligonucleotides and one or more hydrophilic linkers to a dendritic polymer; and
bonding a cellular internalization enhancer to each hydrophilic linker.

42. The method for producing the oligonucleotide conjugate according to claim 41, further comprising a step of bonding a capping agent to the dendritic polymer.
